# EUROPEAN PATENT APPLICATION

(11) **EP 4 450 590 A2**
(43) Date of publication of application: **23.10.2024**
(21) Application number: 24199330.2
(22) Date of filing: 07.04.2015
(51) Int. Cl.: C09K 11/06

(54) **MULTI-COMPONENT HOST MATERIAL AND ORGANIC ELECTROLUMINESCENT DEVICE COMPRISING THE SAME**

(30) Priority: 08.04.2014 KR 20140041844; 10.07.2014 KR 20140086754; 26.03.2015 KR 20150042704
(62) Divisional of application: 15777273.2
(71) Applicant: Rohm And Haas Electronic Materials Korea Ltd., Chungcheongnam-do 331-980 (KR)
(72) Inventor: AHN, Hee-Choon, 443-400 Gyeonggi-do (KR); KIM, Young-Kwang, 331-980 Chungcheongnam-do (KR); MOON, Doo-Hyeon, 445-768 Gyeonggi-do (KR); LEE, Su-Hyun, 440-200 Gyeonggi-do (KR); LEE, Seon-Woo, 331-980 Chungcheongnam-do (KR); KIM, Chi-Sik, 445-752 Gyeonggi-do (KR); PARK, Kyoung-Jin, 462-838 Gyeonggi-do (KR); KIM, Nam-Kyun, 448-527 Gyeonggi-do (KR); CHOI, Kyung-Hoon, 445-160 Gyeonggi-do (KR); SHIM, Jae-Hoon, 331-980 Chungcheongnam-do (KR); CHO, Young-Jun, 463-400 Gyeonggi-do (KR); LEE, Kyung-Joo, 121-773 Seoul (KR)
(74) Representative: Houghton, Mark Phillip

(57) **Abstract**

The present invention relates to an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is a specific bicarbazole derivative containing an aryl group, and a second host compound is a specific carbazole derivative including a nitrogen-containing heteroaryl group. According to the present invention, the organic electroluminescent device using the multi-component host compounds has a high efficiency and long lifespan compared with the conventional device using one component host compound.

## Description

### Technical Field

The present invention relates to a multi-component host material and an organic electroluminescent device comprising the same.

### Background Art

An electroluminescent (EL) device is a self-light-emitting device with the advantage of providing a wider viewing angle, a greater contrast ratio, and a faster response time. An organic EL device was first developed by Eastman Kodak, by using small aromatic diamine molecules and aluminum complexes as materials for forming a light-emitting layer (see Appl. Phys. Lett. 51, 913, 1987).

The organic EL device changes electric energy into light by the injection of a charge into an organic light-emitting material and commonly comprises an anode, a cathode, and an organic layer formed between the two electrodes. The organic layer of the organic EL device may be composed of a hole injection layer (HIL), a hole transport layer (HTL), an electron blocking layer (EBL), a light-emitting layer (EML) (containing host and dopant materials), an electron buffer layer, a hole blocking layer (HBL), an electron transport layer (ETL), an electron injection layer (EIL), etc.; the materials used in the organic layer can be classified into a hole injection material, a hole transport material, an electron blocking material, a light-emitting material, an electron buffer material, a hole blocking material, an electron transport material, an electron injection material, etc., depending on functions. In the organic EL device, holes from an anode and electrons from a cathode are injected into a light-emitting layer by the injection of a charge, and an exciton having high energy is produced by the recombination of holes and electrons. The organic light-emitting compound moves into an excited state by the energy and emits light which changes from energy when the organic light-emitting compound returns to the ground state from the excited state.

The most important factor determining luminescent efficiency in an organic EL device is the light-emitting material. The light-emitting material is required to have the following features: high quantum efficiency, high movement degree of an electron and a hole, formability of a uniform layer, and stability. The light-emitting material is classified into blue light-emitting materials, green light-emitting materials, and red light-emitting materials according to the light-emitting color, and further includes yellow light-emitting materials or orange light-emitting materials. Furthermore, the light-emitting material is classified into a host material and a dopant material in the functional aspect. Recently, an urgent task is the development of an organic EL device having high efficacy and long operating lifespan. In particular, the development of highly excellent light-emitting material over conventional light-emitting materials is urgent considering EL properties required in medium- and large-sized OLED panels. For this, preferably, as a solvent in a solid state and energy transmitter, a host material should have high purity and suitable molecular weight in order to be deposited under vacuum. Furthermore, a host material is required to have high glass transition temperature and pyrolysis temperature to guarantee thermal stability, high electrochemical stability to provide long lifespan, easy formability of an amorphous thin film, good adhesion with adjacent layers, and no movement between layers.

A mixed system of a dopant/host material can be used as a light-emitting material to improve color purity, luminescent efficiency, and stability. Generally, the device having the most excellent EL properties comprises the light-emitting layer, wherein a dopant is doped onto a host. If the dopant/host material system is used, the selection of the host material is important since the host material greatly influences on efficiency and performance of a light-emitting device.

WO 2013/168688 A1, Japanese Patent No. 3139321, Korean Patent No. 10-1170666, Korean Patent Application Laying-open No. 10-2012-0013173, and WO 2013/112557 A1 disclose organic EL devices comprising a dopant/host material system. The above literature use one host component having a carbazole-carbazole skeleton or exclude a host having a cabazole skeleton from second and third hosts.

The present inventors have found that an organic EL device using a multi-component host compounds having a specific bicarbazole derivative which contains an aryl group and a specific carbazole derivative which includes a nitrogen-containing heteroaryl group has high efficiency and long lifespan, compared with using one component host compound in a light-emitting layer.

### Disclosure of the Invention

### Problems to be Solved

The object of the present invention is to provide an organic EL device having high efficiency and long lifespan.

### Solution to Problems

The above objective can be achieved by an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by the following formula 1 which is a specific bicarbazole derivative containing an aryl group, and a second host compound is represented by the following formula 2 which is a specific carbazole derivative including a nitrogen-containing heteroaryl group: 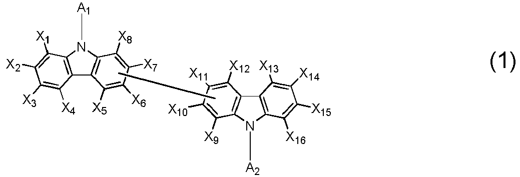 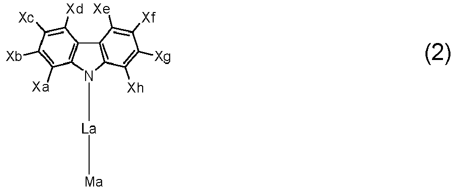 wherein
A₁ and A₂ each independently represent a substituted or unsubstituted (C6-C30)aryl group;
X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) may be replaced with at least one hetero atom selected from nitrogen, oxygen and sulfur;
Ma represents a substituted or unsubstituted nitrogen-containing 5- to 30-membered heteroaryl group;
La represents a single bond, or a substituted or unsubstituted (C6-C30)arylene group;
Xa to Xh each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
the fused aromatic or heteroaromatic ring is selected from the group consisting of benzene, indole, indene, benzofuran and benzothiophene, which may be further substituted with a (C1-C10)alkyl group or a (C6-C15)aryl group; and
the heteroaryl group contains at least one hetero atom selected from B, N, O, S, P(=O), Si and P.

### Effects of the Invention

According to the present invention, the organic EL device having high efficiency and long lifespan is provided and the production of a display device or a lighting device is possible by using the organic EL device.

### Embodiments of the Invention

Hereinafter, the present invention will be described in detail. However, the following description is intended to explain the invention, and is not meant in any way to restrict the scope of the invention.

The compound of formula 1 is represented by the following formula 3, 4, 5, or 6: 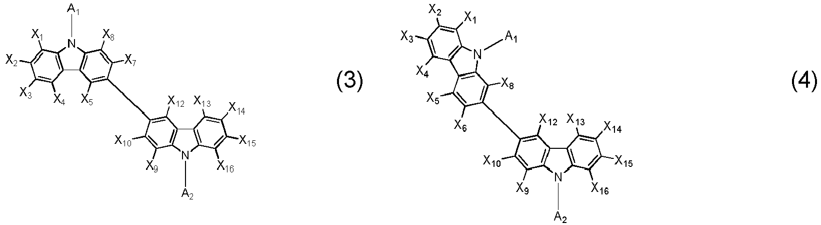 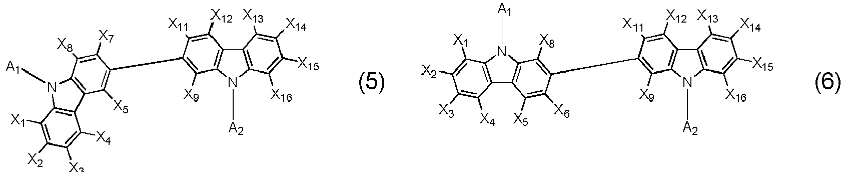 wherein
A₁, A₂ and X₁ to X₁₆ are as defined in formula 1.

In formula 1, A₁ and A₂ each independently represent a substituted or unsubstituted (C6-C30)aryl group; preferably, a substituted or unsubstituted (C6-C18)aryl group; more preferably, a (C6-C18)aryl group which is unsubstituted or substituted with a (C1-C6)alkyl group, a (C6-C12)aryl group, or a tri(C6-C12)arylsilyl group ; and even more preferably, phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, or fluoranthenyl.

In formula 1, X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur; preferably, hydrogen, a substituted or unsubstituted (C6-C20)aryl group, a substituted or unsubstituted tri(C6-C12)arylsilyl group, or a substituted or unsubstituted 3- to 15-membered heteroaryl group; and more preferably, hydrogen, a substituted or unsubstituted (C6-C18)aryl group, an unsubstituted triphenylsilyl group, a substituted or unsubstituted dibenzothiophene group, or a substituted or unsubstituted dibenzofuran group.

The compound of formula 2 is represented by the following formula 7, 8, or 9: 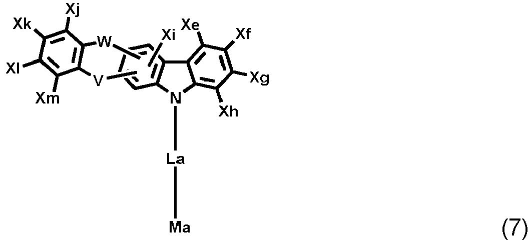 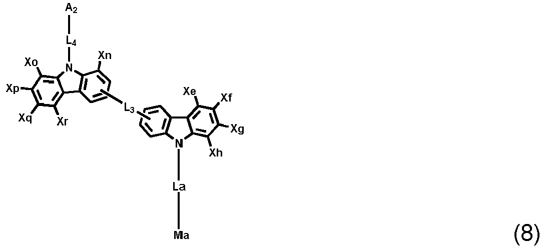 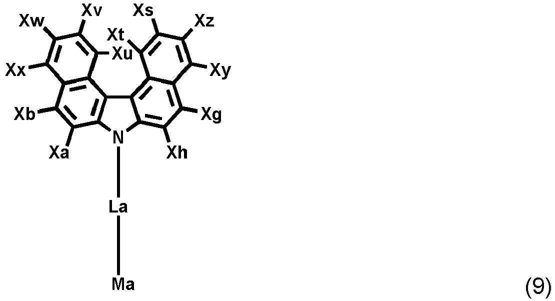 wherein
V and W each independently represent a single bond, NR₁₅, CR₁₆R₁₇, S, or O, provided that both V and W neither represent a single bond nor represent NR₁₅;
A₂ represents a substituted or unsubstituted (C6-C30)aryl group and may be bonded to Xn or Xo;
L₃ and L₄ each independently represent a single bond, or a substituted or unsubstituted (C6-C60)arylene group;
Xi represents hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
Xj to Xz each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, a substituted or unsubstituted 3- to 30-membered heteroaryl group, -NR₅R₆, or-SiR₇R₈R₉; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
Ma, La, Xa, Xb, and Xe to Xh are as defined in formula 2;
R₅ to R₉ each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, or a substituted or unsubstituted 3- to 30-membered heteroaryl group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur; preferably, hydrogen, or a substituted or unsubstituted (C6-C25)aryl group; more preferably, hydrogen or an unsubstituted (C6-C18)aryl group; and specifically, hydrogen, an unsubstituted phenyl group, a biphenyl group, or a fluorenyl group;
R₁₆ and R₁₇ each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, or a substituted or unsubstituted 3- to 30-membered heteroaryl group; and
R₁₅ represents hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3- to 7-membered heterocycloalkyl group, a substituted or unsubstituted (C6-C60)aryl group, or a substituted or unsubstituted 3- to 30-membered heteroaryl group; preferably, a substituted or unsubstituted (C6-C30)aryl group; and more preferably, a substituted or unsubstituted phenyl group, an unsubstituted biphenyl group, an unsubstituted naphthyl group, or a substituted fluorenyl group.

In formula 2, La represents a single bond, or a substituted or unsubstituted (C6-C30)arylene group; preferably, a single bond, or a substituted or unsubstituted (C6-C12)arylene group; and more preferably, a single bond, a (C6-C12)arylene group which is unsubstituted or substituted with a tri(C6-C10)arylsilyl group or a (C6-C12)aryl group.

Furthermore, La represents a single bond, or is represented by one selected from the following formulas 10 to 19: 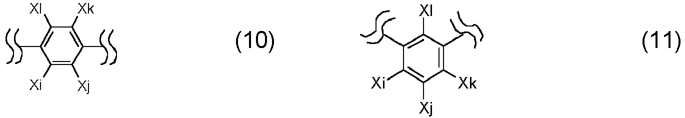 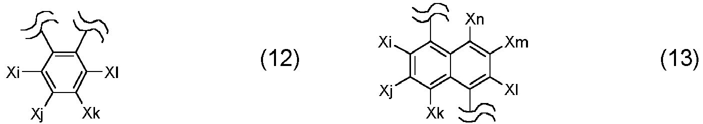 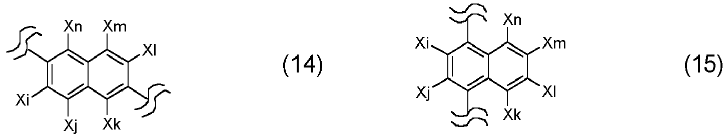 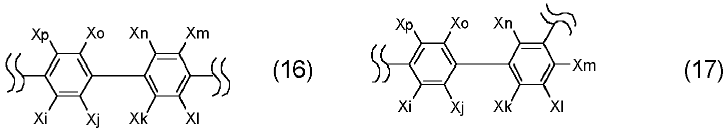 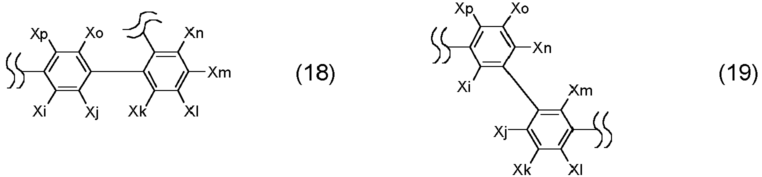 wherein
Xi to Xp each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur; preferably, hydrogen, a cyano group, a substituted or unsubstituted (C6-C15)aryl group, a substituted or unsubstituted 10- to 20-membered heteroaryl group, or a substituted or unsubstituted tri(C6-C10)arylsilyl group; more preferably, hydrogen, a cyano group, a (C6-C15)aryl group which is unsubstituted or substituted with a tri(C6-C10)arylsilyl group, or a 10- to 20-membered heteroaryl group which is unsubstituted or substituted with a (C6-C15)aryl group.

In formula 2, Ma represents a substituted or unsubstituted nitrogen-containing 5- to 11-membered heteroaryl group; preferably, a substituted or unsubstituted nitrogen-containing 6- to 10-membered heteroaryl group; and more preferably, a nitrogen-containing 6- to 10-membered heteroaryl group which is substituted with a substituents(s) selected from the group consisting of an unsubstituted (C6-C18)aryl group, a (C6-C12)aryl group substituted with a cyano group, a (C6-C12)aryl group substituted with a (C1-C6)alkyl group, a (C6-C12)aryl group substituted with a tri(C6-C12)arylsilyl group, and a 6- to 15-membered heteroaryl group.

Furthermore, Ma represents a monocyclic-based heteroaryl group selected from the group consisting of pyrrolyl, imidazolyl, pyrazolyl, triazinyl, tetrazinyl, triazolyl, tetrazolyl, pyridyl, pyrazinyl, pyrimidinyl, pyridazinyl, etc., or a fused ring-based heteroaryl group selected from the group consisting of benzoimidazolyl, isoindolyl, indolyl, indazolyl, benzothiadiazolyl, quinolyl, isoquinolyl, cinnolinyl, quinazolinyl, naphthyridinyl, quinoxalinyl, carbazolyl, phenanthridinyl, etc.; preferably, triazinyl, pyrimidinyl, pyridyl, quinolyl, isoquinolyl, quinazolinyl, naphthyridinyl, or quinoxalinyl.

Herein, "(C1-C30)alkyl(ene)" is meant to be a linear or branched alkyl(ene) having 1 to 30 carbon atoms, in which the number of carbon atoms is preferably 1 to 20, more preferably 1 to 10, and includes methyl, ethyl, n-propyl, isopropyl, n-butyl, isobutyl, tert-butyl, etc. "(C2-C30)alkenyl" is meant to be a linear or branched alkenyl having 2 to 30 carbon atoms, in which the number of carbon atoms is preferably 2 to 20, more preferably 2 to 10, and includes vinyl, 1-propenyl, 2-propenyl, 1-butenyl, 2-butenyl, 3-butenyl, 2-methylbut-2-enyl, etc. "(C2-C30)alkynyl" is a linear or branched alkynyl having 2 to 30 carbon atoms, in which the number of carbon atoms is preferably 2 to 20, more preferably 2 to 10, and includes ethynyl, 1-propynyl, 2-propynyl, 1-butynyl, 2-butynyl, 3-butynyl, 1-methylpent-2-ynyl, etc. "(C3-C30)cycloalkyl" is a mono- or polycyclic hydrocarbon having 3 to 30 carbon atoms, in which the number of carbon atoms is preferably 3 to 20, more preferably 3 to 7, and includes cyclopropyl, cyclobutyl, cyclopentyl, cyclohexyl, etc. "3- to 7-membered heterocycloalkyl" is a cycloalkyl having at least one heteroatom selected from the group consisting of B, N, O, S, P(=O), Si and P, preferably O, S and N, and 3 to 7, preferably 5 to 7 ring backbone atoms, and includes tetrahydrofuran, pyrrolidine, thiolan, tetrahydropyran, etc. "(C6-C30)aryl(ene)" is a monocyclic or fused ring derived from an aromatic hydrocarbon having 6 to 30 carbon atoms, in which the number of carbon atoms is preferably 6 to 20, more preferably 6 to 15, and includes phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, fluoranthenyl, etc. "3- to 30-membered heteroaryl(ene)" is an aryl group having at least one, preferably 1 to 4 heteroatom selected from the group consisting of B, N, O, S, P(=O), Si and P, and 3 to 30 ring backbone atoms; is a monocyclic ring, or a fused ring condensed with at least one benzene ring; has preferably 3 to 20, more preferably 3 to 15 ring backbone atoms; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl including benzofuranyl, benzothiophenyl, isobenzofuranyl, dibenzofuranyl, dibenzothiophenyl, benzoimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenoxazinyl, phenanthridinyl, benzodioxolyl, etc. "Nitrogen-containing 5- to 30-membered heteroaryl(ene) group" is an aryl(ene) group having at least one heteroatom N and 5 to 30 ring backbone atoms. 5 to 20 ring backbone atoms and 1 to 4 heteroatom are preferable, and 5 to 15 ring backbone atoms are more preferable. It is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including pyrrolyl, imidazolyl, pyrazolyl, triazinyl, tetrazinyl, triazolyl, tetrazolyl, pyridyl, pyrazinyl, pyrimidinyl, pyridazinyl, etc., and a fused ring-type heteroaryl including benzoimidazolyl, isoindolyl, indolyl, indazolyl, benzothiadiazolyl, quinolyl, isoquinolyl, cinnolinyl, quinazolinyl, quinoxalinyl, carbazolyl, phenanthridinyl, etc. "Halogen" includes F, Cl, Br and I.

Herein, "substituted" in the expression "substituted or unsubstituted" means that a hydrogen atom in a certain functional group is replaced with another atom or group, i.e., a substituent. Substituents of the substituted alkyl(ene) group, the substituted alkenyl group, the substituted alkynyl group, the substituted cycloalkyl group, the substituted aryl(ene) group, the substituted heteroaryl(ene) group, the substituted trialkylsilyl group, the substituted triarylsilyl group, the substituted dialkylarylsilyl group, the substituted mono- or di-arylamino group, or the substituted mono- or polycyclic, (C3-C30) alicyclic or aromatic ring are each independently at least one selected from the group consisting of deuterium; a halogen; a cyano group; a carboxyl group; a nitro group; a hydroxyl group; a (C1-C30)alkyl group; a halo(C1-C30)alkyl group; a (C2-C30)alkenyl group; a (C2-C30)alkynyl group; a (C1-C30)alkoxy group; a (C1-C30)alkylthio group; a (C3-C30)cycloalkyl group; a (C3-C30)cycloalkenyl group; a 3- to 7-membered heterocycloalkyl group; a (C6-C30)aryloxy group; a (C6-C30)arylthio group; a 3- to 30-membered heteroaryl group which is unsubstituted or substituted with a (C6-C30)aryl group; a (C6-C30)aryl group which is unsubstituted or substituted with a cyano group, a 3- to 30-membered heteroaryl group, or a tri(C6-C30)arylsilyl group; a tri(C1-C30)alkylsilyl group; a tri(C6-C30)arylsilyl group; a di(C1-C30)alkyl(C6-C30)arylsilyl group; a (C1-C30)alkyldi(C6-C30)arylsilyl group; an amino group; a mono- or di(C1-C30)alkylamino group; a mono- or di(C6-C30)arylamino group; a (C1-C30)alkyl(C6-C30)arylamino group; a (C1-C30)alkylcarbonyl group; a (C1-C30)alkoxycarbonyl group; a (C6-C30)arylcarbonyl group; a di(C6-C30)arylboronyl group; a di(C1-C30)alkylboronyl group; a (C1-C30)alkyl(C6-C30)arylboronyl group; a (C6-C30)aryl(C1-C30)alkyl group; and a (C1-C30)alkyl(C6-C30)aryl group. Preferably, the substituents are each independently at least one selected from the group consisting of a (C1-C6)alkyl group; a 5- to 15-membered heteroaryl group; a (C6-C18)aryl group which is unsubstituted or substituted with a cyano group or a tri(C6-C12)arylsilyl group; a tri(C6-C12)arylsilyl group; and a (C1-C6)alkyl(C6-C12)aryl group.

The compound of formula 1 as a first host compound may be selected from the group consisting of following compounds, but is not limited thereto:

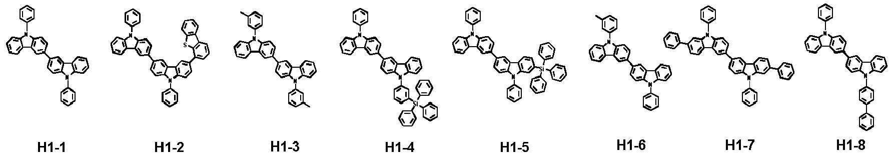

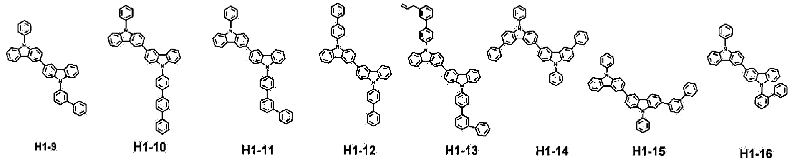

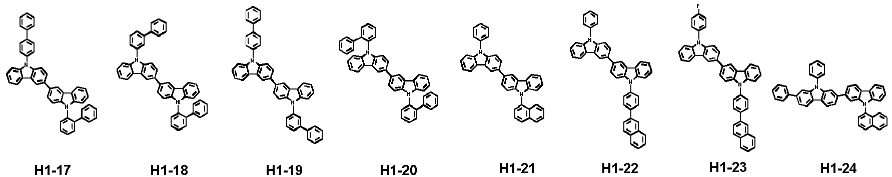

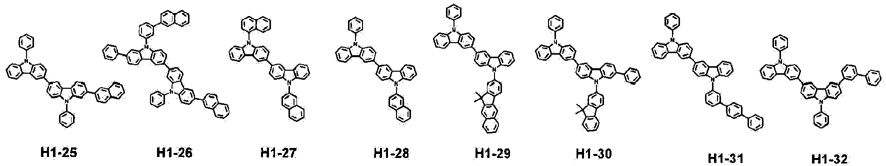

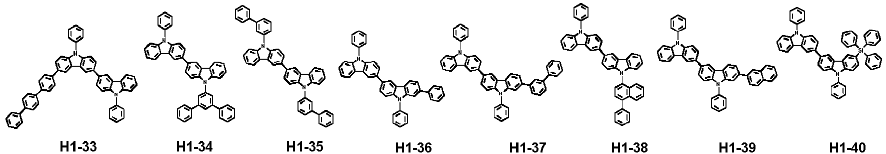

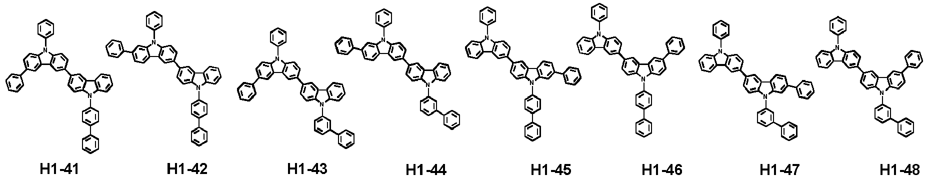

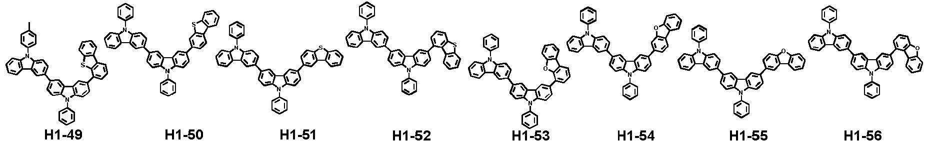

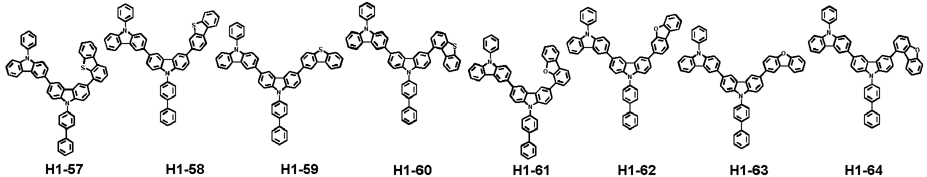

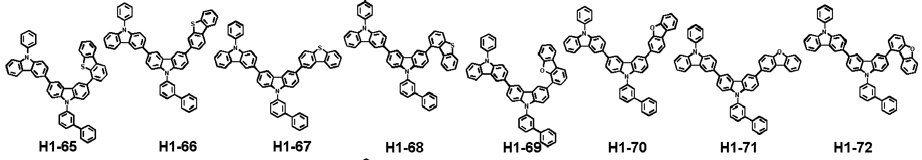

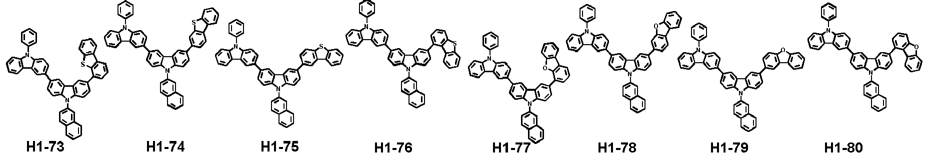

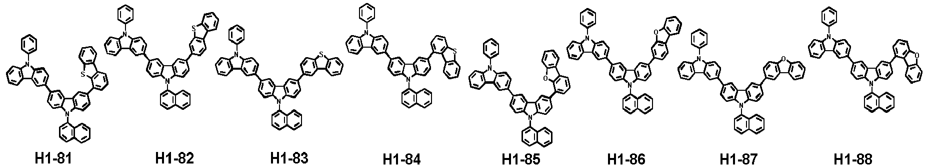

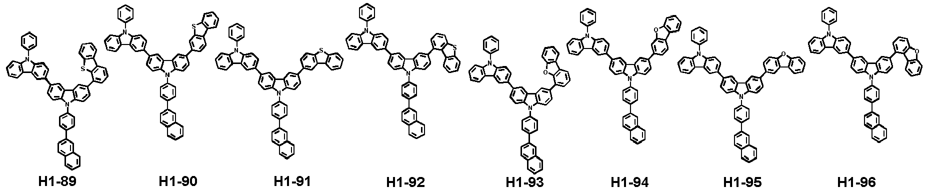

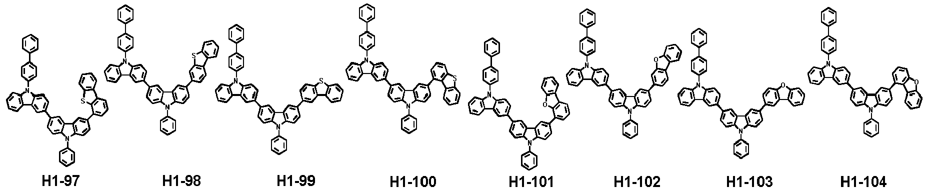

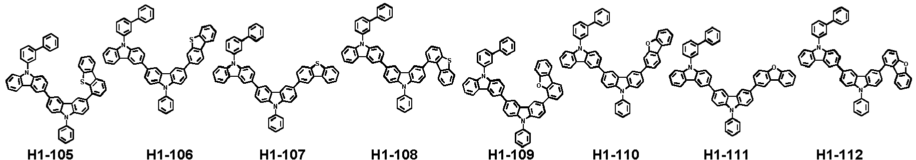

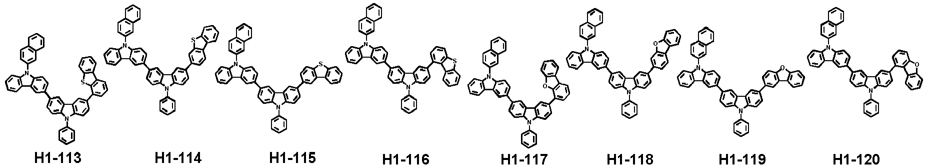

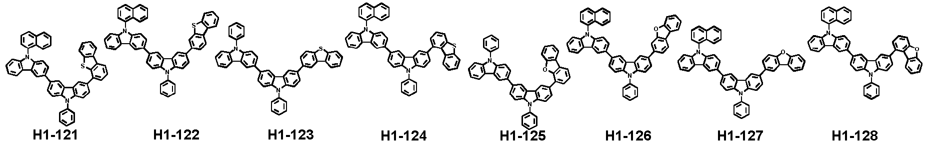

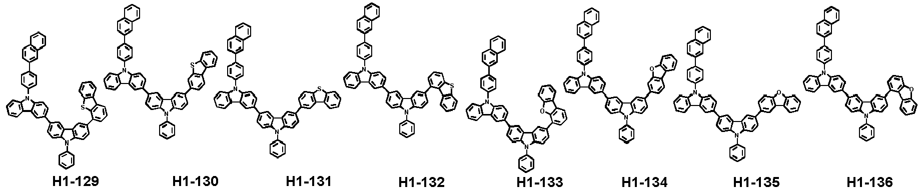

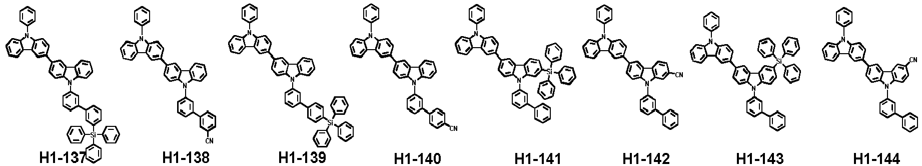

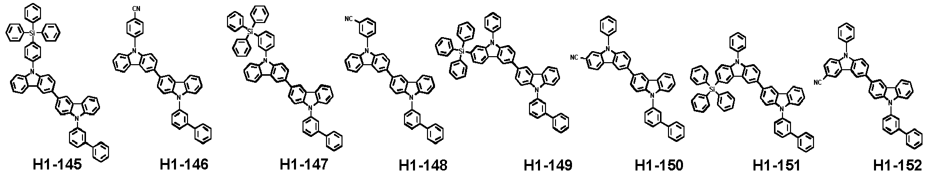

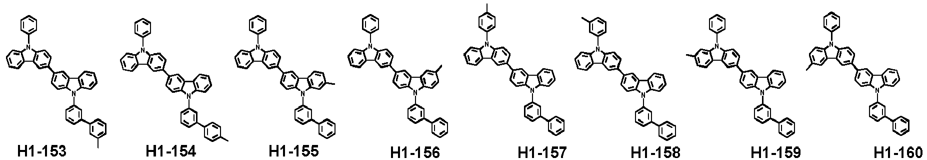

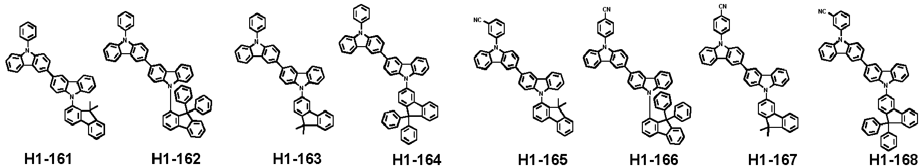

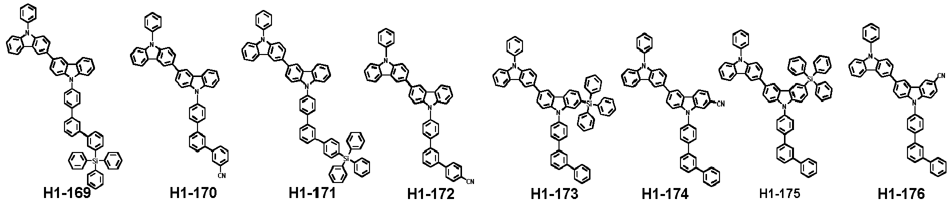

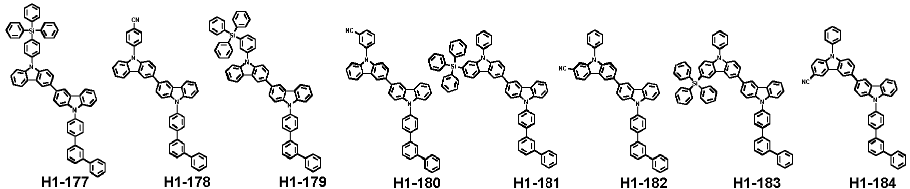

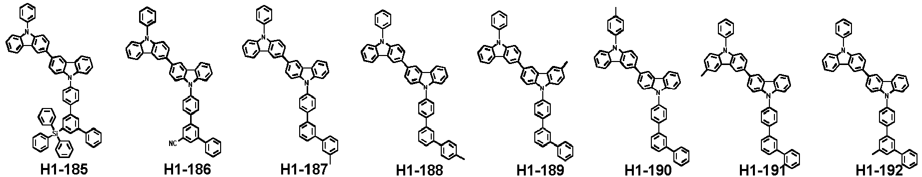

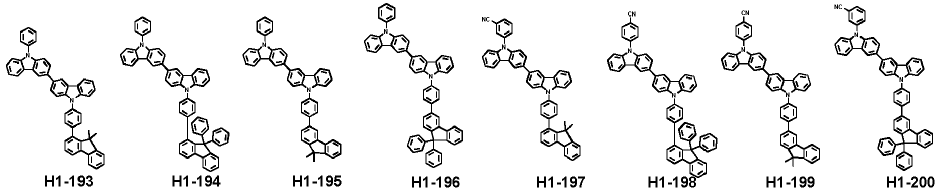

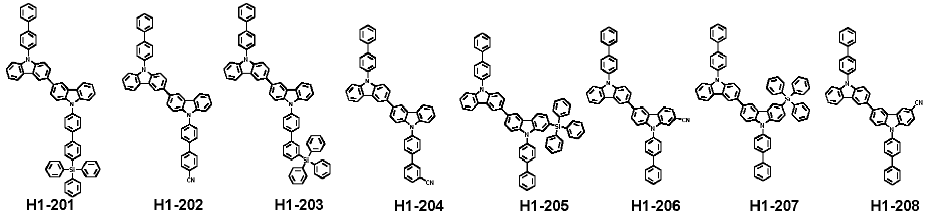

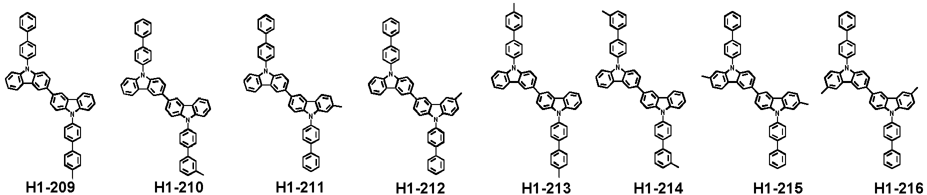

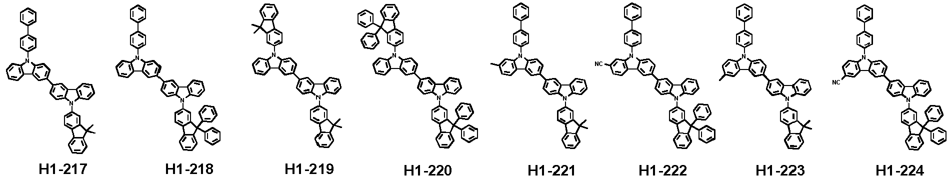

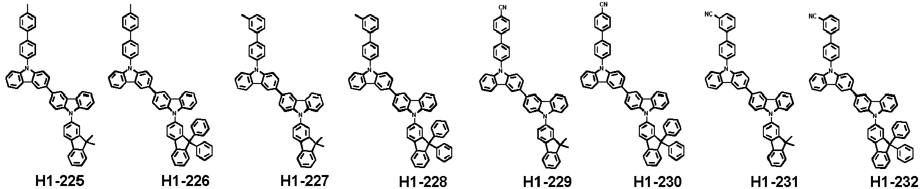

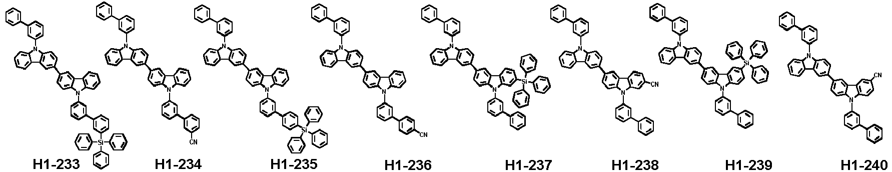

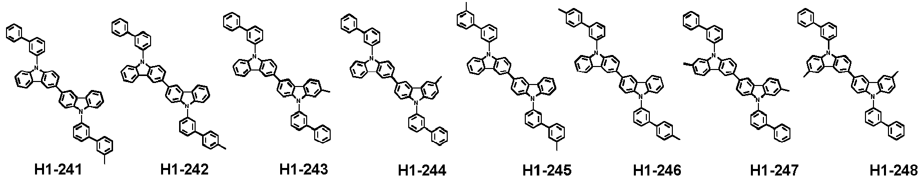

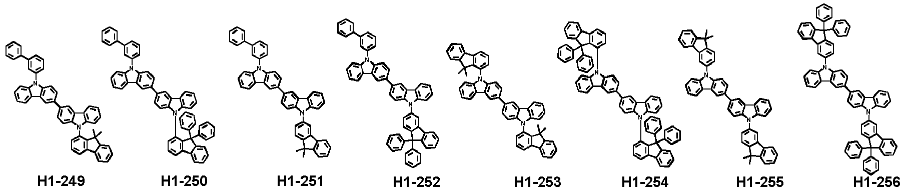

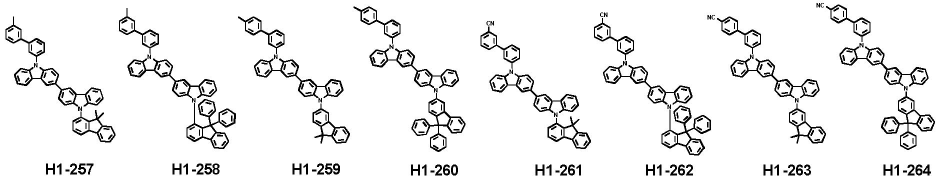

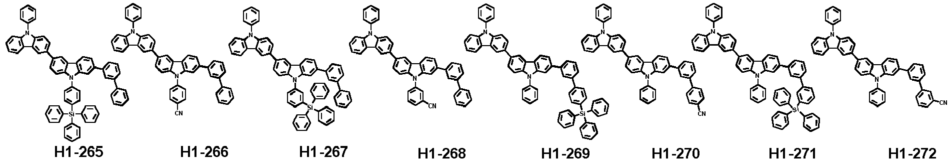

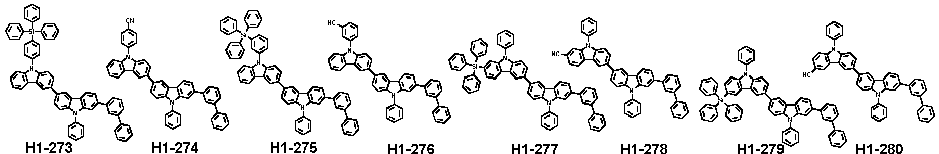

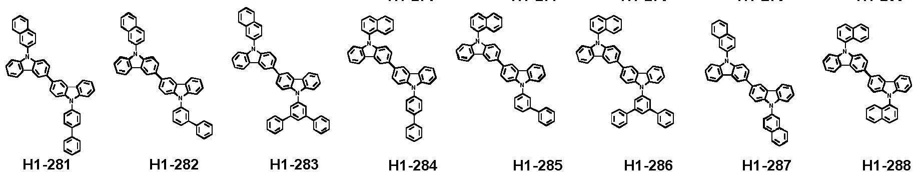

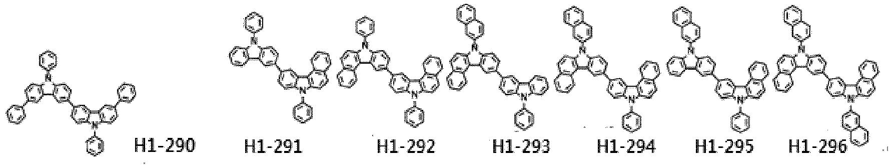

The compound of formula 2 as a second host compound may be selected from the group consisting of following compounds, but is not limited thereto:

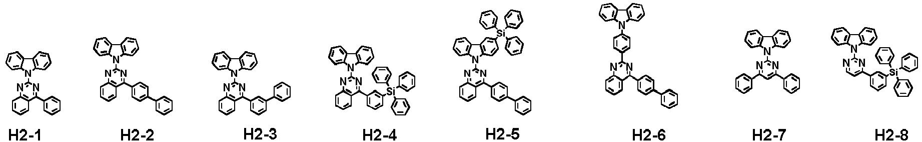

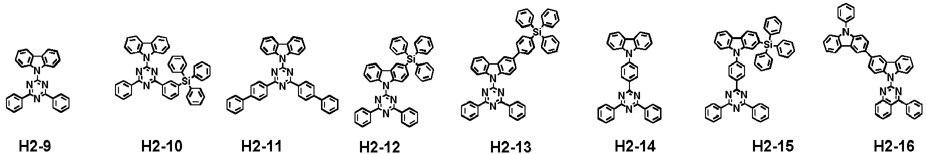

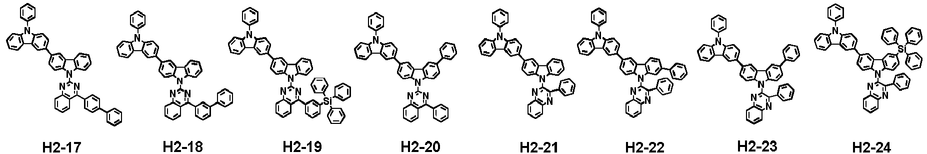

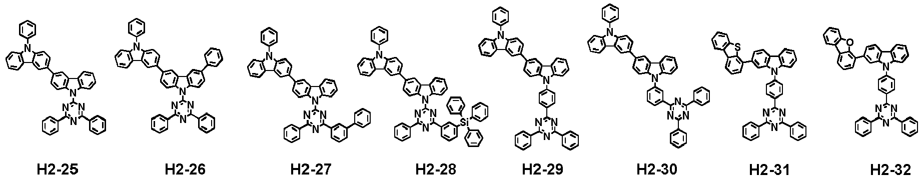

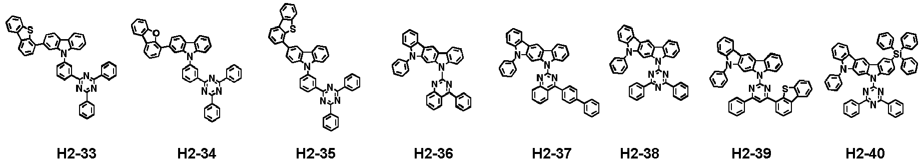

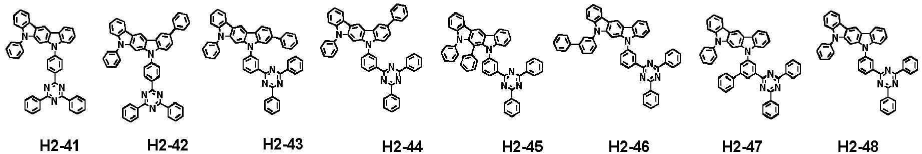

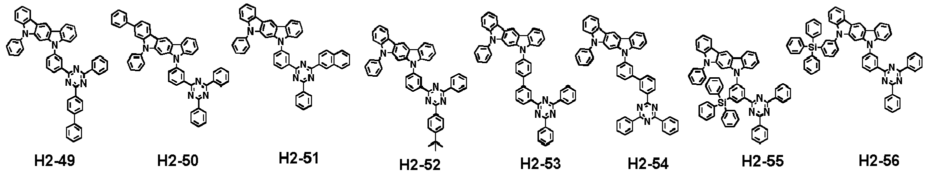

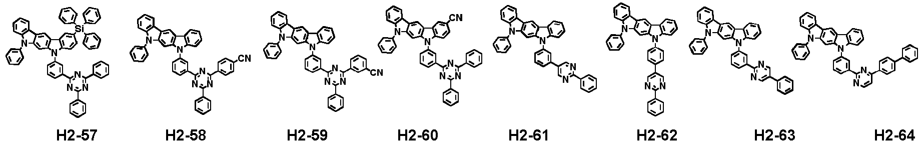

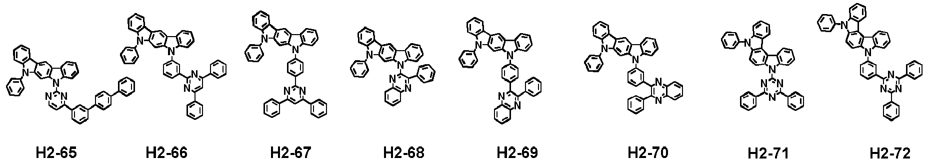

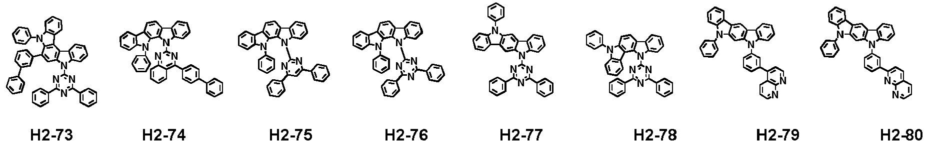

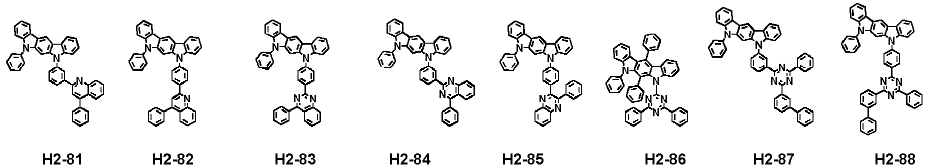

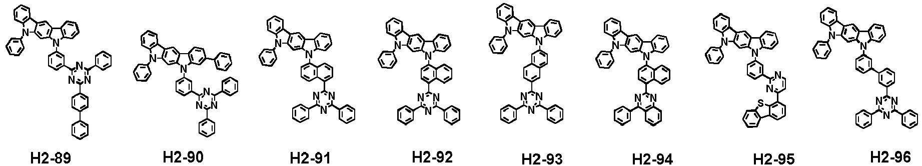

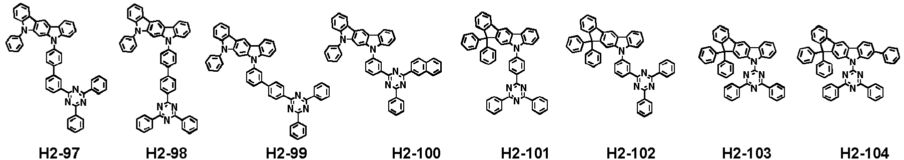

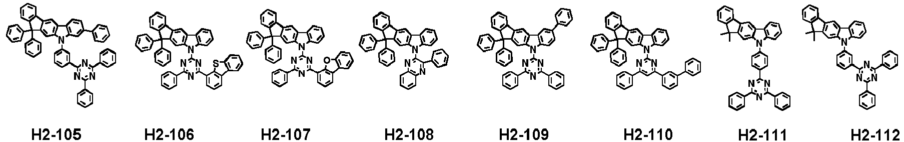

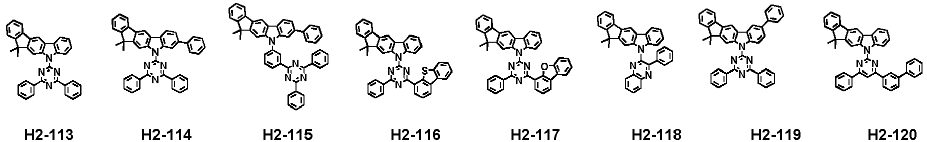

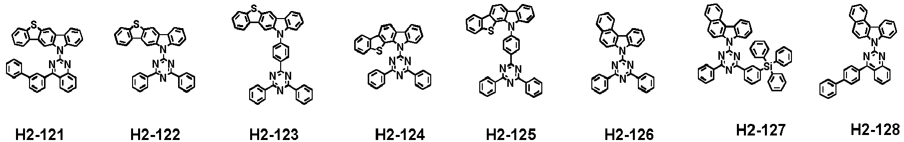

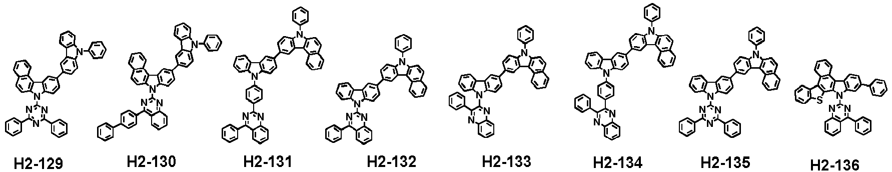

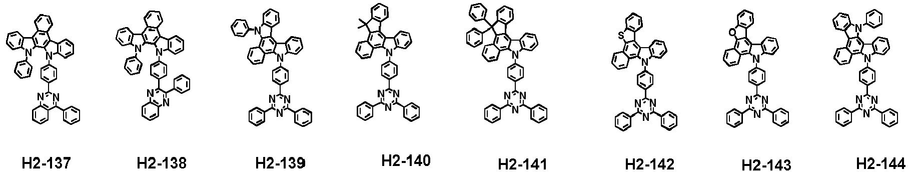

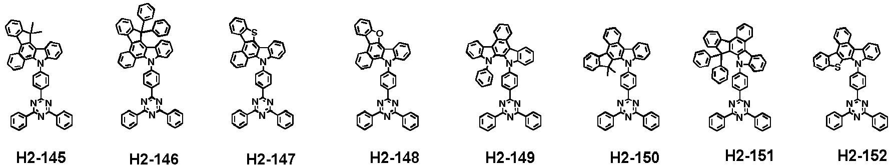

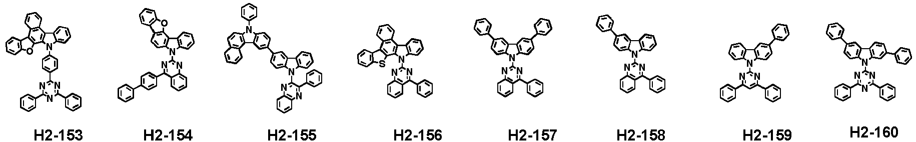

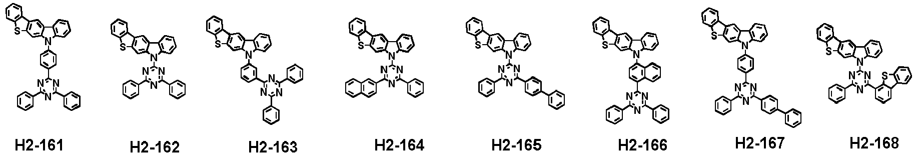

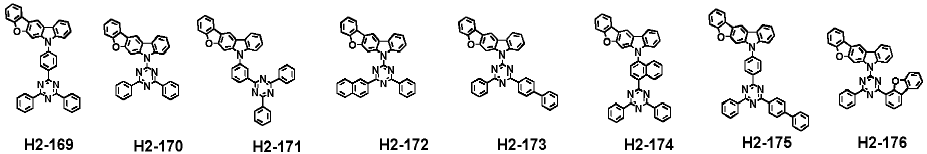

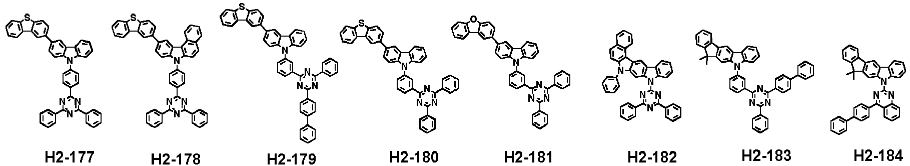

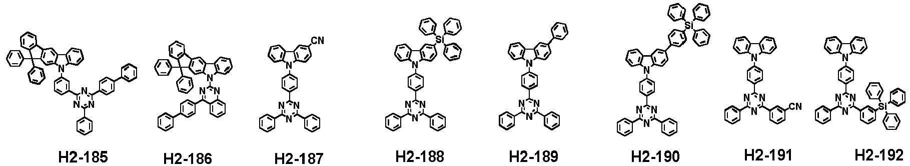

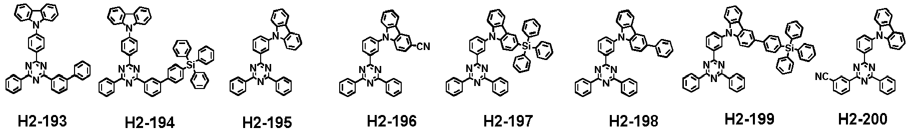

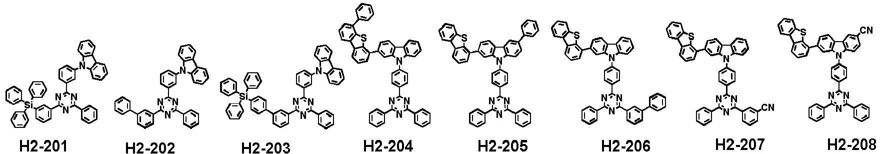

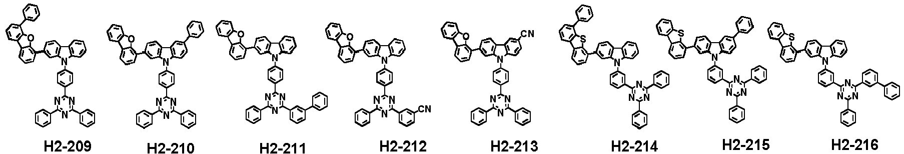

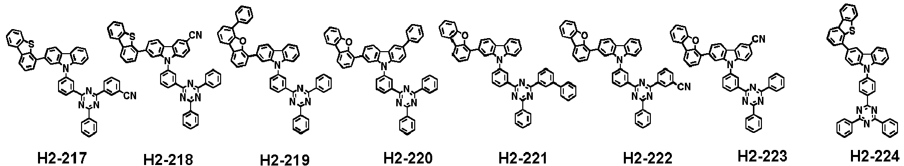

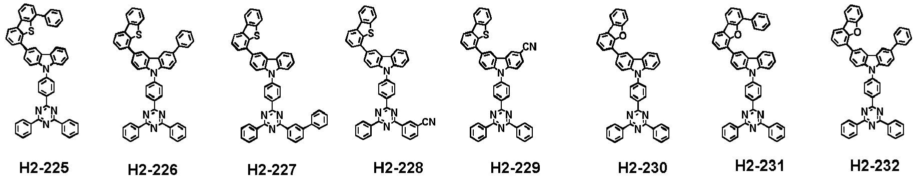

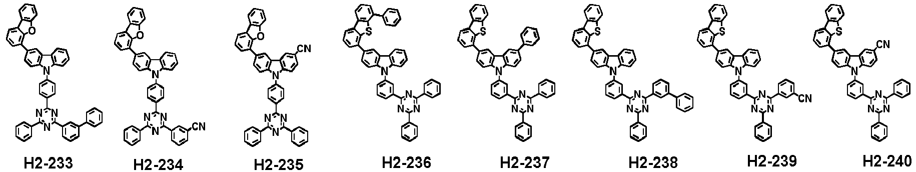

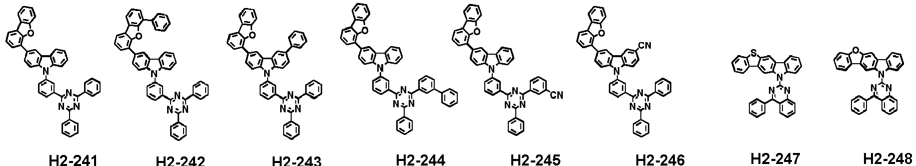

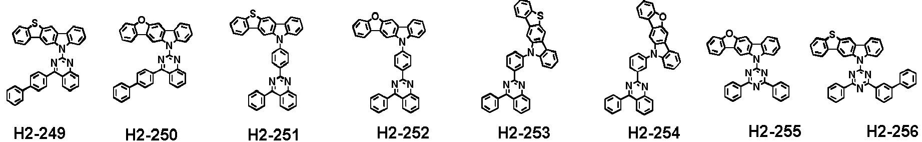

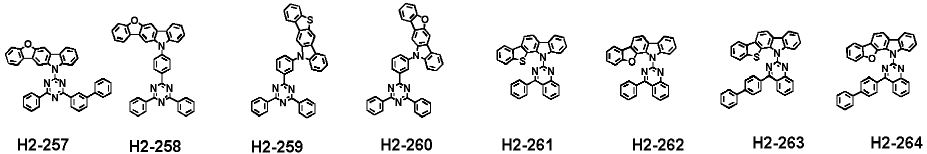

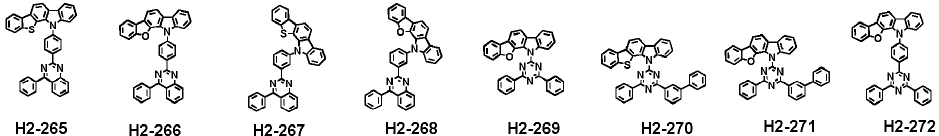

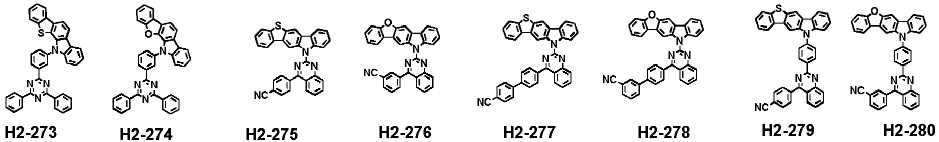

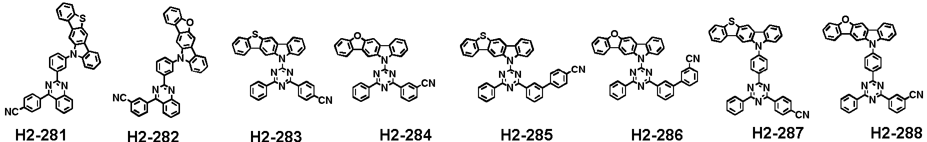

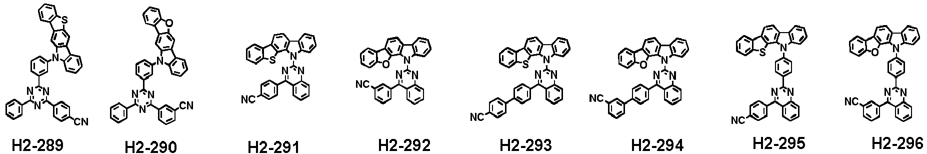

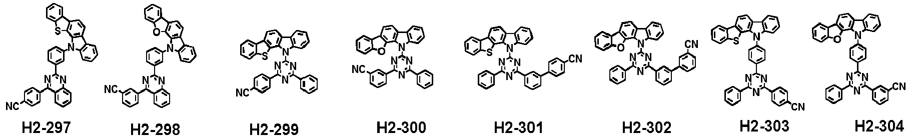

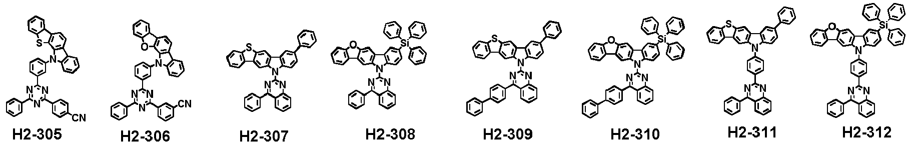

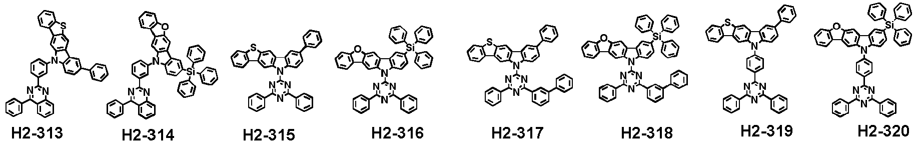

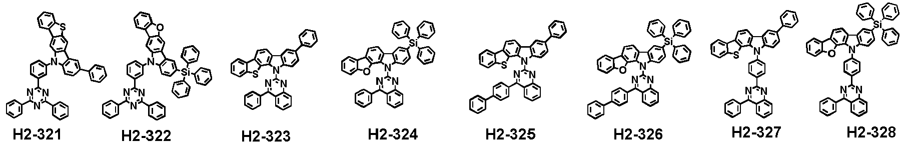

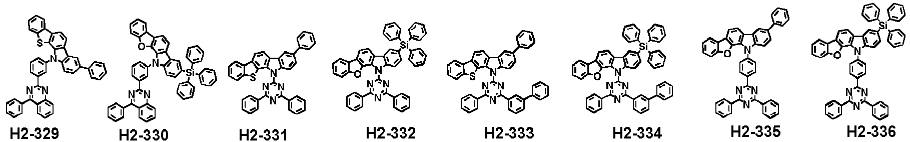

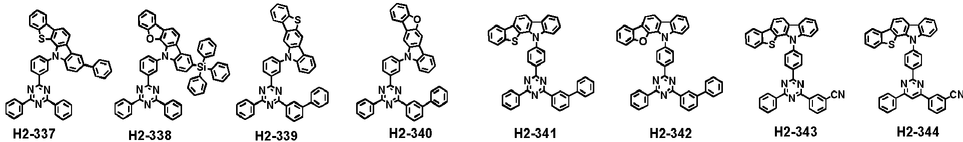

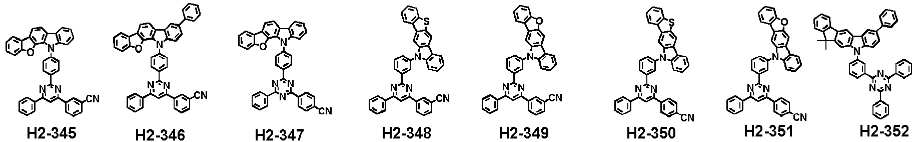

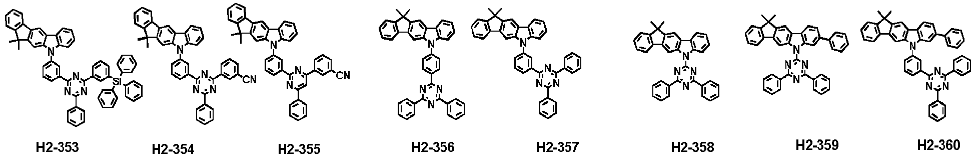

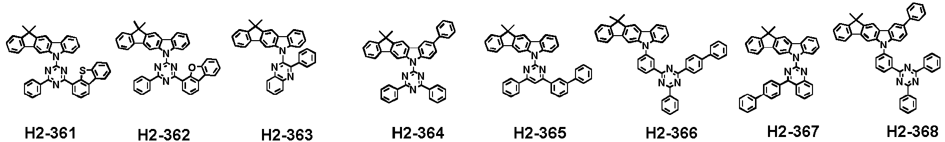

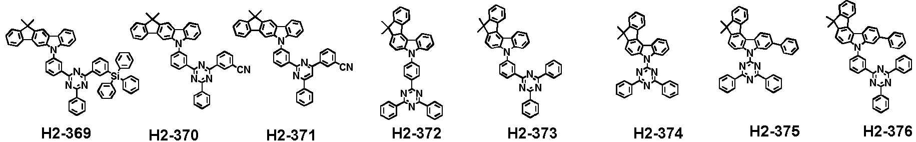

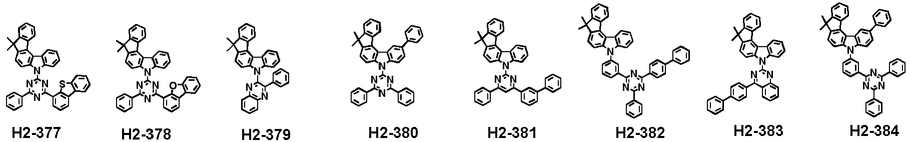

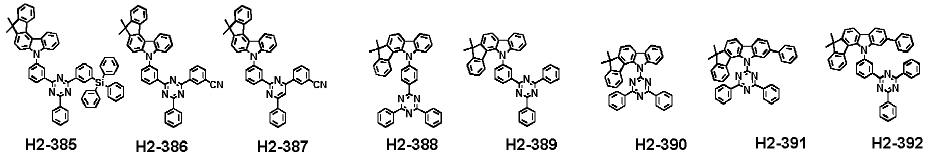

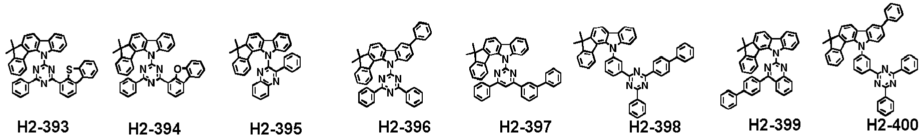

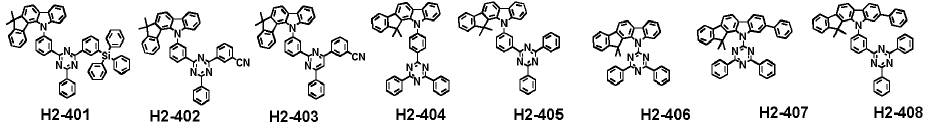

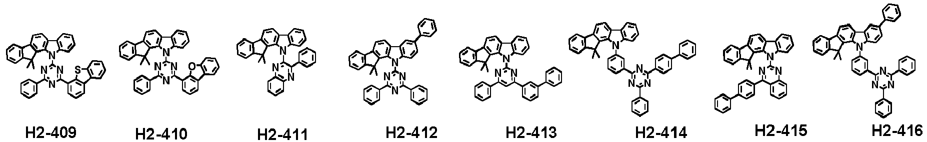

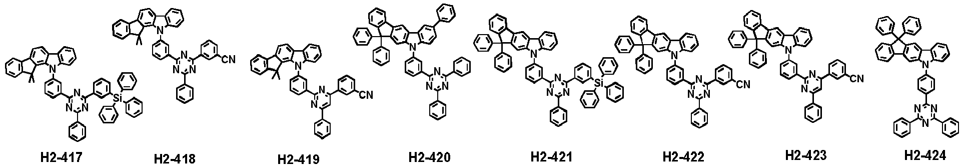

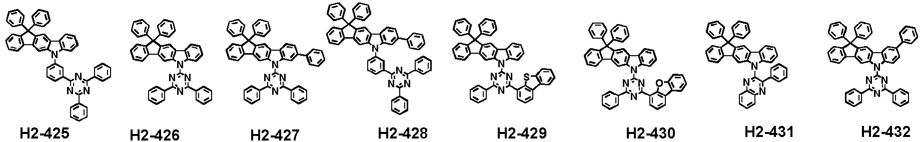

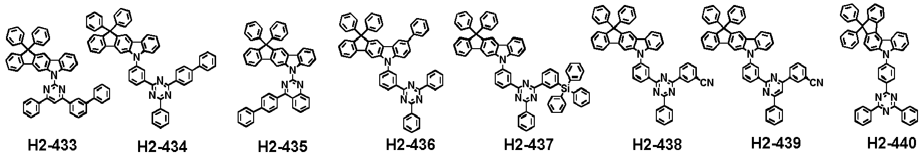

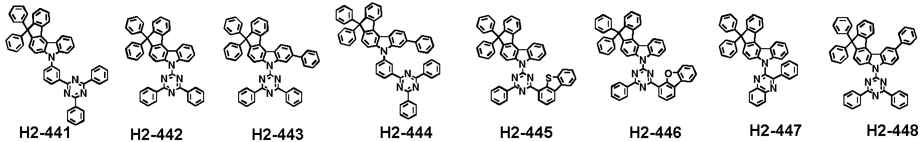

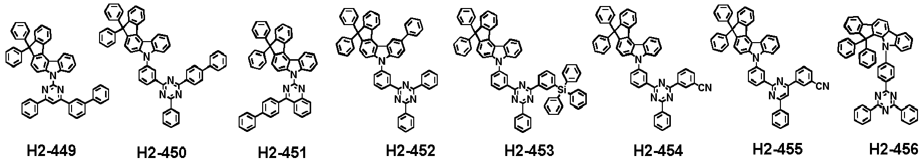

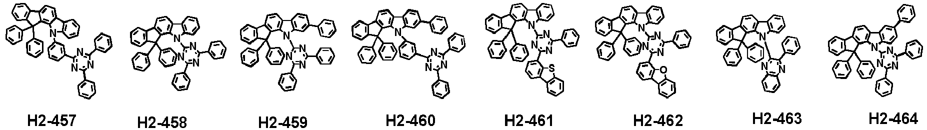

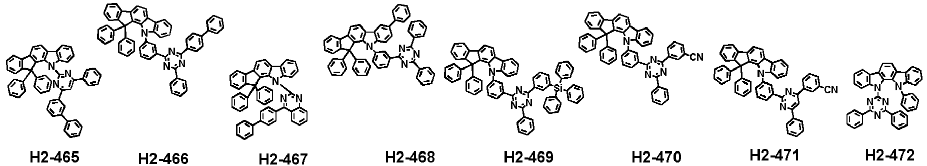

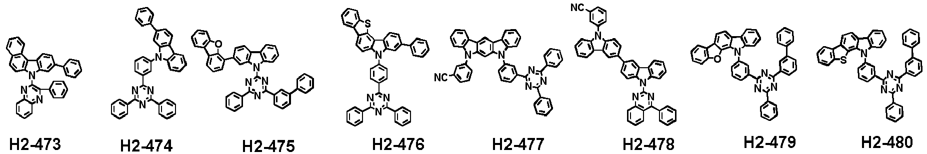

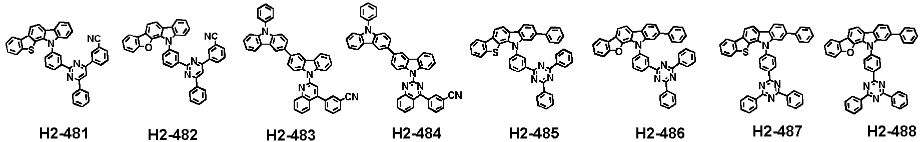

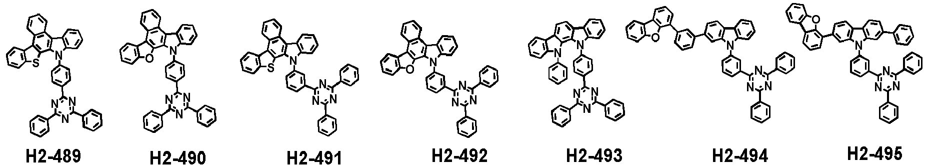

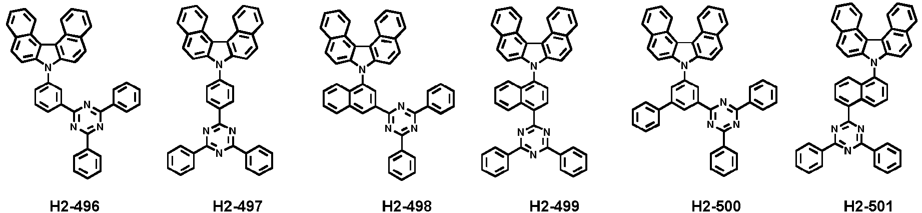

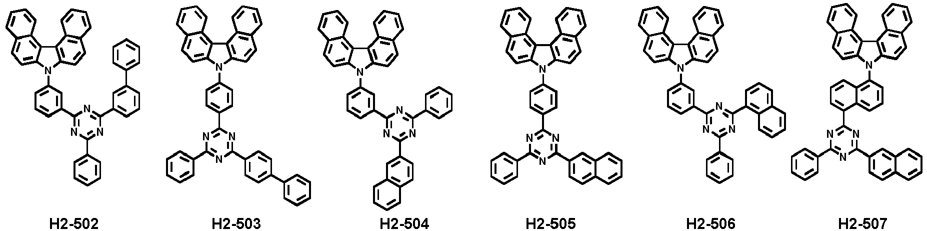

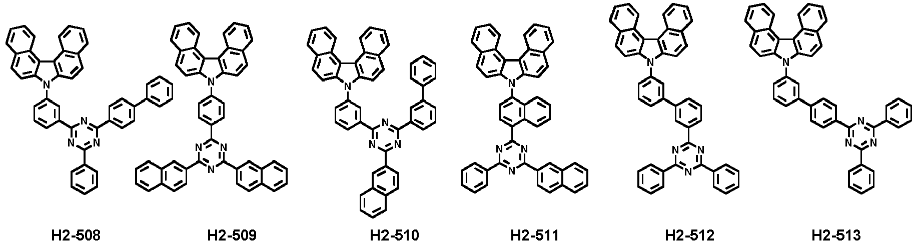

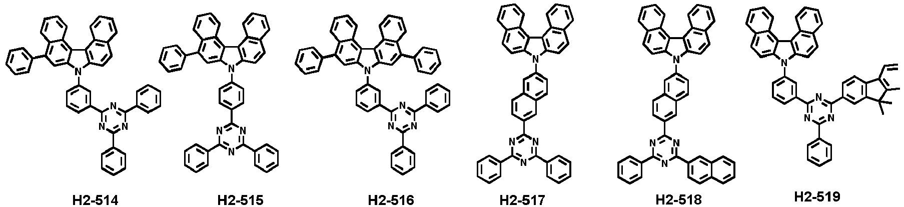

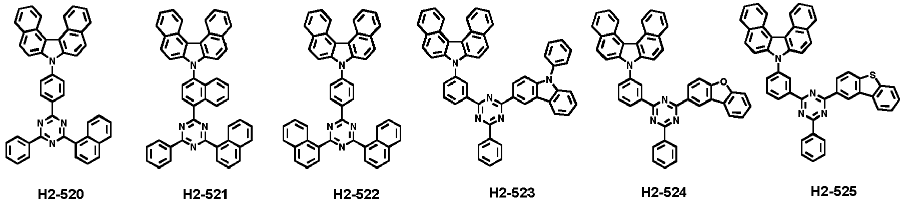

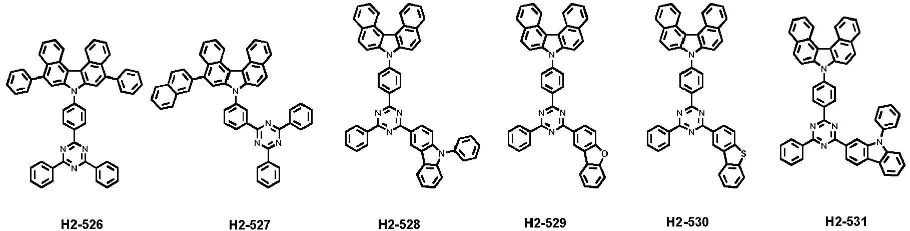

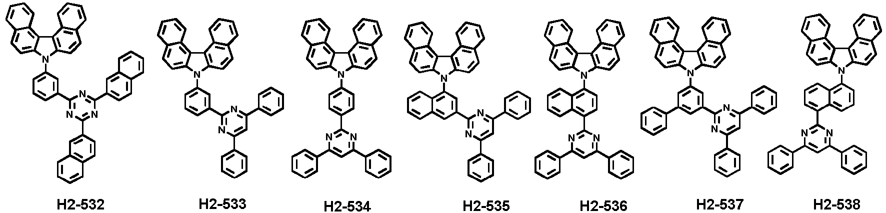

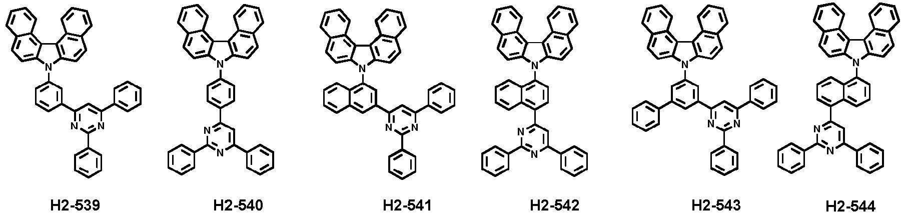

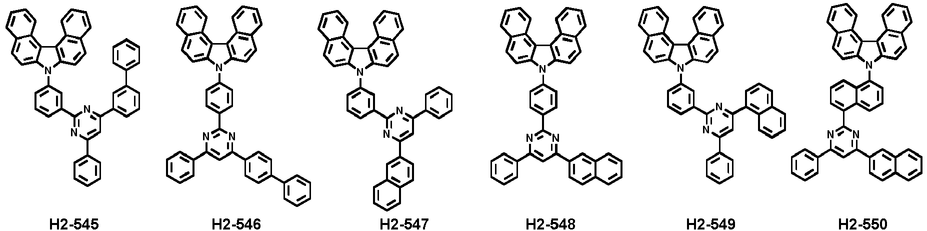

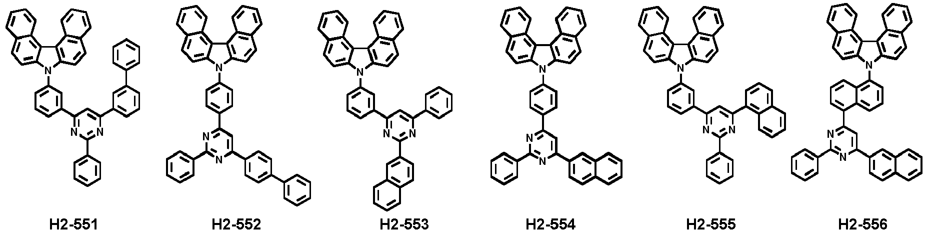

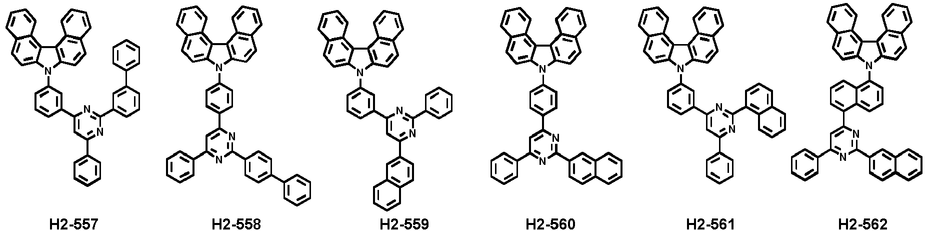

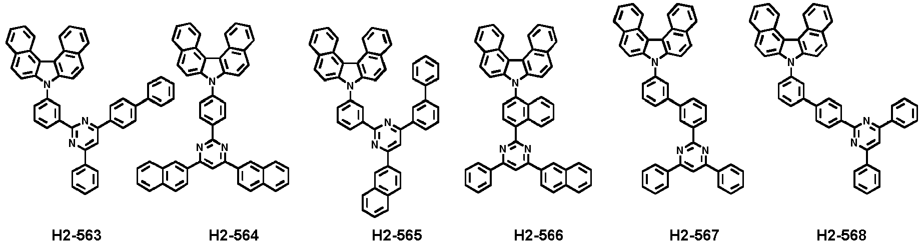

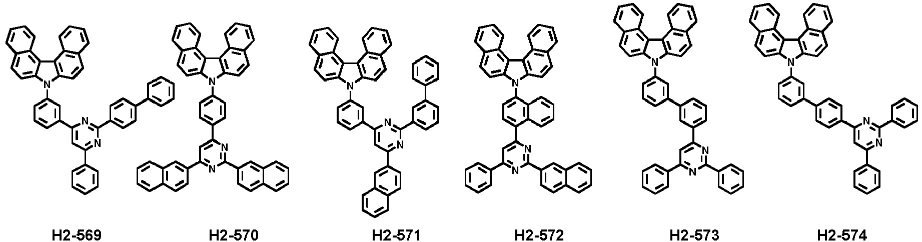

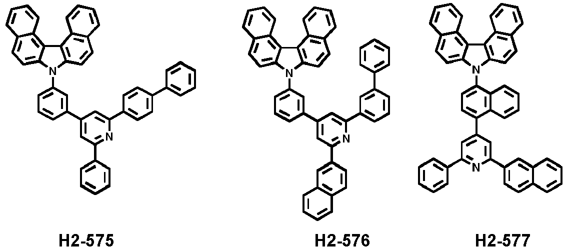

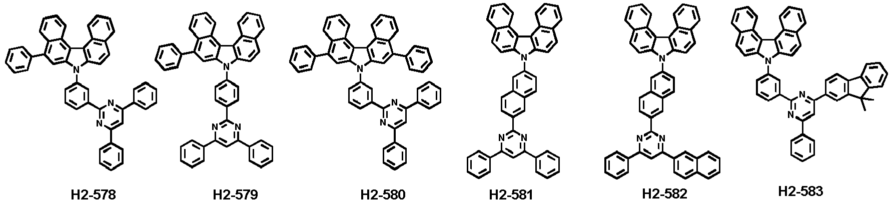

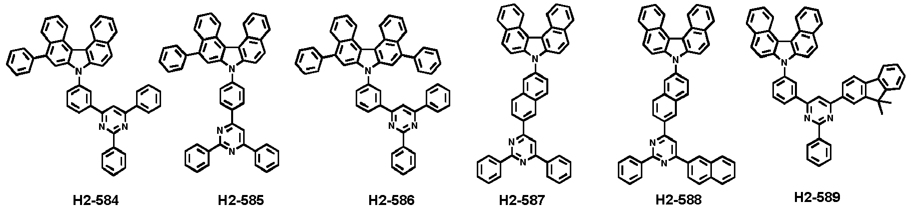

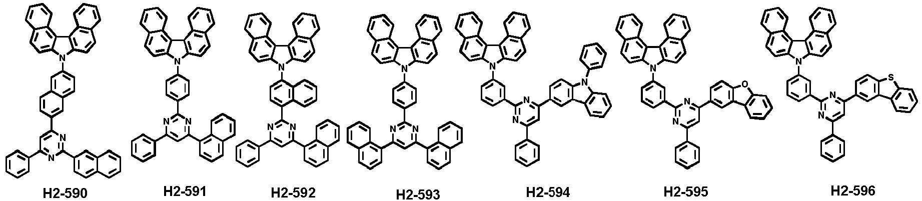

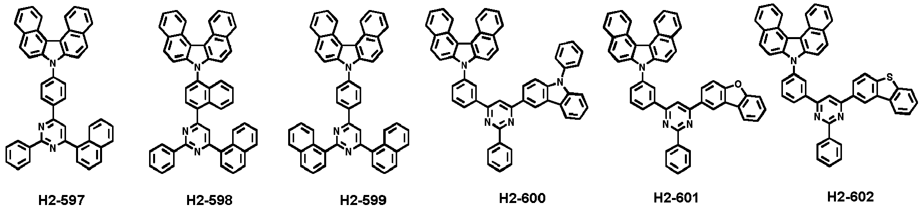

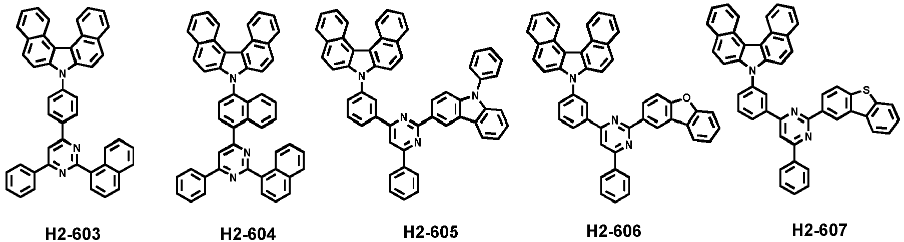

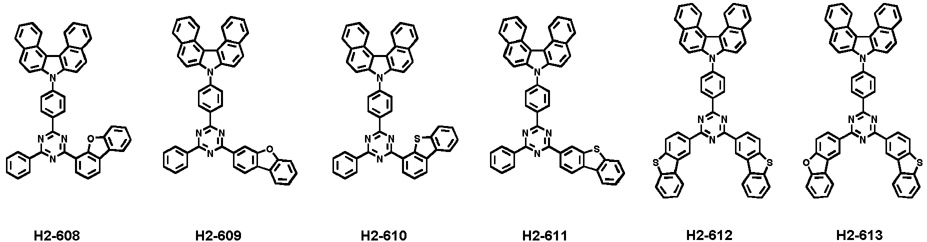

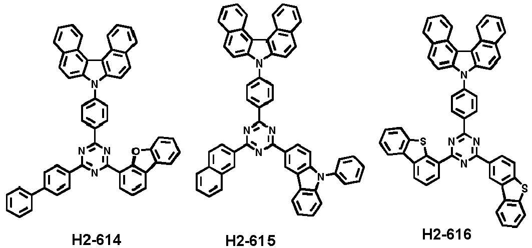

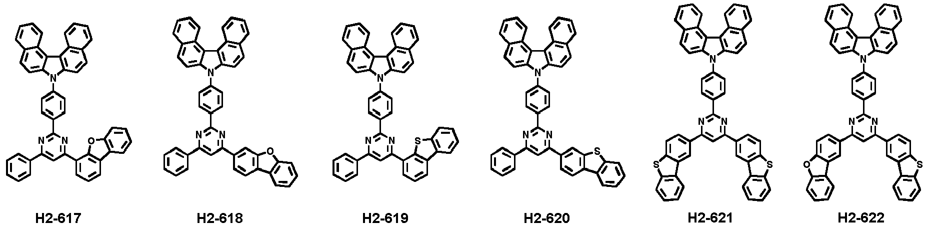

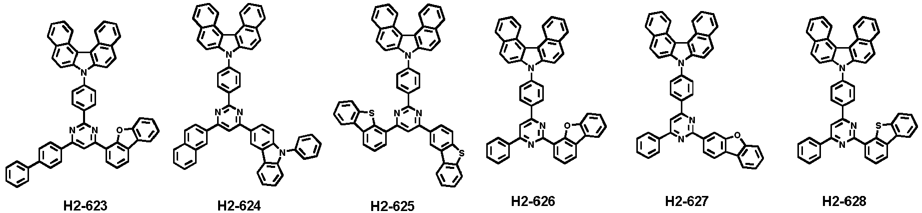

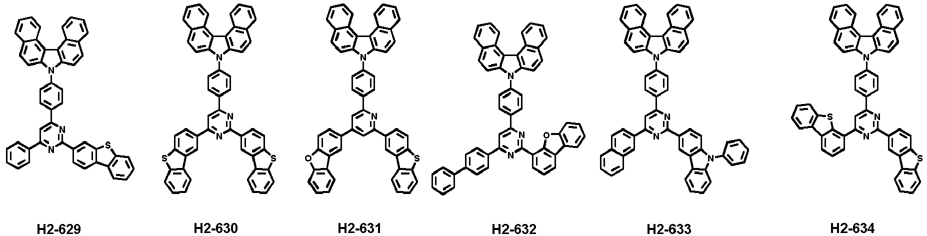

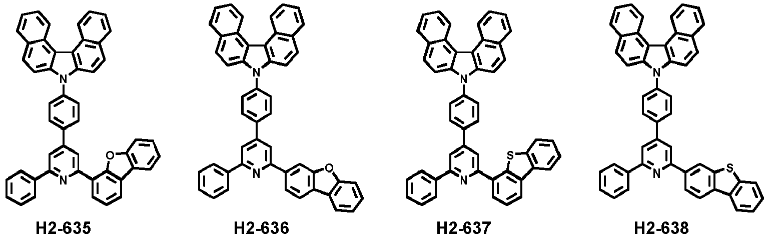

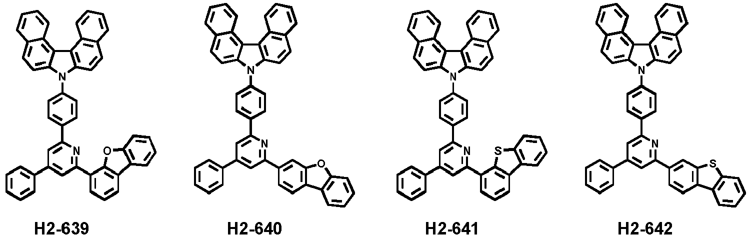

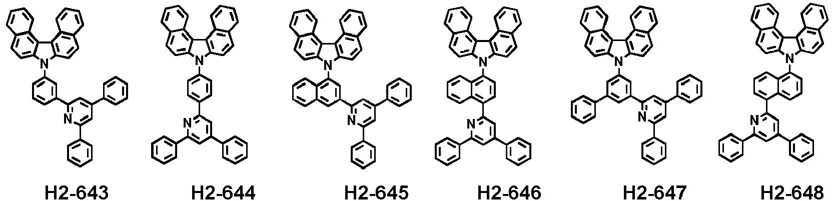

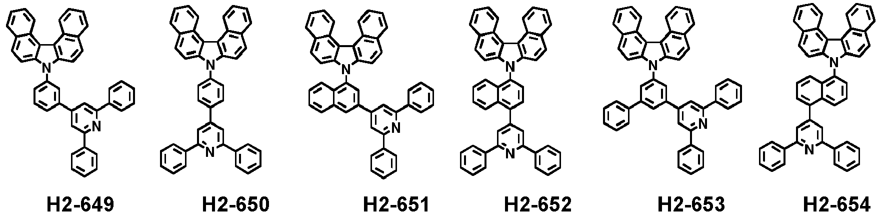

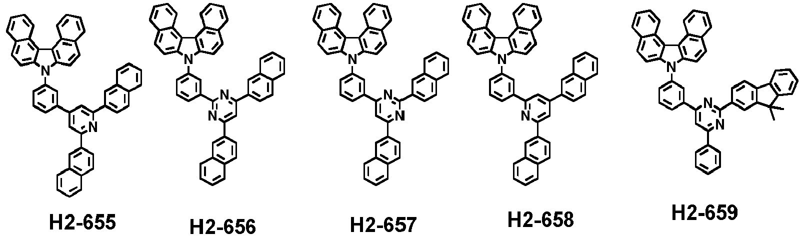

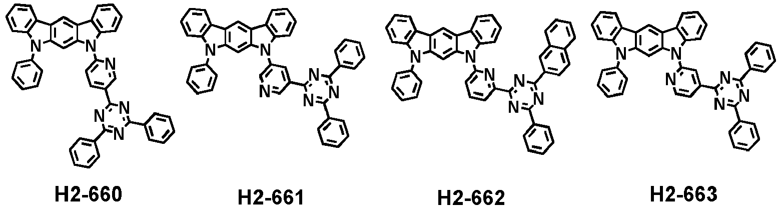

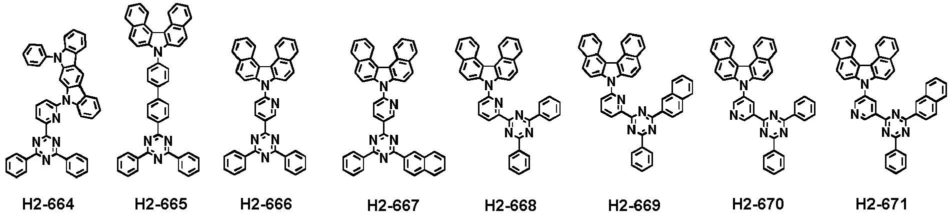

The organic EL device according to the present invention may comprise an anode, a cathode, and at least one organic layer between the two electrodes, wherein the organic layer comprises a light-emitting layer, the light-emitting layer comprises a host and a phosphorescent dopant; the host consists of multi-component host compounds; at least a first host compound of the multi-component host compounds is represented by formula 1 which is a specific bicarbazole derivative containing an aryl group, and a second host compound is represented by formula 2 which is a specific carbazole derivative including a nitrogen-containing heteroaryl group

The light-emitting layer means a layer emitting light and may be a single layer or multi-layers consisting of two or more layers. The doping concentration of dopant compounds to host compounds in the light-emitting layer is preferably less than 20 wt%.

The dopants included in the organic EL device of the present invention are preferably one or more phosphorescent dopants. The phosphorescent dopant material applied to the organic electroluminescent device of the present invention is not specifically limited, but preferably may be selected from complex compounds of iridium (Ir), osmium (Os), copper (Cu), and platinum (Pt), more preferably ortho metallated complex compounds of iridium (Ir), osmium (Os), copper (Cu), and platinum (Pt), and even more preferably ortho metallated iridium complex compounds.

The phosphorescent dopants may be selected from the group consisting of the compounds represented by the following formulae 101 to 103: 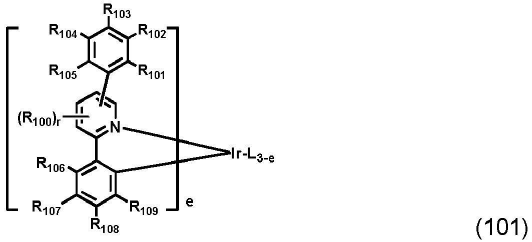 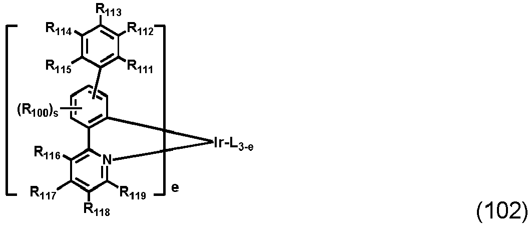 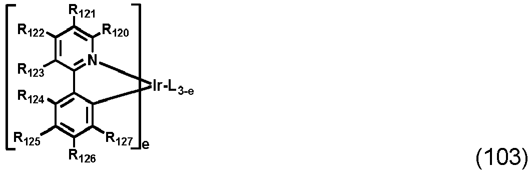 wherein
L is selected from the following structures: 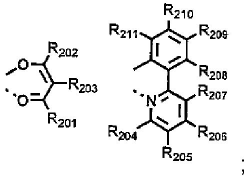 R₁₀₀ represents hydrogen, or a substituted or unsubstituted (C1-C30)alkyl group; R₁₀₁ to R₁₀₉ and R₁₁₁ to R₁₂₃ each independently represent hydrogen, deuterium, a halogen; a (C1-C30)alkyl group unsubstituted or substituted with halogen(s); a cyano group, a substituted or unsubstituted (C1-C30)alkoxy group, a substituted or unsubstituted (C6-C30)aryl group, or a substituted or unsubstituted (C3-C30)cycloalkyl group; R₁₂₀ to R₁₂₃ are linked to an adjacent substituent(s) to form a substituted or unsubstituted mono- or polycyclic, (C3-C30) alicyclic or aromatic ring, for example, quinoline; R₁₂₄ to R₁₂₇ each independently represent hydrogen, deuterium, a halogen, a substituted or unsubstituted (C1-C30)alkyl group, or a substituted or unsubstituted (C6-C30)aryl group; when R₁₂₄ to R₁₂₇ are aryl groups, they are linked to an adjacent substituent(s) to form a substituted or unsubstituted mono- or polycyclic, (C3-C30) alicyclic or aromatic ring, or a heteroaromatic ring, for example, fluorene, dibenzothiophene, or dibenzofuran; R₂₀₁ to R₂₁₁ each independently represent hydrogen, deuterium, a halogen, a (C1-C30)alkyl group unsubstituted or substituted with halogen(s), or a substituted or unsubstituted (C6-C30)aryl group; R₂₀₈ to R₂₁₁ may be linked to an adjacent substituent(s) to form a substituted or unsubstituted mono- or polycyclic, (C3-C30) alicyclic or aromatic ring, or a heteroaromatic ring, for example, fluorene, dibenzothiophene, or dibenzofuran; r and s each independently represent an integer of 1 to 3; where r or s is an integer of 2 or more, each of R₁₀₀ may be the same or different; and e represents an integer of 1 to 3.

The phosphorescent dopant material includes the followin:

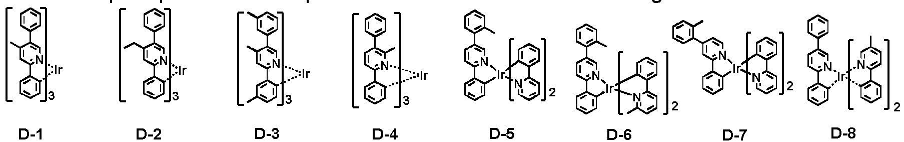

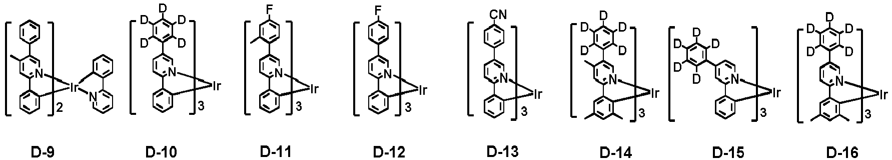

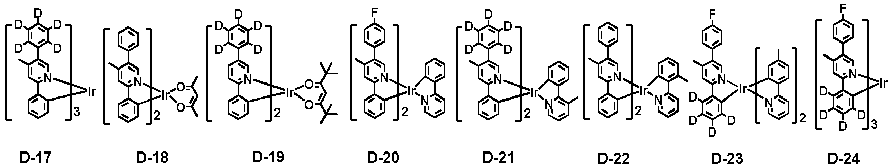

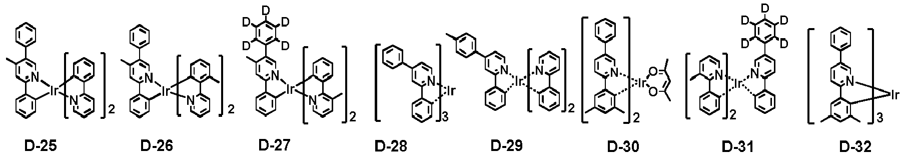

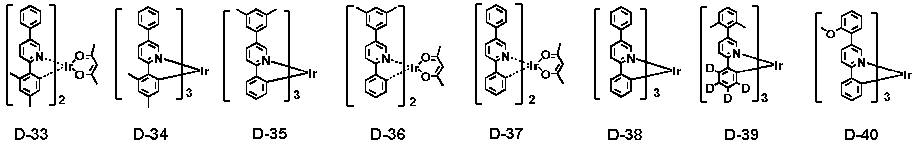

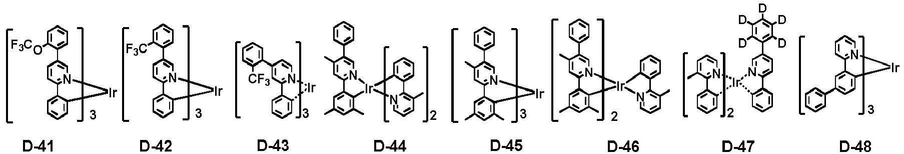

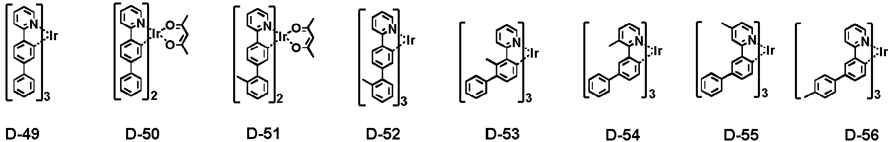

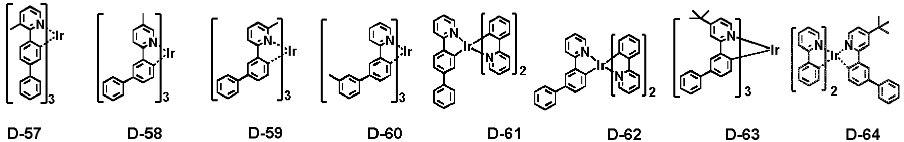

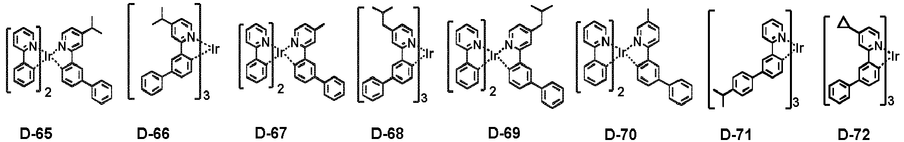

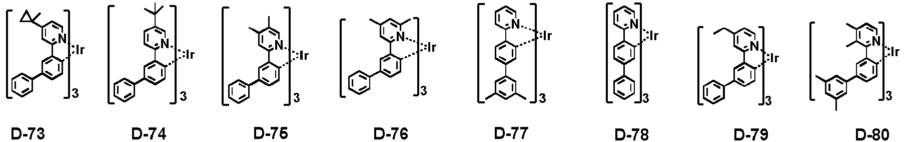

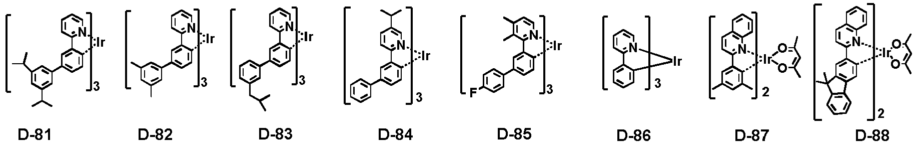

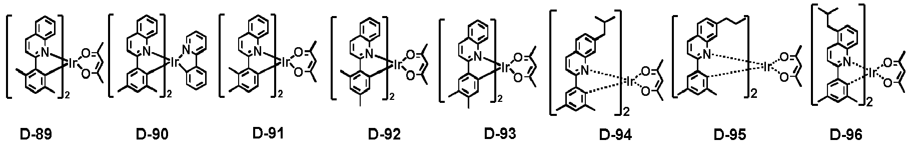

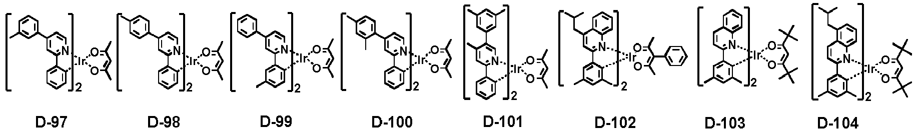

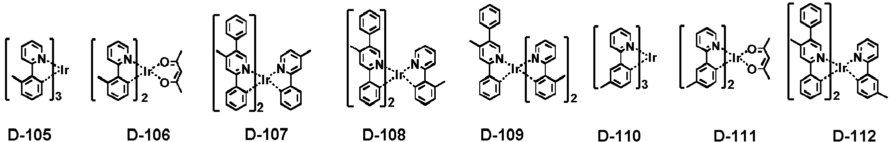

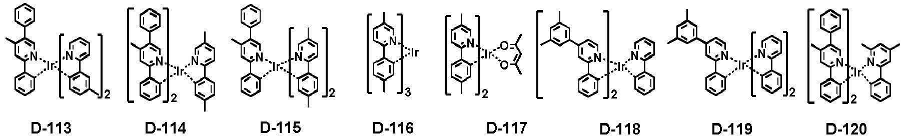

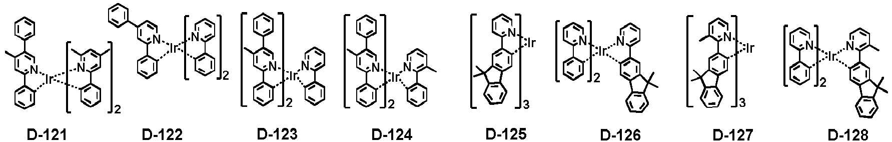

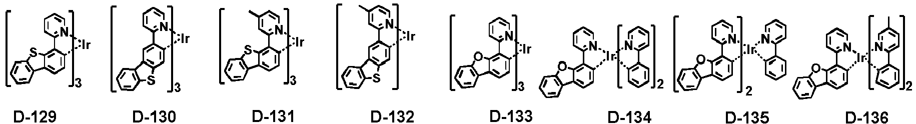

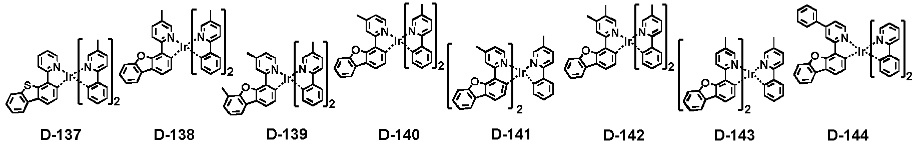

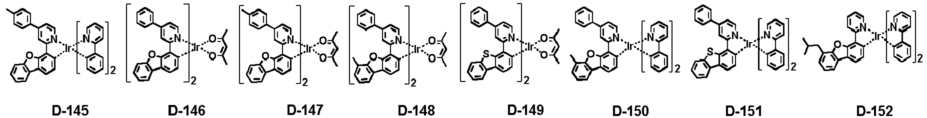

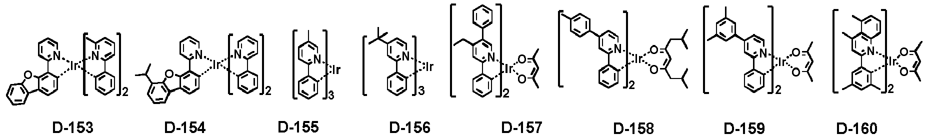

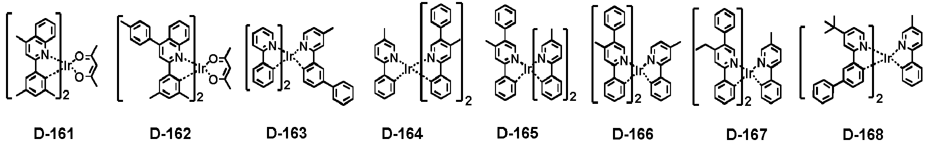

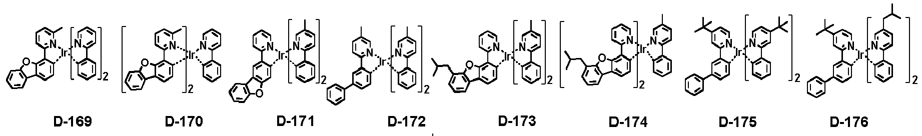

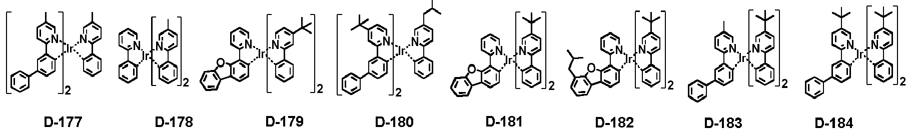

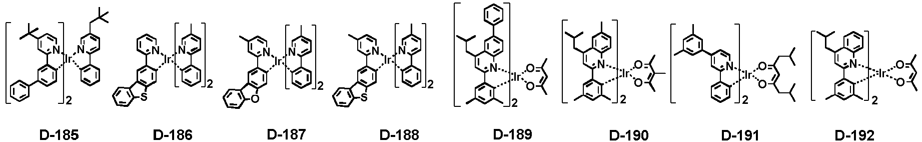

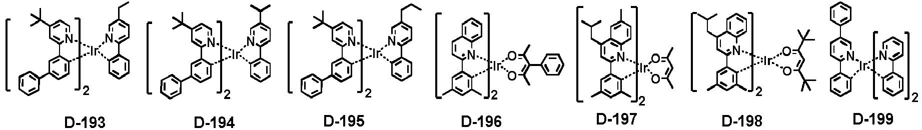

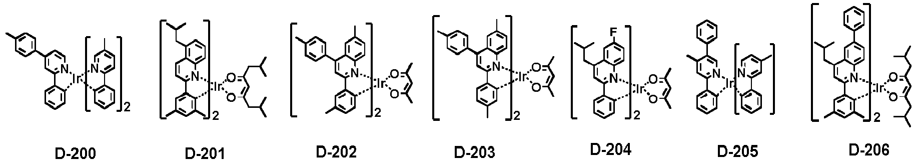

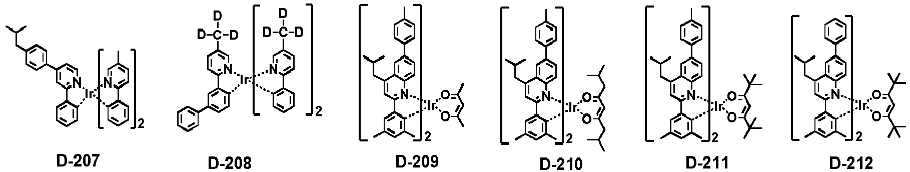

The organic EL device of the present invention may further include at least one compound selected from the group consisting of arylamine-based compounds and styrylarylamine-based compounds in the organic layer.

In the organic EL device of the present invention, the organic layer may further comprise at least one metal selected from the group consisting of metals of Group 1, metals of Group 2, transition metals of the 4^{th} period, transition metals of the 5^{th} period, lanthanides, and organic metals of d-transition elements of the Periodic Table, or at least one complex compound comprising the metal.

Preferably, in the organic electroluminescent device of the present invention, at least one layer (hereinafter, "a surface layer") selected from a chalcogenide layer, a metal halide layer and a metal oxide layer may be placed on an inner surface(s) of one or both electrode(s). Specifically, it is preferred that a chalcogenide (including oxides) layer of silicon or aluminum is placed on an anode surface of a light-emitting medium layer, and a metal halide layer or metal oxide layer is placed on a cathode surface of an electroluminescent medium layer. The surface layer provides operating stability for the organic electroluminescent device. Preferably, the chalcogenide includes SiO_{X}(1≤X≤2), AlO_{X}(1≤X≤1.5), SiON, SiAlON, etc.; the metal halide includes LiF, MgF₂, CaF₂, a rare earth metal fluoride, etc.; and the metal oxide includes Cs₂O, Li₂O, MgO, SrO, BaO, CaO, etc.

A hole injection layer, a hole transport layer, an electron blocking layer, or their combinations can be used between an anode and a light-emitting layer. The hole injection layer may be multi-layers in order to lower a hole injection barrier (or hole injection voltage) from an anode to a hole transport layer or an electron blocking layer, wherein each of the multi-layers simultaneously uses two compounds. The hole transport layer or the electron blocking layer may also be multi-layers.

An electron buffer layer, a hole blocking layer, an electron transport layer, an electron injection layer, or their combinations can be used between a light-emitting layer and a cathode. The electron buffer layer may be multi-layers in order to control the injection of an electron and improve interface properties between the light-emitting layer and the electron injection layer, wherein each of the multi-layers simultaneously uses two compounds. The hole blocking layer or the electron transport layer may also be multi-layers, wherein each of the multi-layers may use a multi-component of compounds.

Preferably, in the organic electroluminescent device of the present invention, a mixed region of an electron transport compound and a reductive dopant, or a mixed region of a hole transport compound and an oxidative dopant may be placed on at least one surface of a pair of electrodes. In this case, the electron transport compound is reduced to an anion, and thus it becomes easier to inject and transport electrons from the mixed region to a light-emitting medium. Further, the hole transport compound is oxidized to a cation, and thus it becomes easier to inject and transport holes from the mixed region to a light-emitting medium. Preferably, the oxidative dopant includes various Lewis acids and acceptor compounds; and the reductive dopant includes alkali metals, alkali metal compounds, alkaline earth metals, rare-earth metals, and mixtures thereof. A reductive dopant layer may be employed as a charge-generating layer to prepare an organic electroluminescent device having two or more light-emitting layers and emitting white light.

In order to form each layer constituting the organic electroluminescent device of the present invention, dry film-forming methods, such as vacuum deposition, sputtering, plasma, ion plating methods, etc., or wet film-forming methods, such as spin coating, dip coating, flow coating methods, etc., can be used. When forming a layer by using a first host and a second host according to the present invention, co-deposition or mixed-deposition may be used.

When using a wet film-forming method, a thin film is formed by dissolving or dispersing the material constituting each layer in suitable solvents, such as ethanol, chloroform, tetrahydrofuran, dioxane, etc. The solvents are not specifically limited as long as the material constituting each layer is soluble or dispersible in the solvents, which do not cause any problems in forming a layer.

Furthermore, a display device or a light device can be produced by using the organic EL device of the present invention.

Hereinafter, the preparation methods of devices by using host compounds and dopant compounds of the present invention will be explained in detail with reference to the following examples:

### Device Example 1-1: Production of an OLED device by co-deposition of the first host compound and the second host compound according to the present invention as a host

An OLED device comprising the organic electroluminescent compound of the present invention was produced as follows: A transparent electrode indium tin oxide (ITO) thin film (10 Ω/sq) on a glass substrate for an OLED device (Samsung Corning, Republic of Korea) was subjected to an ultrasonic washing with trichloroethylene, acetone, ethanol, and distilled water, sequentially, and was then stored in isopropanol. Next, the ITO substrate was mounted on a substrate holder of a vacuum vapor depositing apparatus. N⁴,N^{4'}-diphenyl-N⁴,N^{4'}-bis(9-phenyl-9H-carbazole-3-yl)-[1,1'-biphenyl]-4,4'-diamine as HI-1 was introduced into a cell of the vacuum vapor depositing apparatus, and the pressure in the chamber of the apparatus was then controlled to 10⁻⁶ torr. Thereafter, an electric current was applied to the cell to evaporate the introduced material, thereby forming a hole injection layer 1 having a thickness of 80 nm on the ITO substrate. 1,4,5,8,9,12-hexaazatriphenylene hexacarbonitrile as HI-2 was then introduced into another cell of the vacuum vapor depositing apparatus, and an electric current was applied to the cell to evaporate the introduced material, thereby forming a hole injection layer 2 having a thickness of 5 nm on hole injection layer 1. N-([1,1'-biphenyl]-4-yl)-9,9-dimethyl-N-(4-(9-phenyl-9H-carbazole-3-yl)phenyl)-9H-fluorene-2-amine as HT-1 was introduced into one cell of the vacuum vapor depositing apparatus. Thereafter, an electric current was applied to the cell to evaporate the introduced material, thereby forming a hole transport layer 1 having a thickness of 10 nm on hole injection layer 2. N,N-di([1,1'-biphenyl]-4-yl)-4'-(9H-carbazole-9-yl)-[1,1'-biphenyl]-4-amine as HT-2 was then introduced into another cell of the vacuum vapor depositing apparatus, and an electric current was applied to the cell to evaporate the introduced material, thereby forming a hole transport layer 2 having a thickness of 60 nm on hole transport layer 1. Thereafter, compounds H1-1 and H2-2 as hosts were respectively introduced into two cells of the vacuum vapor depositing apparatus and compound D-96 as a dopant was introduced into another cell. The two host materials were evaporated at the same rates of 1:1, and the dopant was evaporated at a different rate and deposited in a doping amount of 3 wt%, based on the total weight of the host and dopant, to form a light-emitting layer having a thickness of 40 nm on the hole transport layer. Next, 2,4-bis(9,9-dimethyl-9H-fluorene-2yl)-6-(naphthalene-2-yl)-1,3,5-triazine as ET-1 and lithium quinolate as El-1 were evaporated at the same rates of 1:1 on another two cells to form an electron transport layer having a thickness of 30 nm on the light-emitting layer. After depositing lithium quinolate of El-1 having a thickness of 2 nm as an electron injection layer on the electron transport layer, an Al cathode having a thickness of 80 nm was then deposited by another vacuum vapor deposition apparatus on the electron injection layer. Thus, an OLED device was produced.

The produced OLED device showed the driving voltage at a luminance of 1,000nit, luminescent efficiency, CIE color coordinate, and the lifespan taken to be reduced from 100% to 90% of the constant current at a luminance of 5,000nit as provided in Table 1 below.

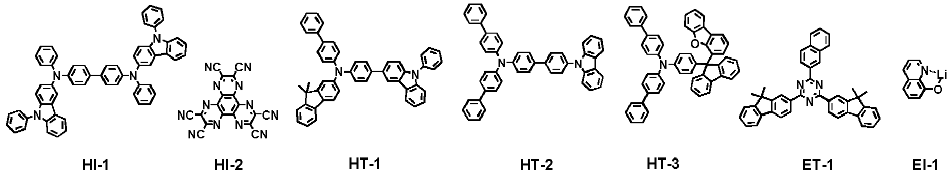

### Comparative Example 1-1: Production of an OLED device by using only the second host compound according to the present invention as a host

An OLED device was produced in the same manner as in Device Example 1-1, except that only the second host compound was used as a host in a light-emitting layer.

The luminescent properties of the OLED devices produced in Device Example 1-1 and Comparative Example 1-1 are provided in Table 1 below.

**Table 1**

| | Hole Transport Layer | Host | Dopant | Voltage (V) | Efficiency (cd/A) | Color Coordinate (x, y) | T90 lifespan (hr) |
|---|---|---|---|---|---|---|---|
| Device Example 1-1 | HT-1/HT-2 | H1-1:H2-2 | D-96 | 4.3 | 25.5 | 0.663, 0.336 | 360 |
| Comparative Example 1-1 | HT-1/HT-2 | H2-2 | D-96 | 4.1 | 28.2 | 0.662, 0.337 | 100 |

### Device Examples 2-1 to 2-13: Production of an OLED device by co-deposition of the first host compound and the second host compound according to the present invention as a host

An OLED device was produced in the same manner as in Device Example 1-1, except that hole injection layer 2 has a thickness of 3 nm, hole transport layer 1 has a thickness of 40 nm, hole transport layer 2 is not present, D-25 as a dopant was deposited in a doping amount on 15wt% in a light-emitting layer, the electron transport layer having a thickness of 35 nm was deposited via the evaporation rate of 4:6, the combinations of the first host compound and the second host compound used as hosts in a light-emitting layer are based on Device Examples 2-1 to 2-13 as provided in Table 2 below, and the lifespan taken to be reduced from 100% to 90% of the constant current at a luminance of 15,000nit as provided in Table 2 below.

### Device Examples 2-14 to 2-18: Production of an OLED device by co-deposition of the first host compound and the second host compound according to the present invention as a host

An OLED device was produced in the same manner as in Device Examples 2-1 to 2-13, except that hole injection layer 2 has a thickness of 3 nm, hole transport layer 1 has a thickness of 40 nm, hole transport layer 2 is not present, D-1 as a dopant was used in a light-emitting layer, the electron transport layer having a thickness of 35 nm was deposited via the evaporation rate of 4:6, the combinations of the first host compound and the second host compound used as hosts in a light-emitting layer are based on Device Examples 2-14 to 2-18 as provided in Table 2 below, and the lifespan taken to be reduced from 100% to 90% of the constant current at a luminance of 15,000nit as provided in Table 2 below.

### Device Examples 3-1 to 3-8: Production of an OLED device by co-deposition of the first host compound and the second host compound according to the present invention as a host

An OLED device was produced in the same manner as in Device Examples 2-1 to 2-13, except that hole transport layer 1 has a thickness of 10 nm, hole transport layer 2 of HT-3 has a thickness of 30 nm, D-136 as a dopant was used in a light-emitting layer, and the combinations of the first host compound and the second host compound used as hosts in a light-emitting layer are based on Device Examples 3-1 to 3-8 as provided in Table 2 below.

### Device Example 3-9: Production of an OLED device by co-deposition of the first host compound and the second host compound according to the present invention as a host

An OLED device was produced in the same manner as in Device Examples 2-1 to 2-13, except that hole transport layer 1 has a thickness of 10 nm, hole transport layer 2 of HT-3 has a thickness of 30 nm, D-164 as a dopant was used in a light-emitting layer, and the combinations of the first host compound and the second host compound used as hosts in a light-emitting layer are based on Device Example 3-9 as provided in Table 2 below.

### Device Examples 3-10 to 3-12: Production of an OLED device by co-deposition of the first host compound and the second host compound according to the present invention as a host

An OLED device was produced in the same manner as in Device Examples 2-1 to 2-13, except that hole transport layer 1 has a thickness of 10 nm, hole transport layer 2 of HT-3 has a thickness of 30 nm, D-168 as a dopant was used in a light-emitting layer, and the combinations of the first host compound and the second host compound used as hosts in a light-emitting layer are based on Device Examples 3-10 to 3-12 as provided in Table 2 below.

### Device Example 3-13: Production of an OLED device by co-deposition of the first host compound and the second host compound according to the present invention as a host

An OLED device was produced in the same manner as in Device Examples 2-1 to 2-13, except that hole transport layer 1 has a thickness of 10 nm, hole transport layer 2 of HT-3 has a thickness of 30 nm, D-180 as a dopant was used in a light-emitting layer, and the combinations of the first host compound and the second host compound used as hosts in a light-emitting layer are based on Device Example 3-13 as provided in Table 2 below.

### Comparative Examples 2-1 to 2-3: Production of an OLED device by using only the first host compound according to the present invention as a host

An OLED device was produced in the same manner as in Device Examples 2-1 to 2-13, except that the first host compound used as hosts in a light-emitting layer is based on Comparative Examples 2-1 to 2-3 as provided in Table 2 below.

### Comparative Examples 3-1 to 3-9: Production of an OLED device by using only the second host compound according to the present invention as a host

An OLED device was produced in the same manner as in Device Examples 2-1 to 2-13, except that the second host compound used as hosts in a light-emitting layer is based on Comparative Examples 3-1 to 3-9 as provided in Table 2 below.

### Comparative Example 4-1: Production of an OLED device by using only the second host compound according to the present invention as a host

An OLED device was produced in the same manner as in Device Examples 3-1 to 3-8, except that the second host compound used as hosts in a light-emitting layer is based on Comparative Example 4-1 as provided in Table 2 below.

The luminescent properties of the OLED devices produced in the above Device Examples and Comparative Examples are provided in Table 2 below.

**Table 2**

| | Hole Transport Laver | Host | Dopant | Voltage [V] | Efficiency [cd/A] | Color Coordinate (x, y) | T90 lifespan [hr] |
|---|---|---|---|---|---|---|---|
| Device Example 2-1 | HT-1 | H1-1:H2-25 | D-25 | 3.3 | 43.2 | 0.297, 0.660 | 100 |
| Device Example 2-2 | HT-1 | H1-1:H2-31 | D-25 | 3 | 58.8 | 0.303, 0.657 | 143 |
| Device Example 2-3 | HT-1 | H1-1:H2-48 | D-25 | 2.8 | 55.3 | 0.302, 0.657 | 124 |
| Device Example 2-4 | HT-1 | H1-1:H2-34 | D-25 | 3 | 55.7 | 0.302, 0.657 | 127 |
| Device Example 2-5 | HT-1 | H1-11:H2-31 | D-25 | 2.9 | 56.9 | 0.306, 0.656 | 147 |
| Device Example 2-6 | HT-1 | H1-12:H2-31 | D-25 | 2.9 | 54.5 | 0.304, 0.657 | 206 |
| Device Example 2-7 | HT-1 | H1-14:H2-31 | D-25 | 3.1 | 49.1 | 0.306, 0.655 | 124 |
| Device Example 2-8 | HT-1 | H1-4:H2-31 | D-25 | 2.9 | 55.2 | 0.300, 0.657 | 131 |
| Device Example 2-9 | HT-1 | H1-35:H2-31 | D-25 | 2.9 | 55.6 | 0.303, 0.656 | 161 |
| Device Example 2-10 | HT-1 | H1-1:H2-101 | D-25 | 3 | 55.6 | 0.303, 0.656 | 124 |
| Device Example 2-11 | HT-1 | H1-9:H2-31 | D-25 | 2.9 | 56 | 0.301, 0.657 | 203 |
| Device Example 2-12 | HT-1 | H1-2:H2-31 | D-25 | 2.8 | 54.9 | 0.307, 0.656 | 116 |
| Device Example 2-13 | HT-1 | H1-34:H2-31 | D-25 | 3 | 52.5 | 0.303, 0.657 | 160 |
| Device Example 2-14 | HT-1 | H1-1:H2-31 | D-1 | 2.8 | 57.8 | 0.315, 0.658 | 254 |
| Device Example 2-15 | HT-1 | H1-1:H2-48 | D-1 | 2.8 | 60.2 | 0.316, 0.659 | 240 |
| Device Example 2-16 | HT-1 | H1-11:H2-31 | D-1 | 2.8 | 52.4 | 0.317, 0.658 | 274 |
| Device Example 2-17 | HT-1 | H1-11:H2-48 | D-1 | 2.7 | 54.3 | 0.316, 0.659 | 272 |
| Device Example 2-18 | HT-1 | H1-11:H2-87 | D-1 | 2.9 | 51.9 | 0.319, 0.655 | 240 |
| Device Example 3-1 | HT-1/HT-3 | H1-1:H2-30 | D-136 | 3.3 | 63.9 | 0.324, 0.660 | 240 |
| Device Example 3-2 | HT-1/HT-3 | H1-1:H2-31 | D-136 | 3.2 | 71.2 | 0.326, 0.659 | 265 |
| Device Example 3-3 | HT-1/HT-3 | H1-1:H2-48 | D-136 | 3.1 | 68 | 0.325, 0.659 | 265 |
| Device Example 3-4 | HT-1/HT-3 | H1-1:H2-87 | D-136 | 3.3 | 67.4 | 0.327, 0.658 | 290 |
| Device Example 3-5 | HT-1/HT-3 | H1-11:H2-31 | D-136 | 3.1 | 69.2 | 0.327, 0.658 | 292 |
| Device Example 3-6 | HT-1/HT-3 | H1-11:H2-48 | D-136 | 3.2 | 64 | 0.326, 0.658 | 322 |
| Device Example 3-7 | HT-1/HT-3 | H1-11: H2-87 | D-136 | 3.1 | 65.2 | 0.327, 0.657 | 367 |
| Device Example 3-8 | HT-1/HT-3 | H1-35:H2-125 | D-136 | 3.1 | 65.2 | 0.330, 0.655 | 408 |
| Device Example 3-9 | HT-1/HT-3 | H1-35:H2-31 | D-164 | 3.2 | 61.5 | 0.316, 0.656 | 241 |
| Device Example 3-10 | HT-1/HT-3 | H1-1:H2-31 | D-168 | 3.2 | 62.1 | 0.281, 0.665 | 148 |
| Device Example 3-11 | HT-1/HT-3 | H1-35:H2-31 | D-168 | 3.2 | 59.4 | 0.278, 0.668 | 162 |
| Device Example 3-12 | HT-1/HT-3 | H1-12:H2-125 | D-168 | 3.1 | 56.6 | 0.288, 0.665 | 164 |
| Device Example 3-13 | HT-1/HT-3 | H1-12:H2-125 | D-180 | 3.1 | 49.7 | 0.291, 0.664 | 240 |
| Comparative Example 2-1 | HT-1 | H1-12 | D-25 | 5.9 | 3.1 | 0.299, 0.656 | × |
| Comparative Example 2-2 | HT-1 | H1-4 | D-25 | 6.7 | 3 | 0.289, 0.658 | × |
| Comparative Example 2-3 | HT-1 | H1-35 | D-25 | 6.6 | 3.9 | 0.395, 0.658 | × |
| Comparative Example 3-1 | HT-1 | H2-25 | D-25 | 3.1 | 54.2 | 0.308, 0.655 | 45 |
| Comparative Example 3-2 | HT-1 | H2-31 | D-25 | 2.9 | 42.8 | 0.314, 0.652 | 39 |
| Comparative Example 3-3 | HT-1 | H2-48 | D-25 | 2.6 | 49.6 | 0.314, 0.652 | 67 |
| Comparative Example 3-4 | HT-1 | H2-101 | D-25 | 2.8 | 50.3 | 0.315, 0.651 | 24 |
| Comparative Example 3-5 | HT-1 | H2-34 | D-25 | 2.7 | 49.2 | 0.312, 0.652 | 38 |
| Comparative Example 3-6 | HT-1 | H2-30 | D-25 | 2.8 | 55.3 | 0.314, 0.652 | 70 |
| Comparative Example 3-7 | HT-1 | H2-31 | D-1 | 2.9 | 33.5 | 0.323, 0.653 | 130 |
| Comparative Example 3-8 | HT-1 | H2-48 | D-1 | 2.6 | 41.2 | 0.325, 0.653 | 124 |
| Comparative Example 3-9 | HT-1 | H2-87 | D-1 | 2.8 | 37.9 | 0.323, 0.653 | 146 |
| Comparative Example 4-1 | HT-1/HT-3 | H2-125 | D-136 | 3.0 | 64.9 | 0.337, 0.649 | 124 |

### Device Examples 4-1 to 4-7: Production of an OLED device by co-deposition of the first host compound and the second host compound according to the present invention as a host

An OLED device was produced in the same manner as in Device Example 1-1, except that HT-4 was used as a hole transport layer 2, the combinations of the first host compound and the second host compound used as hosts in a light-emitting layer are based on Device Examples 4-1 to 4-7 as provided in Table 3 below, and the lifespan taken to be reduced from 100% to 95% of the constant current at a luminance of 5,000nit as provided in Table 3 below.

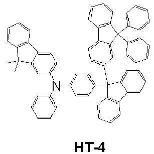

### Comparative Examples 5-1 and 5-2: Production of an OLED device by using only the second host compound according to the present invention as a host

An OLED device was produced in the same manner as in Device Examples 4-1 to 4-7, except that the second host compound used as hosts in a light-emitting layer is based on Comparative Examples 5-1 and 5-2 as provided in Table 3 below.

The luminescent properties of the OLED devices produced in Device Examples 4-1 to 4-7, and Comparative Examples 5-1 and 5-2 are provided in Table 3 below.

**Table 3**

| | Hole Transport Layer | Host | Dopant | Voltage [V] | Efficiency [cd/A] | Color Coordinate (x, y) | T95 lifespan [hr] |
|---|---|---|---|---|---|---|---|
| Device Example 4-1 | HT-1 / HT-4 | H1-287 : H2-496 | D-96 | 3.8 | 30.8 | 0.667, 0.333 | 310 |
| Device Example 4-2 | HT-1 / HT-4 | H1-12 : H2-504 | D-96 | 3.5 | 30.7 | 0.667, 0.333 | 390 |
| Device Example 4-3 | HT-1 / HT-4 | H1-9 : H2-496 | D-96 | 3.9 | 31.1 | 0.665, 0.335 | 130 |
| Device Example 4-4 | HT-1 / HT-4 | H1-35 : H2-496 | D-96 | 3.8 | 31.1 | 0.665, 0.334 | 200 |
| Device Example 4-5 | HT-1 / HT-4 | H1-287 : H2-504 | D-96 | 3.7 | 31.3 | 0.666, 0.333 | 200 |
| Device Example 4-6 | HT-1 / HT-4 | H1-282 : H2-504 | D-96 | 3.7 | 31.4 | 0.666, 0.334 | 120 |
| Device Example 4-7 | HT-1 / HT-4 | H1-12 : H2-496 | D-96 | 3.6 | 29.2 | 0.667, 0.333 | 150 |
| Comparative Example 5-1 | HT-1 / HT-4 | H2-496 | D-96 | 3.7 | 31.0 | 0.665, 0.334 | 90 |
| Comparative Example 5-2 | HT-1 / HT-4 | H2-504 | D-96 | 3.7 | 31 | 0.667, 0.333 | 70 |

### Device Examples 5-1 and 5-2: Production of an OLED device by co-deposition of the first host compound and the second host compound according to the present invention as a host

An OLED device was produced in the same manner as in Device Examples 3-1 to 3-11, except that D-134 was used as a dopant in a light-emitting layer, the combinations of the first host compound and the second host compound used as hosts in a light-emitting layer are based on Device Examples 5-1 and 5-2 as provided in Table 4 below, and the lifespan taken to be reduced from 100% to 97% of the constant current at a luminance of 15,000nit as provided in Table 4 below.

### Comparative Examples 6-1 and 6-2: Production of an OLED device by using only the first host compound according to the present invention as a host

An OLED device was produced in the same manner as in Device Examples 5-1 and 5-2, except that the first host compound used as hosts in a light-emitting layer is based on Comparative Examples 6-1 and 6-2 as provided in Table 4 below.

### Comparative Example 7-1: Production of an OLED device by using only the second host compound according to the present invention as a host

An OLED device was produced in the same manner as in Device Examples 5-1 and 5-2, except that the second host compound used as hosts in a light-emitting layer is based on Comparative Example 7-1 as provided in Table 4 below.

The luminescent properties of the OLED devices produced in Device Examples 5-1 and 5-2, Comparative Examples 6-1 and 6-2, and Comparative Example 7-1 are provided in Table 4 below.

**Table 4**

| | Hole Transport Layer | Host | Dopant | Voltage [V] | Efficiency [cd/A] | Color Coordinate (x, y) | T97 lifespan [hr] |
|---|---|---|---|---|---|---|---|
| Device Example 5-1 | HT-1/ HT-3 | H1-12 : H2-660 | D-134 | 3.1 | 63.2 | 0.313, 0.665 | 39 |
| Device Example 5-2 | HT-1/ HT-3 | H1-35 : H2-660 | D-134 | 3.2 | 64.8 | 0.312, 0.665 | 56 |
| Comparative Example 6-1 | HT-1/ HT-3 | H1-12 | D-134 | 6.4 | 2.9 | 0.305, 0.660 | × |
| Comparative Example 6-2 | HT-1/ HT-3 | H1-35 | D-134 | 7.2 | 3.5 | 0.302, 0.664 | × |
| Comparative Example 7-1 | HT-1/ HT-3 | H2-660 | D-134 | 3.0 | 55.4 | 0.321, 0.659 | 5 |

The organic electroluminescent device of the present invention provides longer lifespan compared with conventional devices by comprising a light-emitting layer containing a host and a phosphorescent dopant, wherein the host consists of multi-component host compounds, at least a first host compound of the multi-component host compounds has a specific bicarbazole derivative containing an aryl group, and a second host compound has a specific carbazole derivative including a nitrogen-containing heteroaryl group.

The numbered paragraphs below form part of the disclosure:
1. An organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by the following formula 1 which is a bicarbazole derivative containing an aryl group, and a second host compound is represented by the following formula 2 which is a carbazole derivative including a nitrogen-containing heteroaryl group: 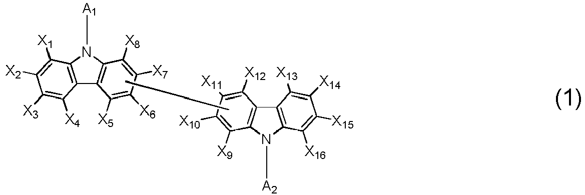 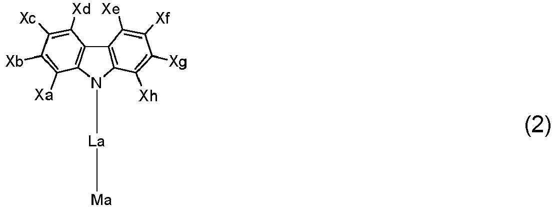 wherein
   A₁ and A₂ each independently represent a substituted or unsubstituted (C6-C30)aryl group;
   X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) may be replaced with at least one hetero atom selected from nitrogen, oxygen and sulfur;
   Ma represents a substituted or unsubstituted nitrogen-containing 5- to 30-membered heteroaryl group;
   La represents a single bond, or a substituted or unsubstituted (C6-C30)arylene group;
   Xa to Xh each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
   the fused aromatic or heteroaromatic ring is selected from the group consisting of benzene, indole, indene, benzofuran and benzothiophene, which may be further substituted with a (C1-C10)alkyl group or a (C6-C15)aryl group; and
   the heteroaryl group contains at least one hetero atom selected from B, N, O, S, P(=O), Si and P.
2. The organic electroluminescent device according to 1, wherein the compound of formula 1 is represented by the following formula 3, 4, 5, or 6: 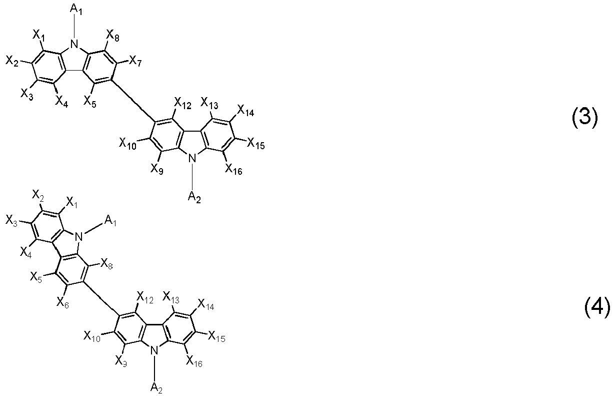 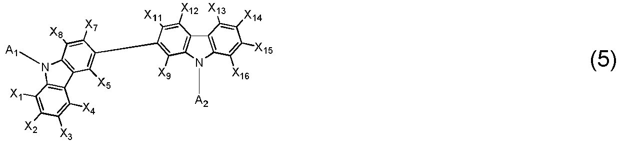 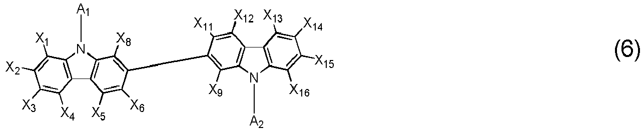 wherein
   A₁ and A₂ each independently represent a substituted or unsubstituted (C6-C30)aryl group; and
   X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur.
3. The organic electroluminescent device according to 1, wherein the compound of formula 2 is represented by the following formula 7, 8, or 9: 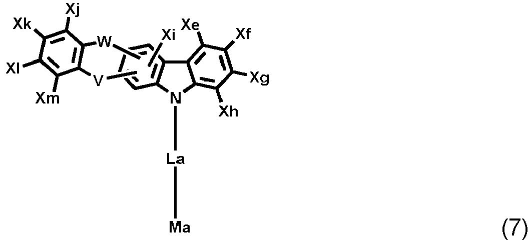 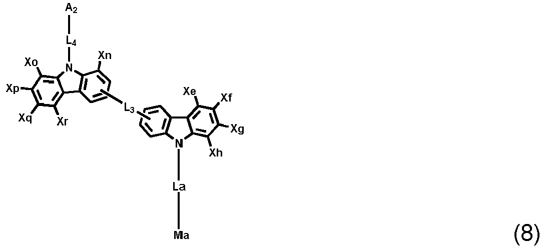 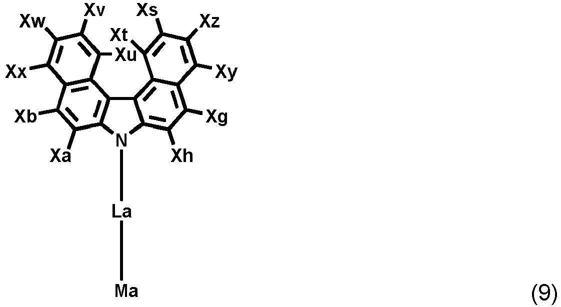 wherein
   V and W each independently represent a single bond, NR₁₅, CR₁₆R₁₇, S, or O, provided that both V and W neither represent a single bond nor represent NR₁₅;
   A₂ represents a substituted or unsubstituted (C6-C30)aryl group and may be bonded to Xn or Xo;
   L₃ and L₄ each independently represent a single bond, or a substituted or unsubstituted (C6-C60)arylene group;
   Xi represents hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
   Xj to Xz each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, a substituted or unsubstituted 3- to 30-membered heteroaryl group, -NR₅R₆, or-SiR₇R₈R₉; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
   Ma, La, Xa, Xb, and Xe to Xh are as defined in formula 2;
   R₅ to R₉ each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, or a substituted or unsubstituted 3- to 30-membered heteroaryl group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur;
   R₁₆ and R₁₇ each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3-to 7-membered heterocycloalkyl group, a substituted or unsubstituted (C6-C60)aryl group, or a substituted or unsubstituted 3- to 30-membered heteroaryl group; and
   R₁₅ represents hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3- to 7-membered heterocycloalkyl group, a substituted or unsubstituted (C6-C60)aryl group, or a substituted or unsubstituted 3- to 30-membered heteroaryl group.
4. The organic electroluminescent device according to 1, wherein La in formula 2 represents a single bond, or is represented by one selected from the following formulas 10 to 19: 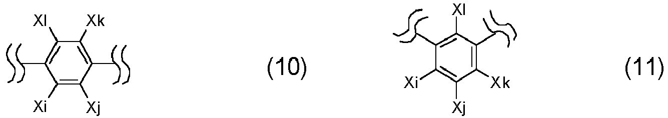 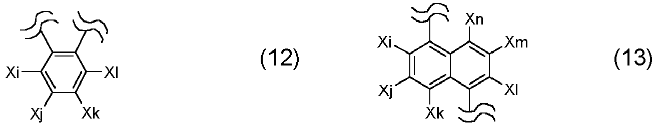 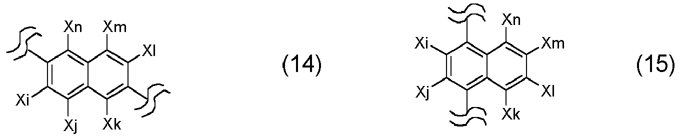 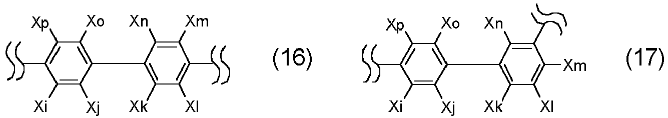 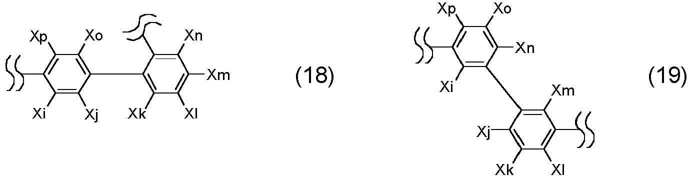 wherein
   Xi to Xp each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur.
5. The organic electroluminescent device according to 1, wherein Ma in formula 2 is a monocyclic-based heteroaryl group selected from the group consisting of pyrrolyl, imidazolyl, pyrazolyl, triazinyl, tetrazinyl, triazolyl, tetrazolyl, pyridyl, pyrazinyl, pyrimidinyl and pyridazinyl, or a fused ring-based heteroaryl group selected from the group consisting of benzoimidazolyl, isoindolyl, indolyl, indazolyl, benzothiadiazolyl, quinolyl, isoquinolyl, cinnolinyl, quinazolinyl, naphthyridinyl, quinoxalinyl, carbazolyl and phenanthridinyl.
6. The organic electroluminescent device according to 1, wherein A₁ and A₂ in formula 1 each independently represent phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, or fluoranthenyl.
7. The organic electroluminescent device according to 1, wherein Xa to Xh in formula 2 each independently represent hydrogen; a cyano group; a (C6-C15)aryl group which is unsubstituted or substituted with a tri(C6-C10)arylsilyl group, or a 10- to 20-membered heteroaryl group which is unsubstituted or substituted with a (C6-C12)aryl group; or are linked between adjacent substituents to form a substituted or unsubstituted benzene, a substituted or unsubstituted indole, a substituted or unsubstituted indene, a substituted or unsubstituted benzofuran, or a substituted or unsubstituted benzothiophene.
8. The organic electroluminescent device according to 1, wherein the triarylsilyl as X₁ to X₁₆ in formula 1 is triphenylsilyl.
9. The organic electroluminescent device according to 1, wherein the compound represented by formula 1 is selected from the group consisting of the following compounds: 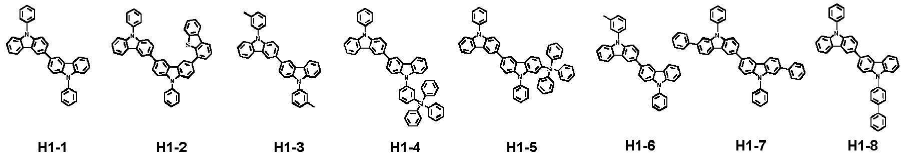 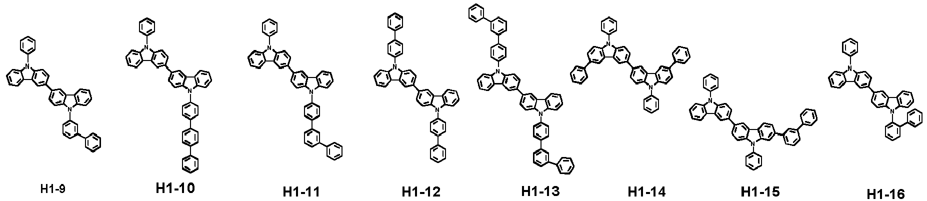 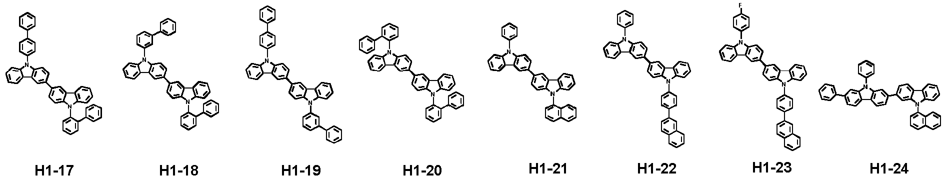 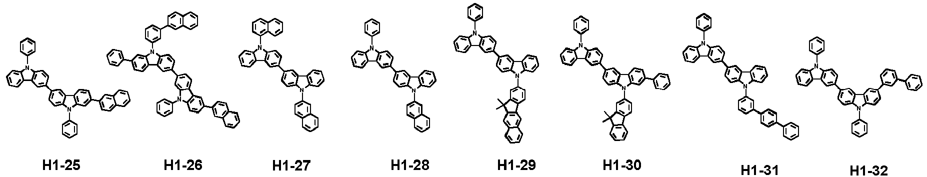 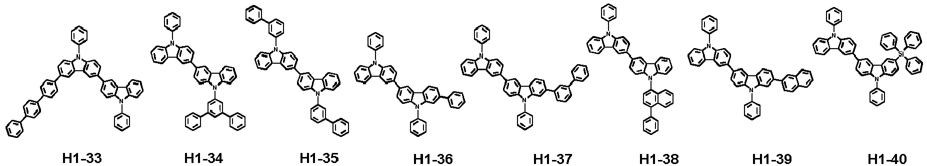 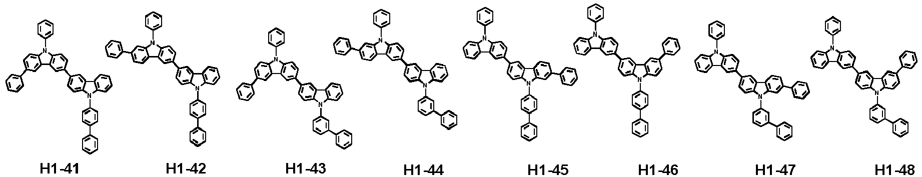 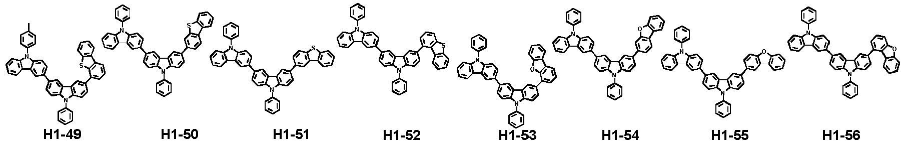 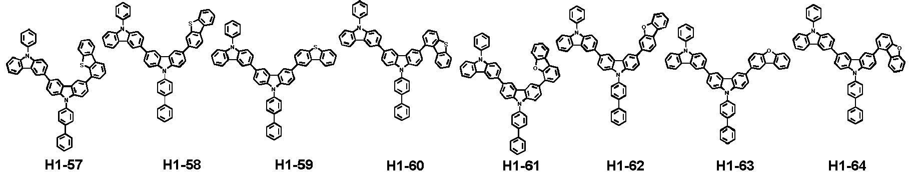 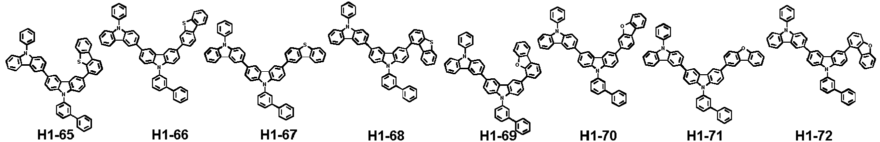 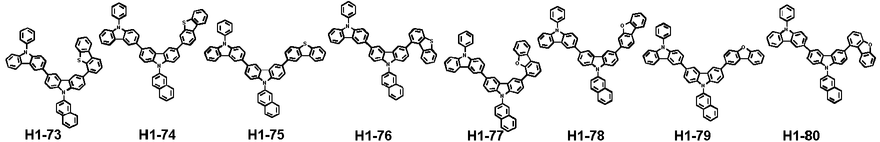 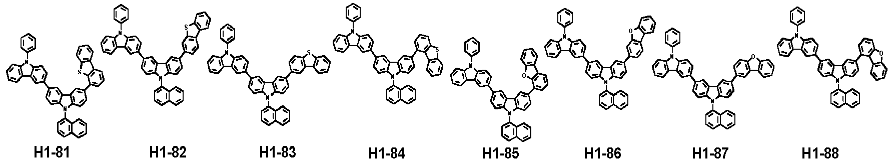 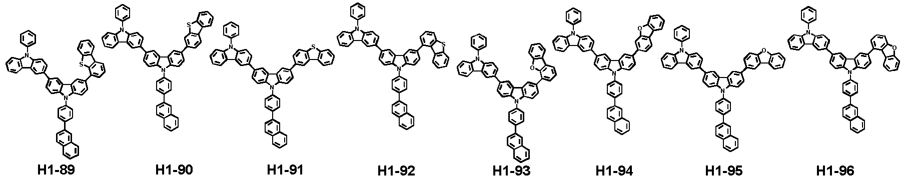 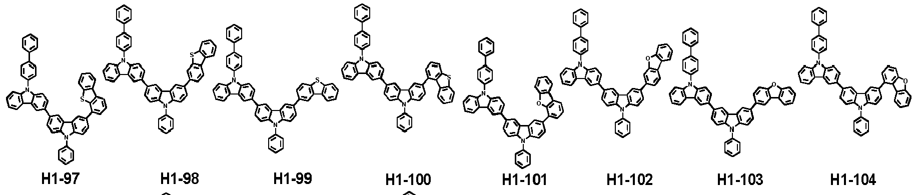 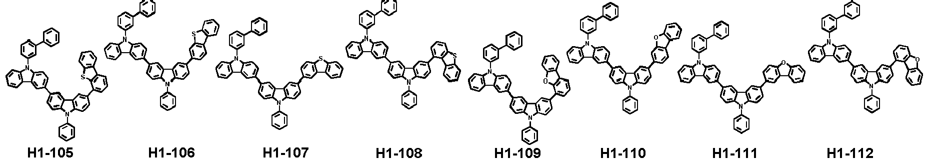 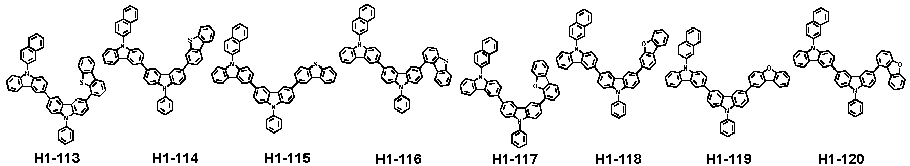 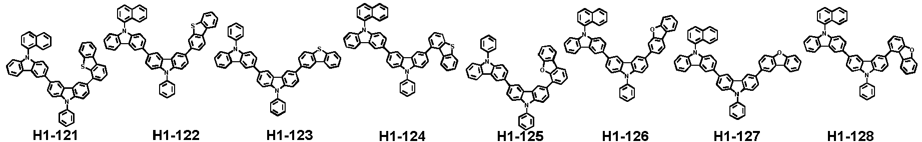 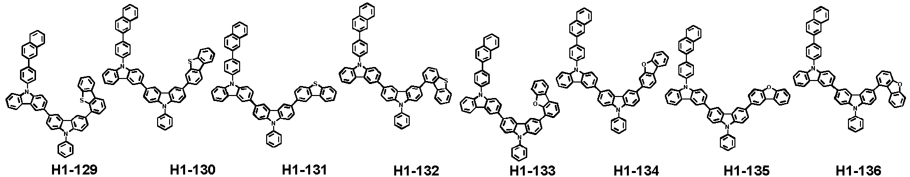 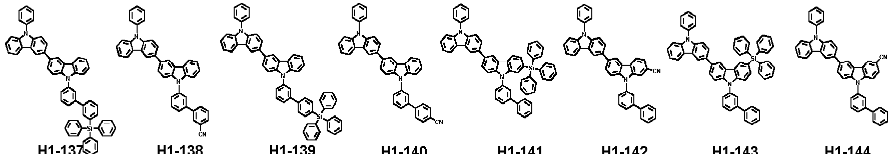 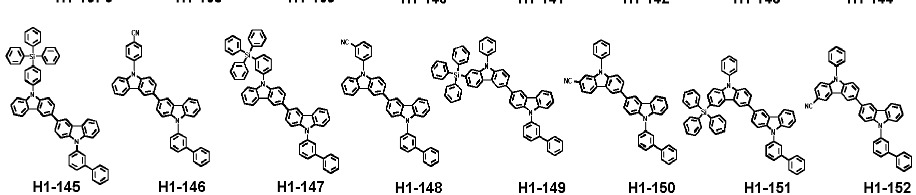 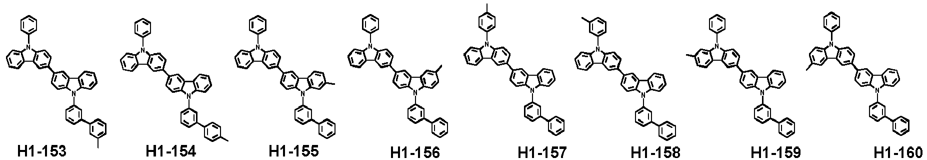 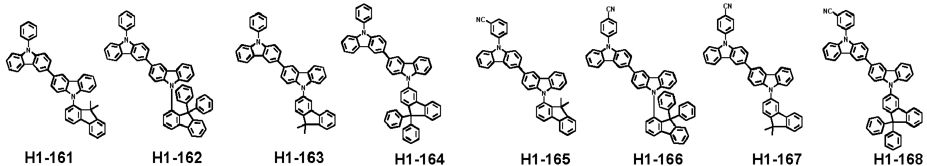 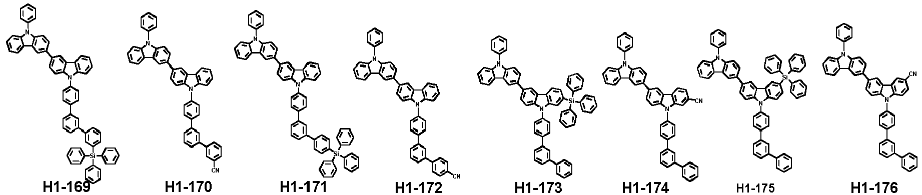 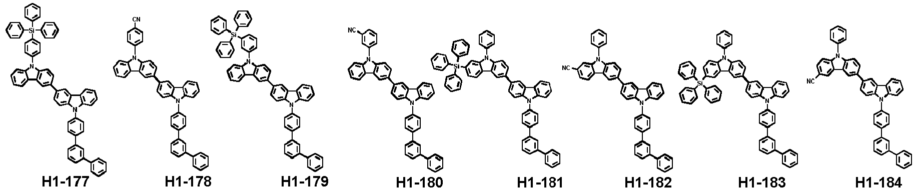 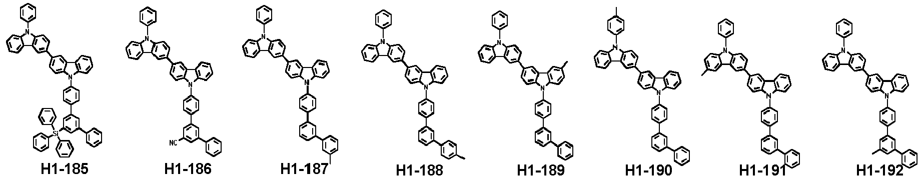 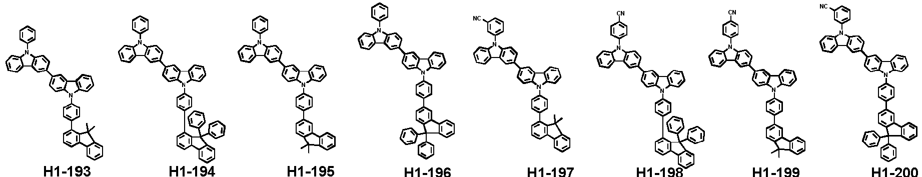 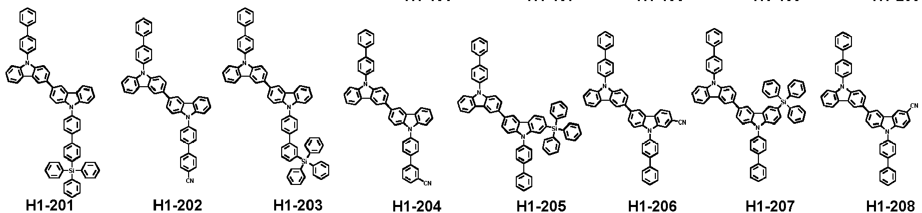 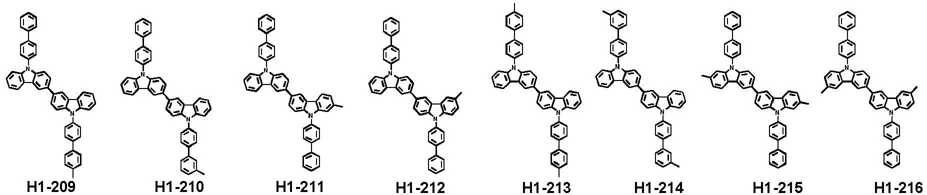 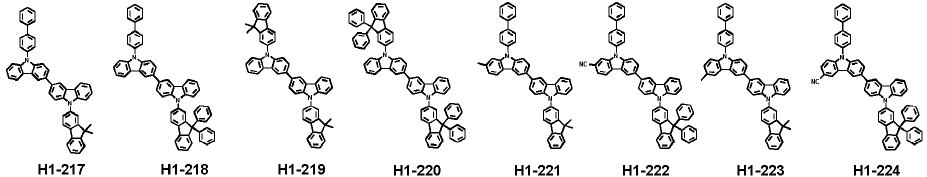 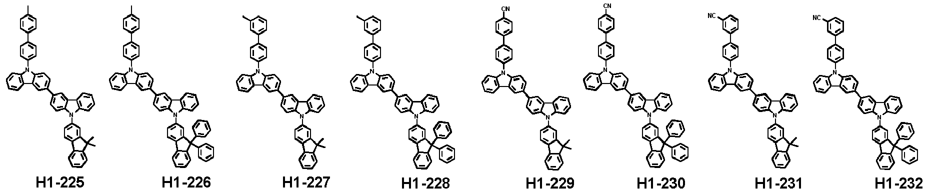 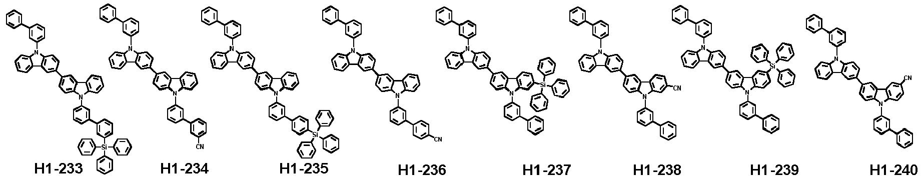 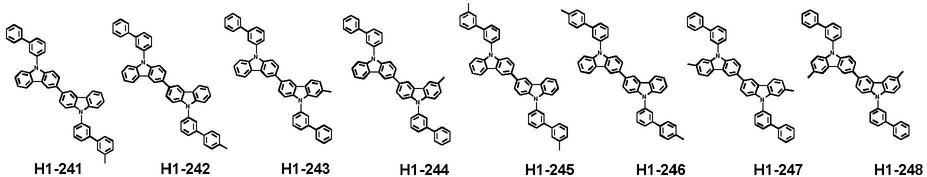 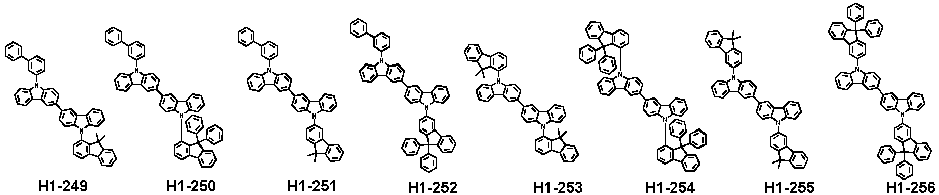 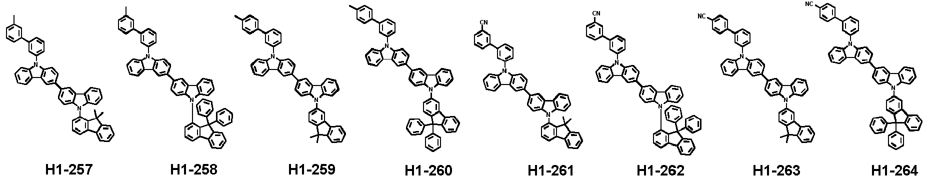 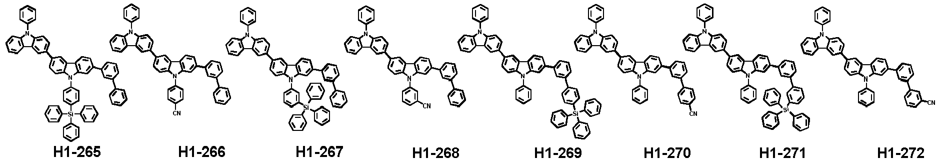 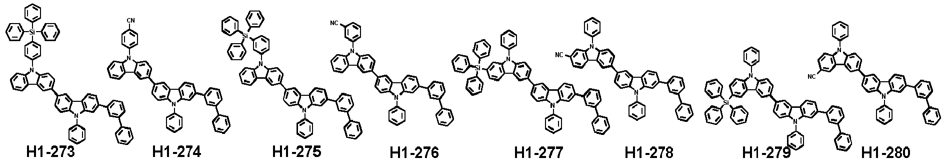 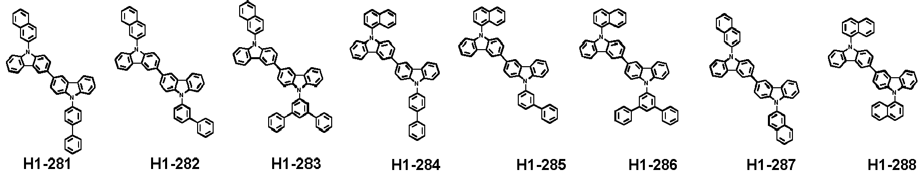 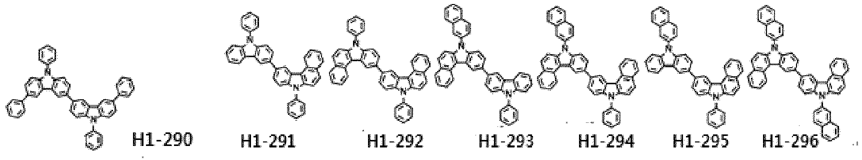
10. The organic electroluminescent device according to 1, wherein the compound represented by formula 2 is selected from the group consisting of the following compounds:

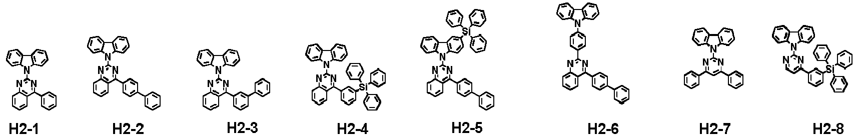

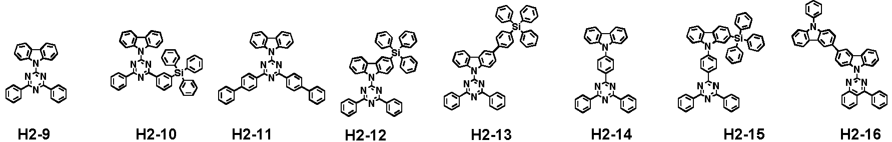

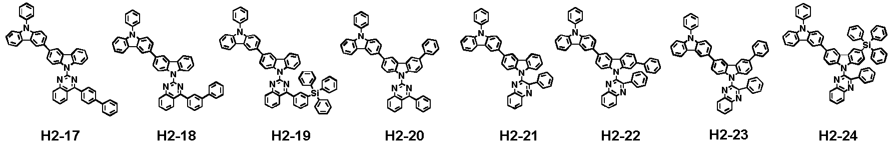

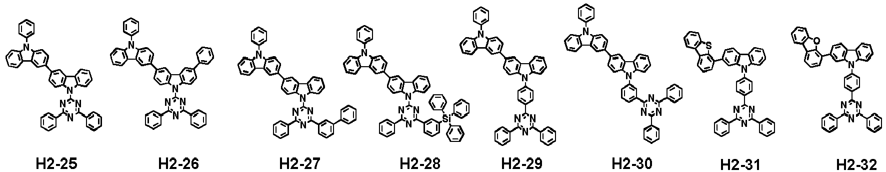

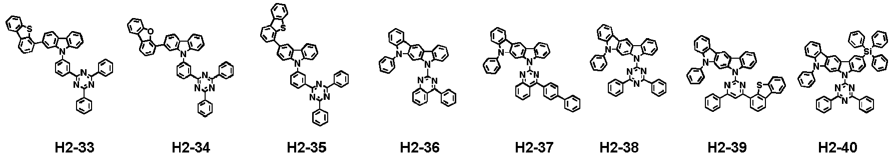

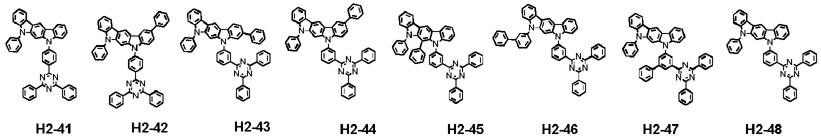

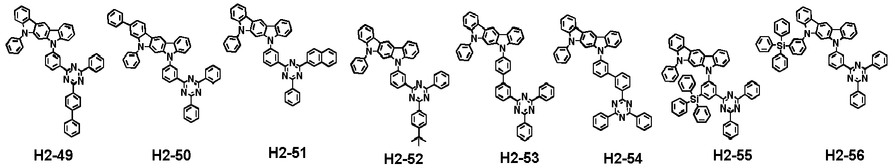

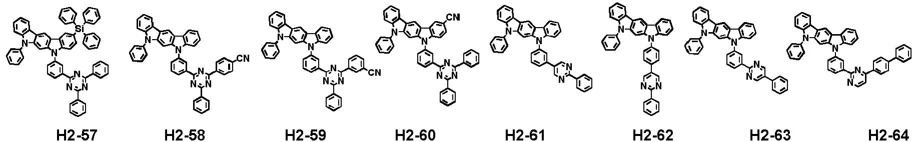

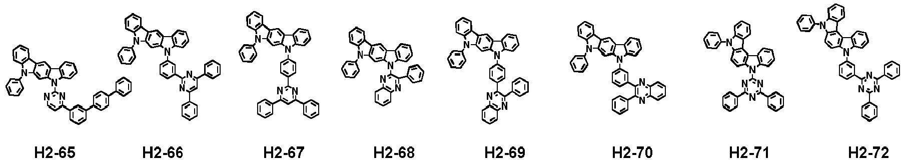

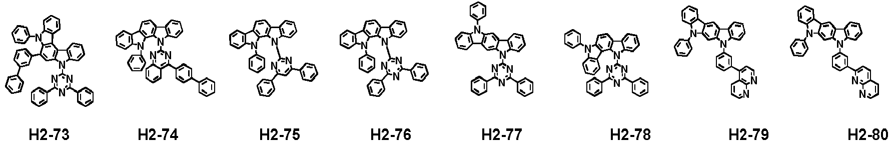

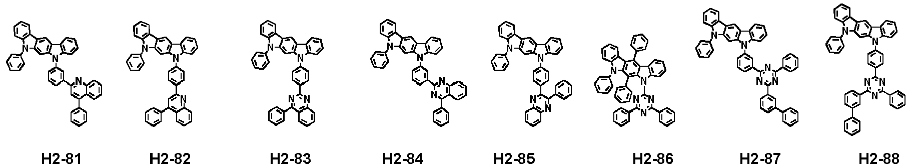

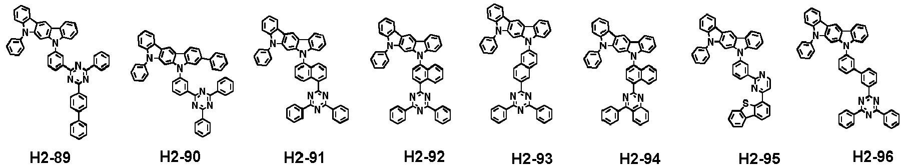

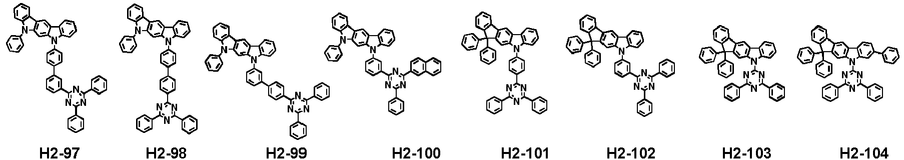

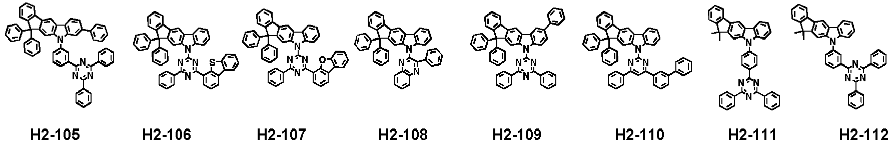

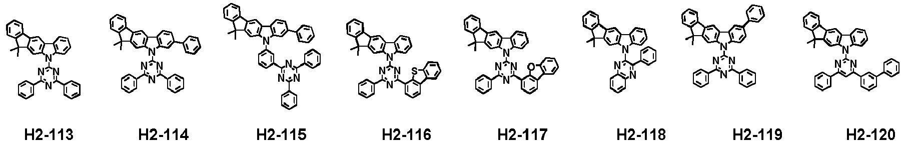

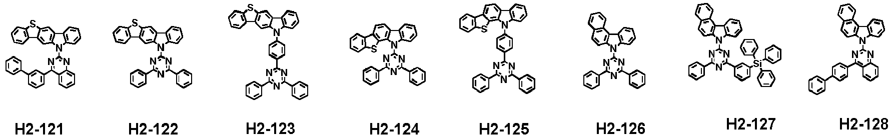

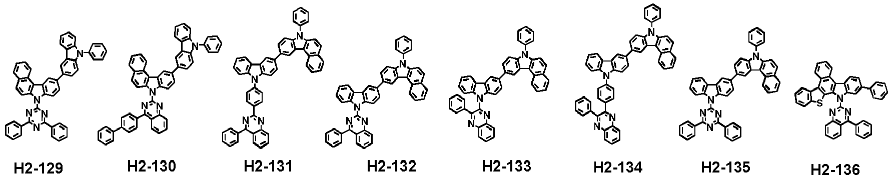

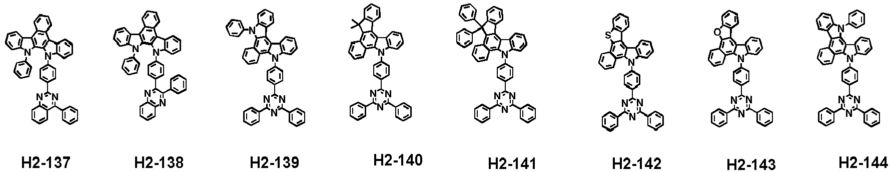

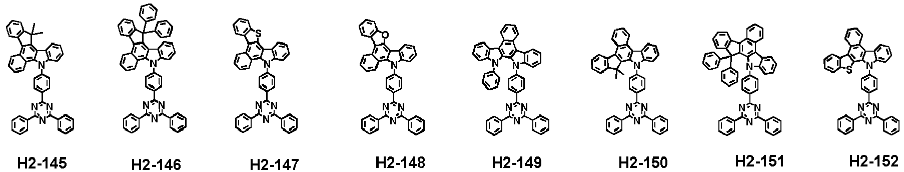

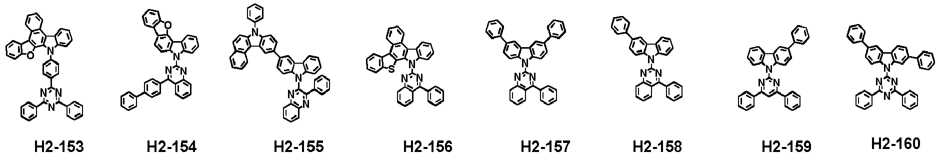

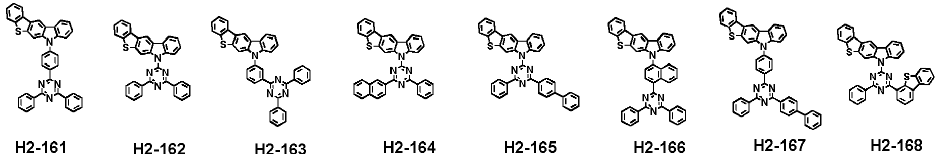

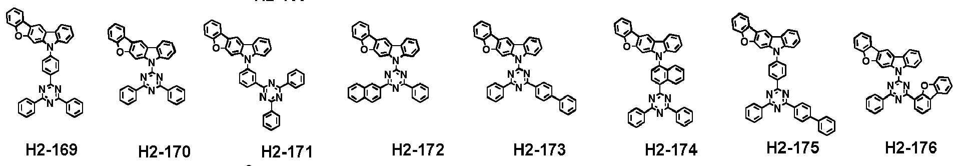

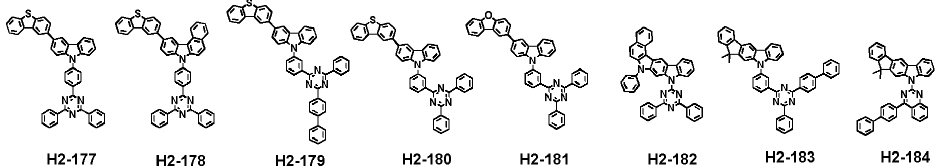

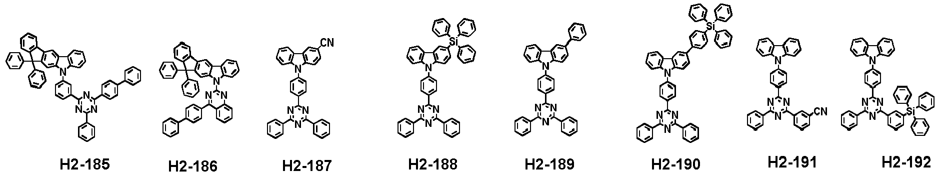

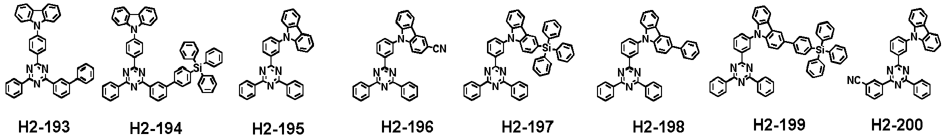

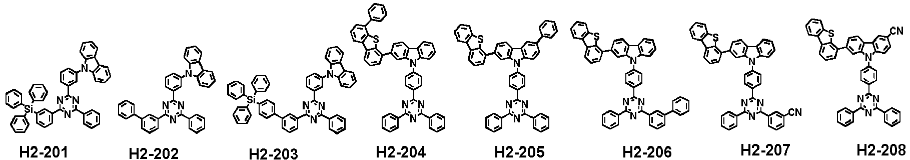

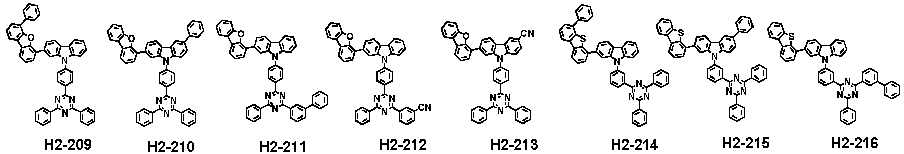

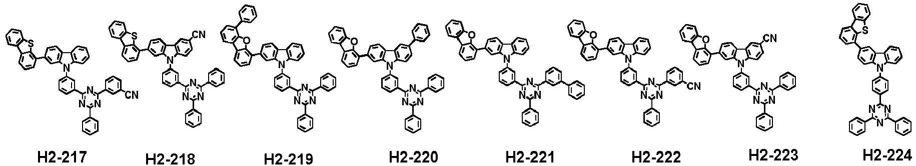

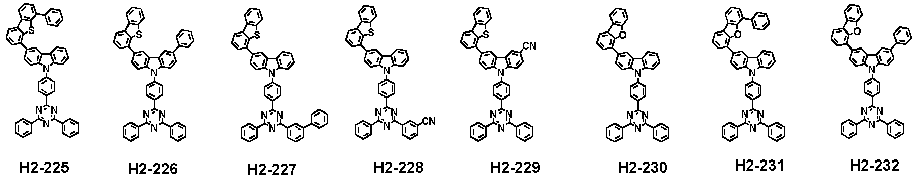

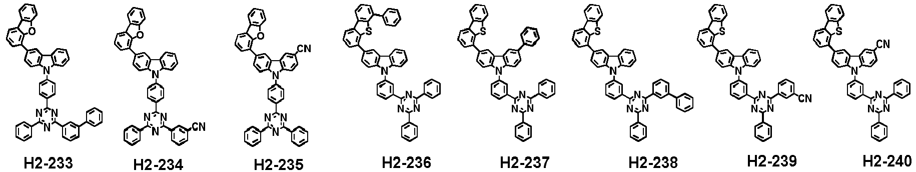

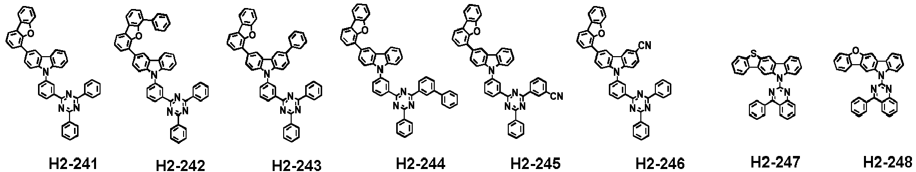

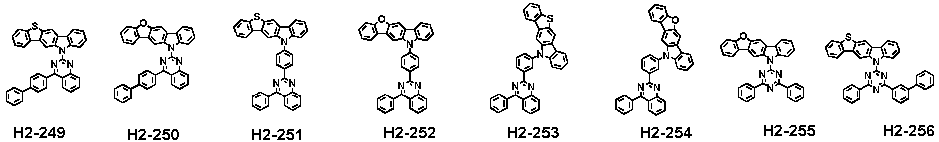

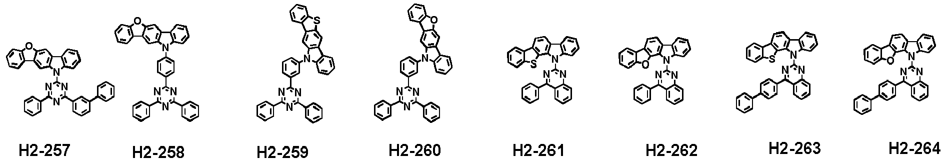

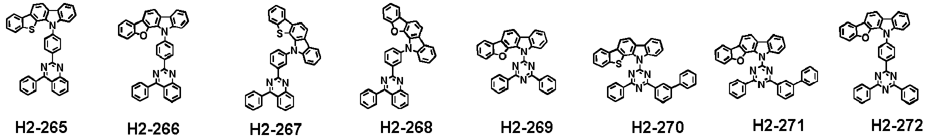

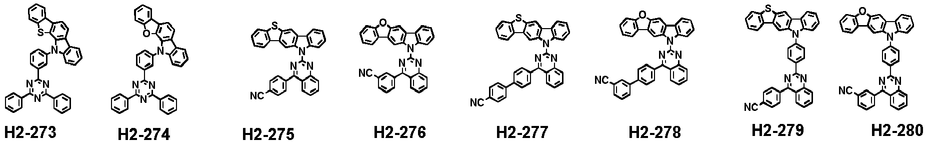

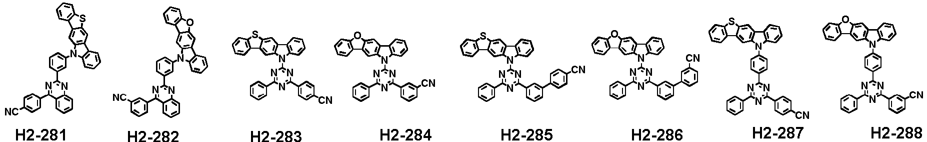

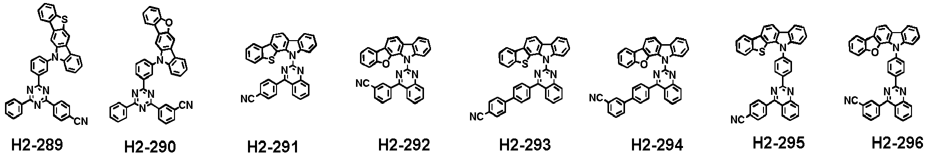

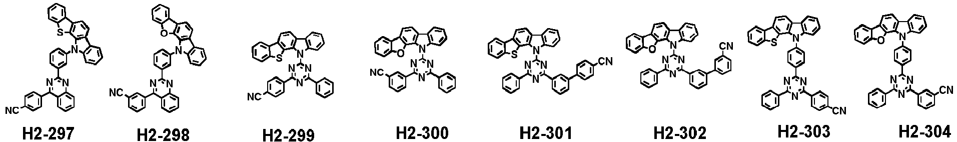

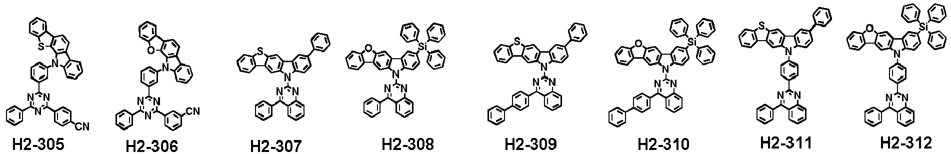

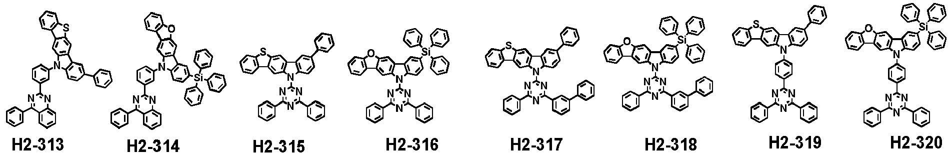

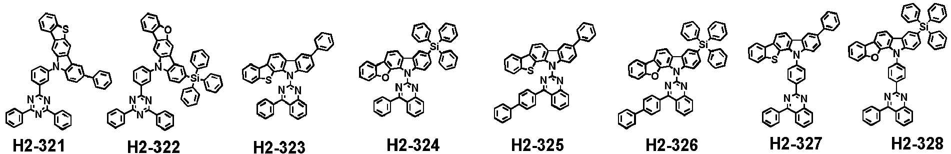

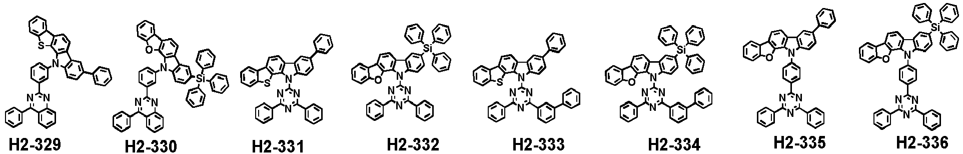

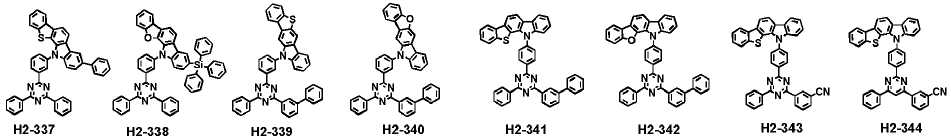

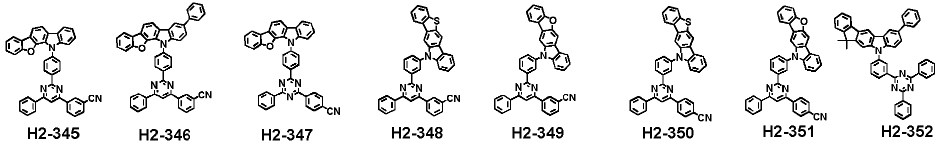

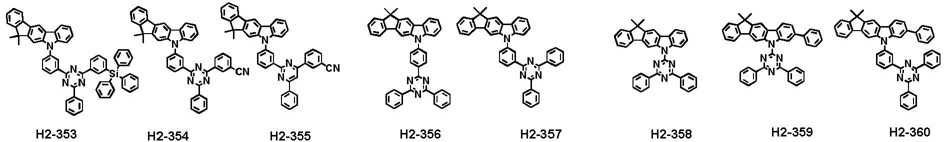

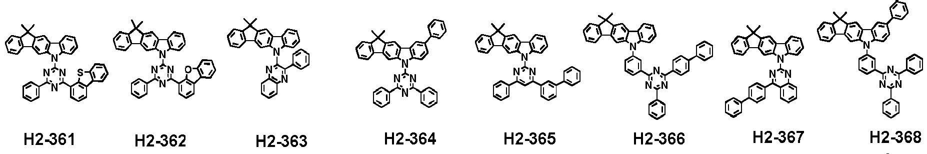

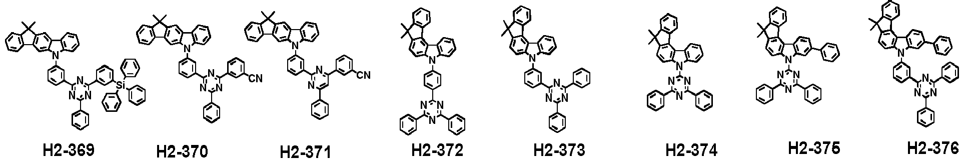

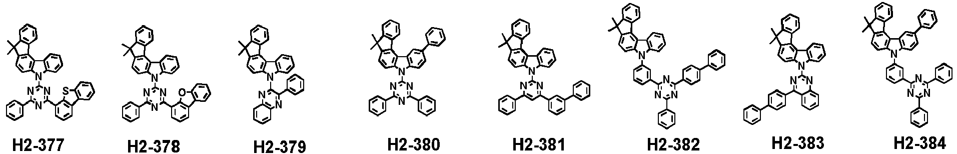

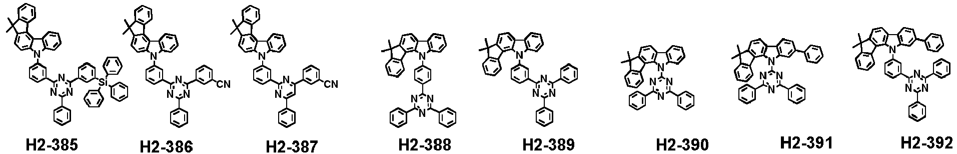

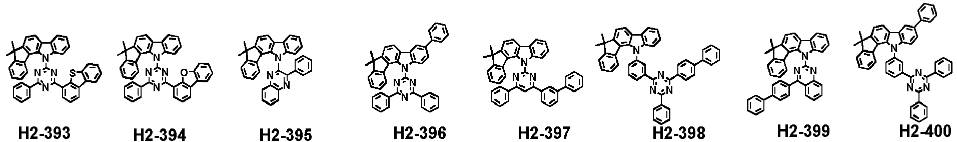

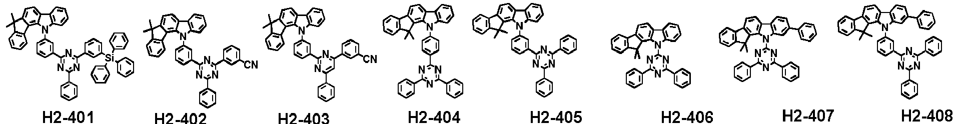

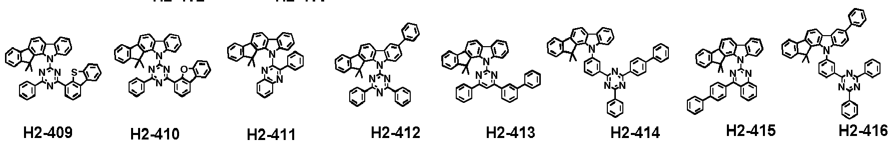

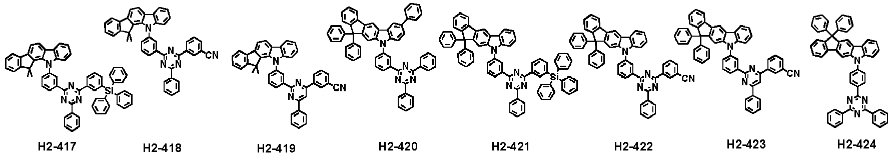

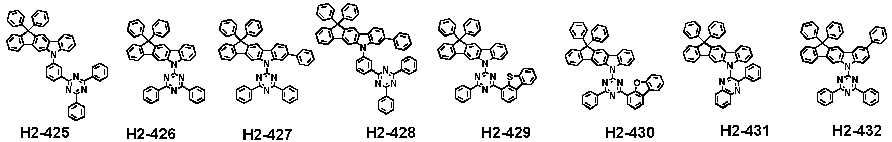

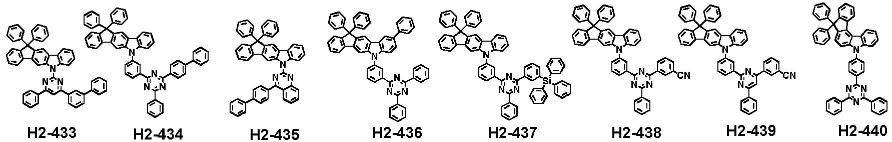

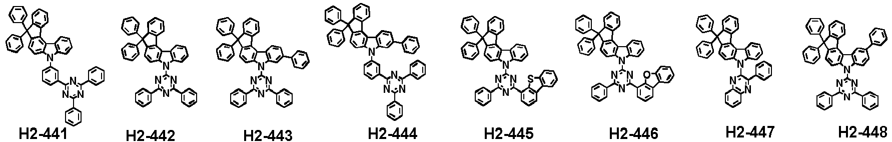

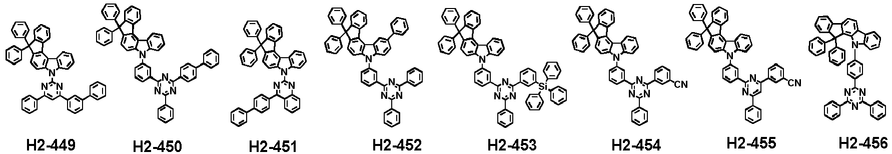

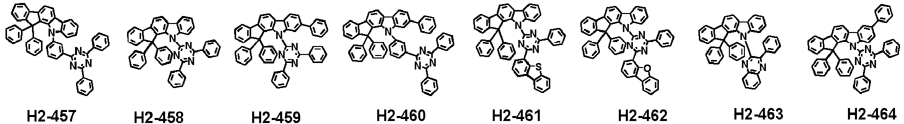

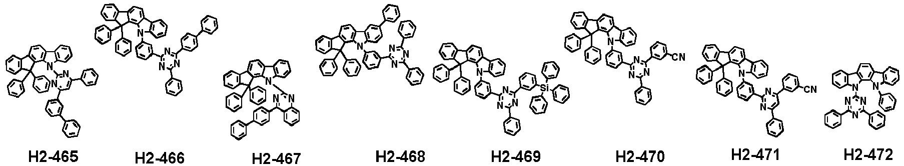

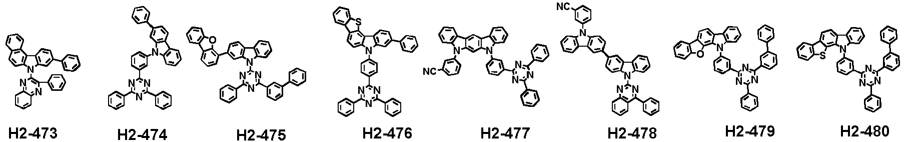

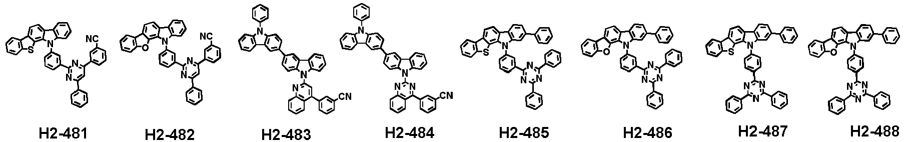

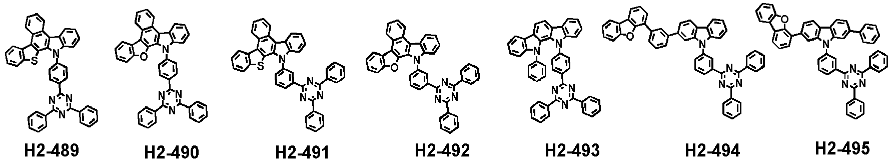

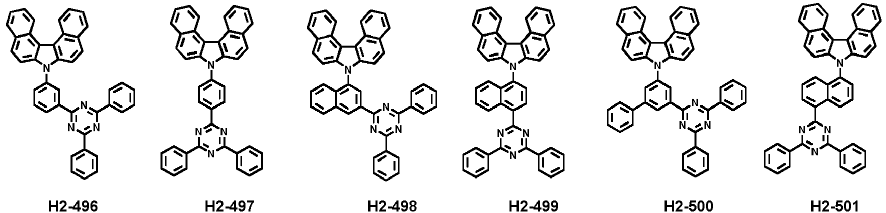

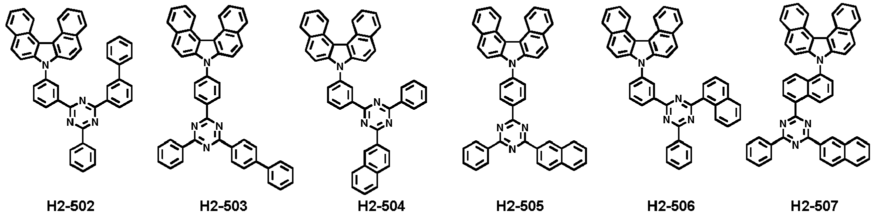

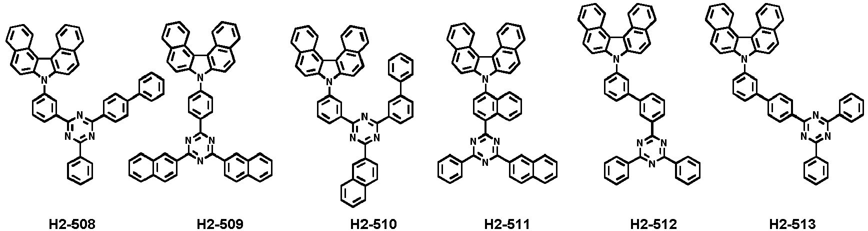

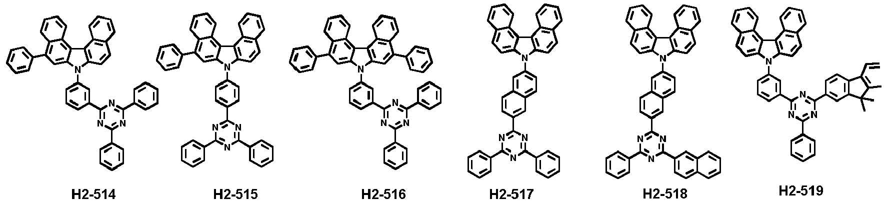

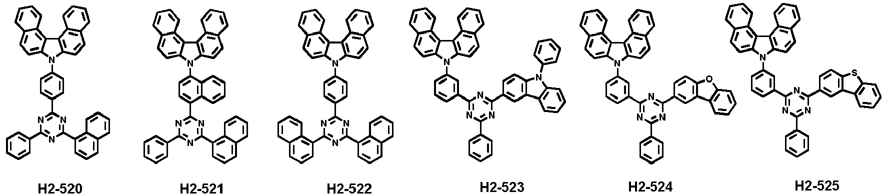

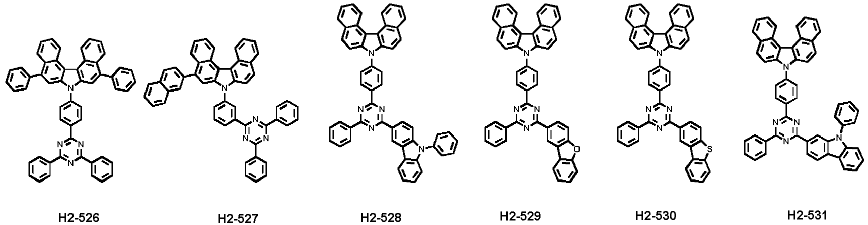

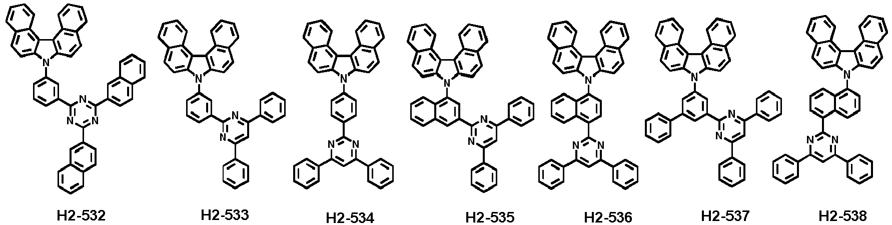

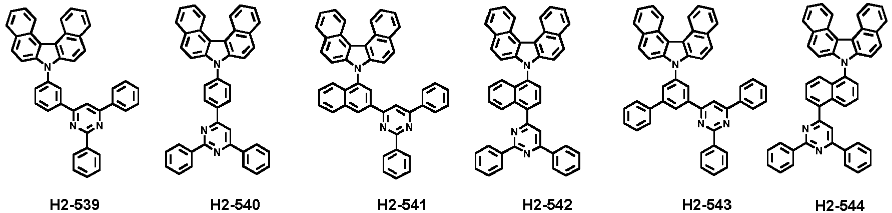

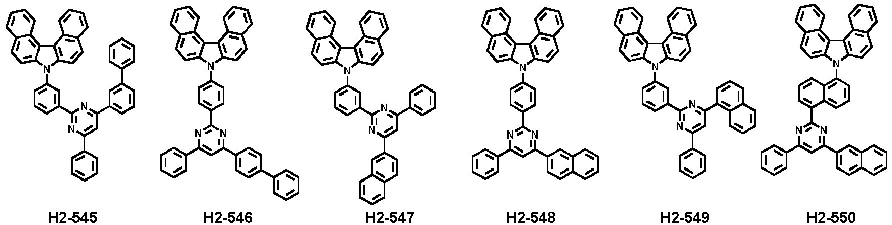

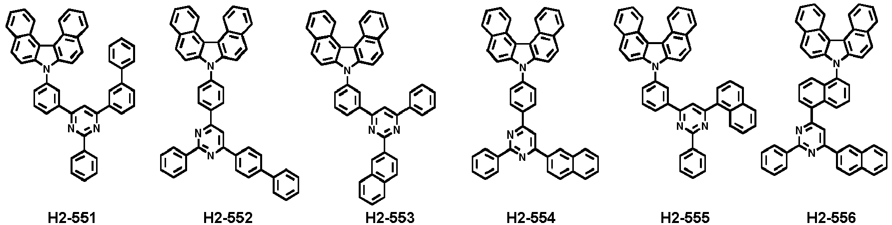

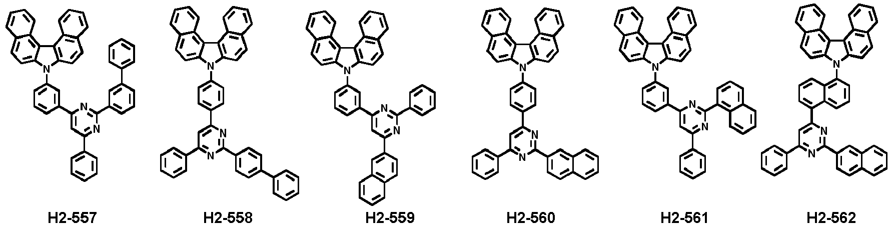

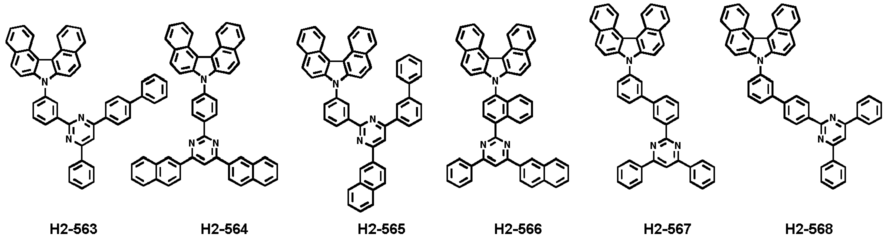

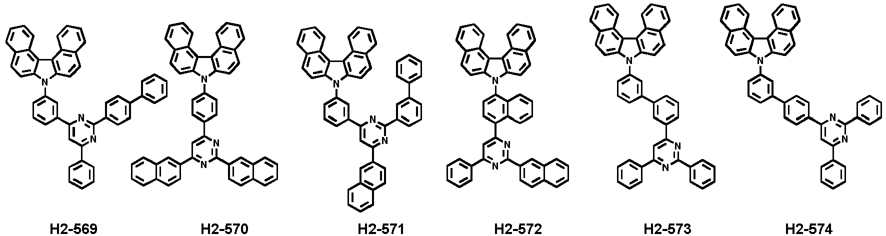

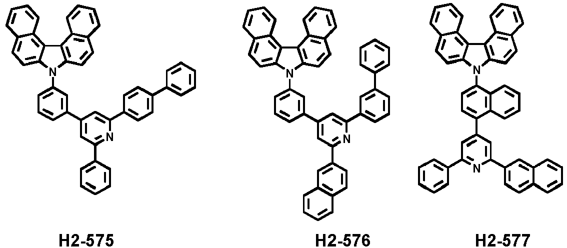

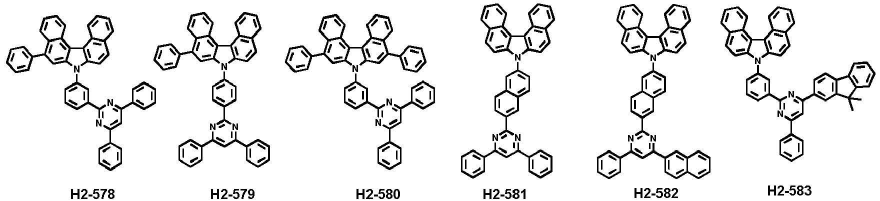

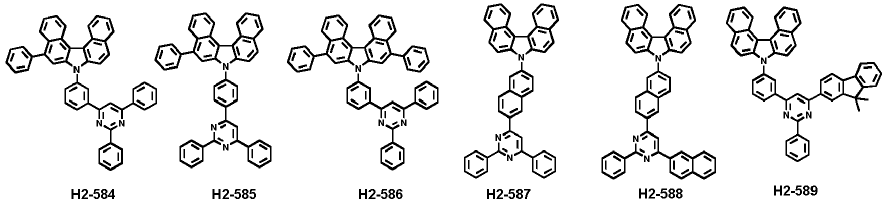

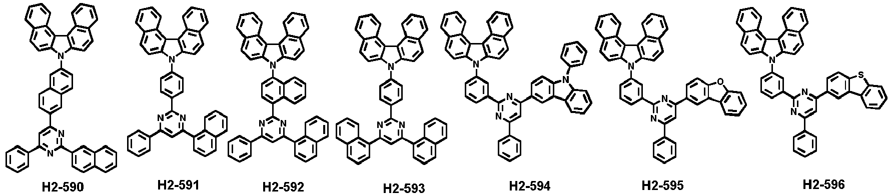

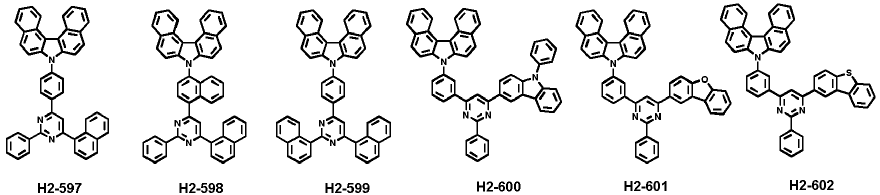

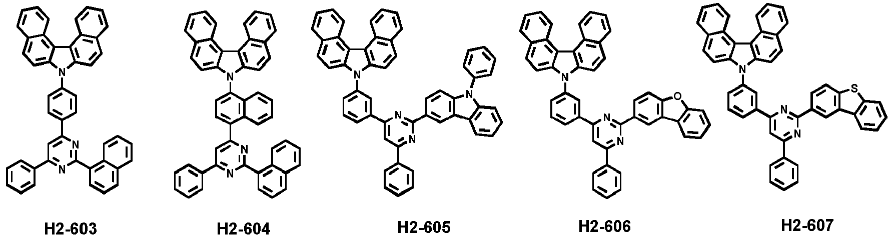

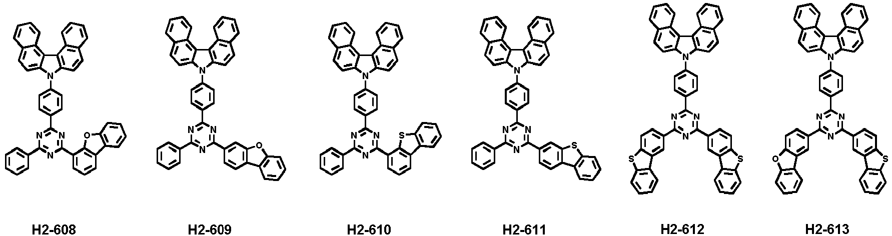

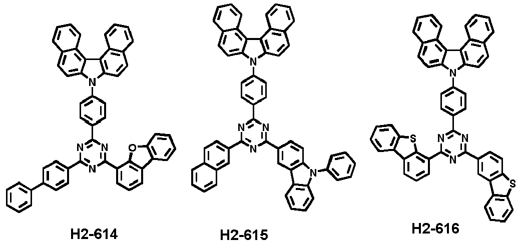

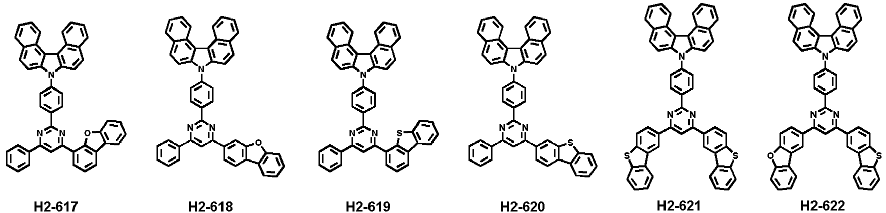

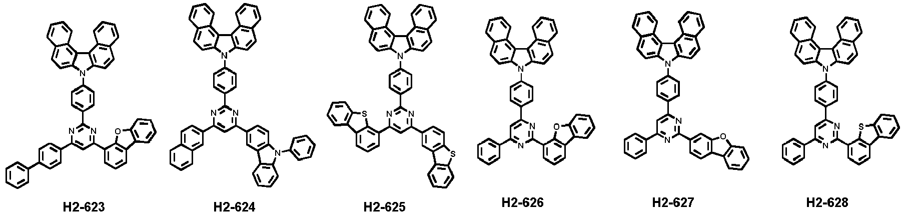

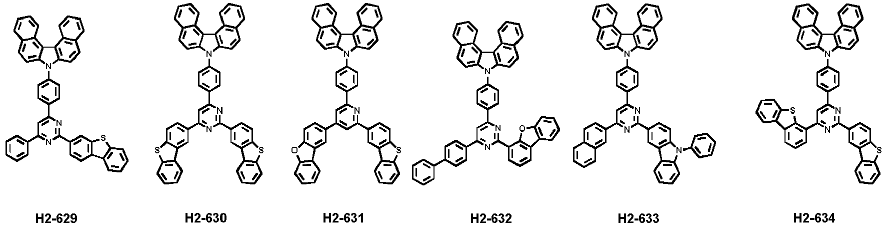

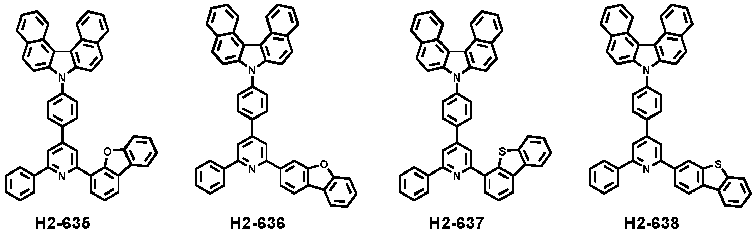

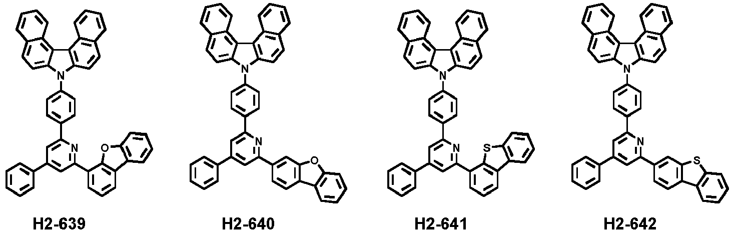

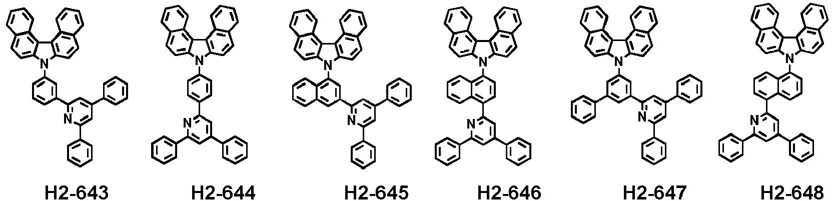

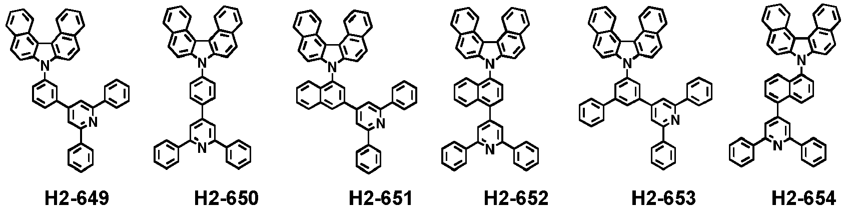

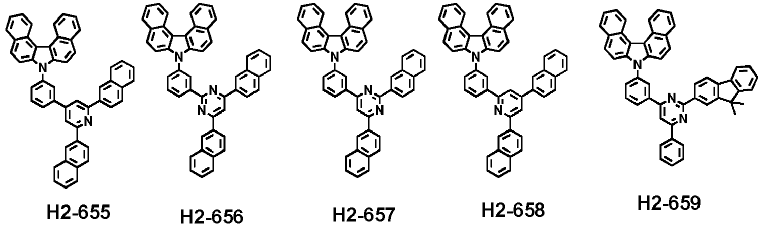

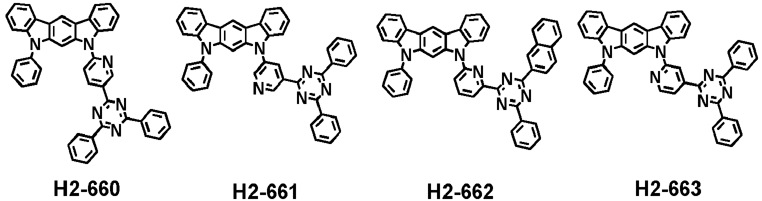

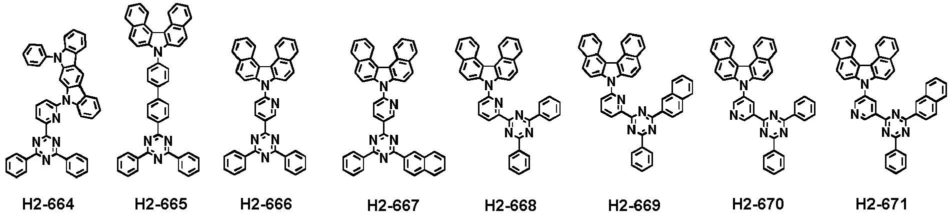

## Claims

1. An organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by the following formula 1 which is a bicarbazole derivative containing an aryl group, and a second host compound is represented by the following formula 2 which is a carbazole derivative including a nitrogen-containing heteroaryl group: 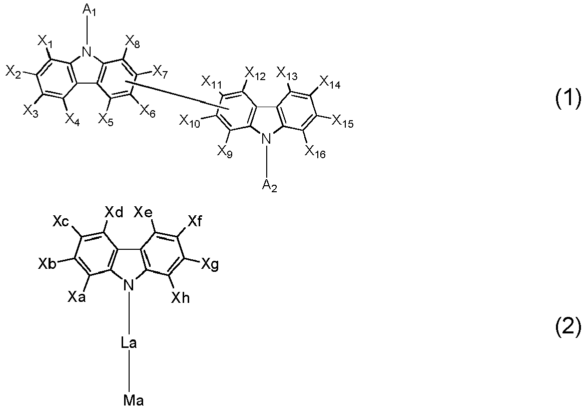 wherein
A₁ and A₂ each independently represent a substituted or unsubstituted (C6-C30)aryl group;
X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) may be replaced with at least one hetero atom selected from nitrogen, oxygen and sulfur;
Ma represents a substituted or unsubstituted nitrogen-containing 5- to 30-membered heteroaryl group;
La represents a single bond, or a substituted or unsubstituted (C6-C30)arylene group;
Xa to Xh each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; and
the heteroaryl group contains at least one hetero atom selected from B, N, O, S, P(=O), Si and P.

2. The organic electroluminescent device according to claim 1, wherein the compound of formula 1 is represented by the following formula 3, 4, 5, or 6: 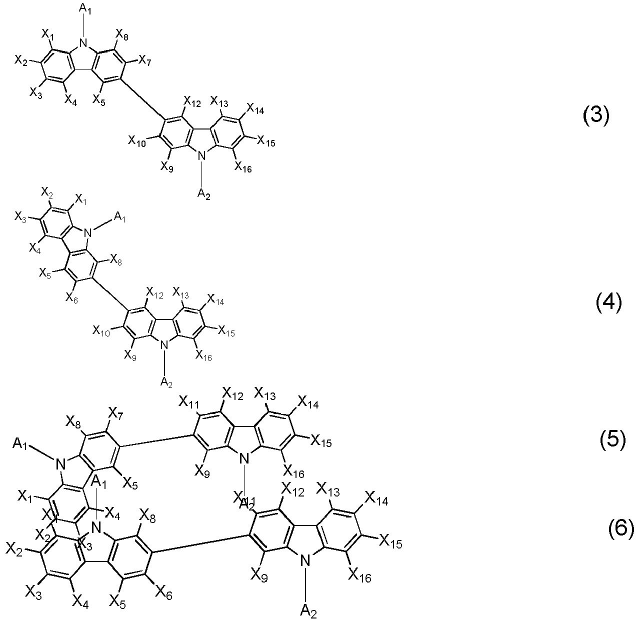 wherein
A₁ and A₂ each independently represent a substituted or unsubstituted (C6-C30)aryl group; and
X₁ to X₁₆ each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur.

3. The organic electroluminescent device according to claim 1, wherein the compound of formula 2 is represented by the following formula 8: 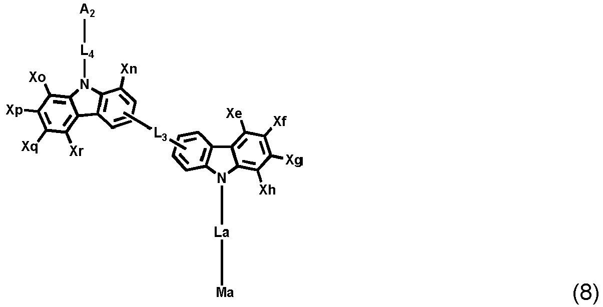 wherein
A₂ represents a substituted or unsubstituted (C6-C30)aryl group and may be bonded to Xn or Xo;
L₃ and L₄ each independently represent a single bond, or a substituted or unsubstituted (C6-C60)arylene group;
Xo to Xr and Xn each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3- to 7-membered heterocycloalkyl group, a substituted or unsubstituted (C6-C60)aryl group, a substituted or unsubstituted 3- to 30-membered heteroaryl group, - NR₅R₆, or -SiR₇R₈R₉;
Ma, La, and Xe to Xh are as defined in formula 2;
R₅, R₆ and R₇ to R₉ each independently represent hydrogen, deuterium, a halogen, a cyano group, a carboxyl group, a nitro group, a hydroxyl group, a substituted or unsubstituted (C1-C30)alkyl group, a substituted or unsubstituted (C3-C30)cycloalkyl group, a substituted or unsubstituted (C3-C30)cycloalkenyl group, a substituted or unsubstituted 3- to 7-membered heterocycloalkyl group, a substituted or unsubstituted (C6-C60)aryl group, or a substituted or unsubstituted 3- to 30-membered heteroaryl group.

4. The organic electroluminescent device according to claim 1, wherein La in formula 2 represents a single bond, or is represented by one selected from the following formulas 10 to 19: 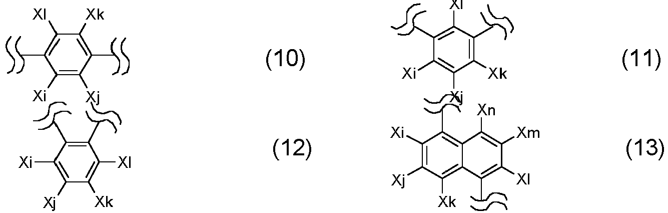 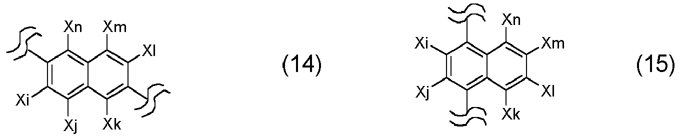 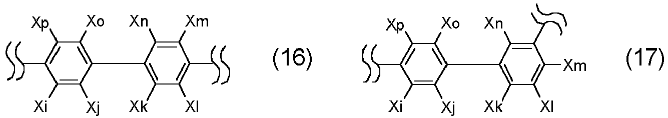 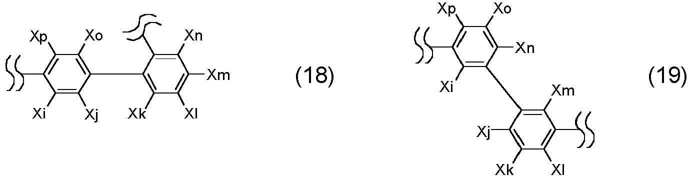 wherein
Xi to Xp each independently represent hydrogen, deuterium, a halogen, a cyano group, a substituted or unsubstituted (C1-C30)alkyl group, a substituted or unsubstituted (C2-C30)alkenyl group, a substituted or unsubstituted (C2-C30)alkynyl group, a substituted or unsubstituted (C3-C30)cycloalkyl group, a substituted or unsubstituted (C6-C60)aryl group, a substituted or unsubstituted 3- to 30-membered heteroaryl group, a substituted or unsubstituted tri(C1-C30)alkylsilyl group, a substituted or unsubstituted tri(C6-C30)arylsilyl group, a substituted or unsubstituted di(C1-C30)alkyl(C6-C30)arylsilyl group, or a substituted or unsubstituted mono- or di-(C6-C30)arylamino group; or are linked between adjacent substituents to form a substituted or unsubstituted mono- or polycyclic, (C3-C30) alicyclic or aromatic ring whose carbon atom(s) ring may be replaced with at least one hetero atom selected from nitrogen, oxygen and sulfur.

5. The organic electroluminescent device according to claim 1, wherein Ma in formula 2 is a monocyclic-based heteroaryl group selected from the group consisting of pyrrolyl, imidazolyl, pyrazolyl, triazinyl, tetrazinyl, triazolyl, tetrazolyl, pyridyl, pyrazinyl, pyrimidinyl and pyridazinyl, or a fused ring-based heteroaryl group selected from the group consisting of benzoimidazolyl, isoindolyl, indolyl, indazolyl, benzothiadiazolyl, quinolyl, isoquinolyl, cinnolinyl, quinazolinyl, naphthyridinyl, quinoxalinyl, carbazolyl and phenanthridinyl.

6. The organic electroluminescent device according to claim 1, wherein A₁ and A₂ in formula 1 each independently represent phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, or fluoranthenyl.

7. The organic electroluminescent device according to claim 1, wherein Xa to Xh in formula 2 each independently represent hydrogen; a cyano group; a (C6-C15)aryl group which is unsubstituted or substituted with a tri(C6-C10)arylsilyl group, or a 10- to 20-membered heteroaryl group which is unsubstituted or substituted with a (C6-C12)aryl group.

8. The organic electroluminescent device according to claim 1, wherein the triarylsilyl as X₁ to X₁₆ in formula 1 is triphenylsilyl.

9. The organic electroluminescent device according to claim 1, wherein the compound represented by formula 1 is selected from the group consisting of the following compounds:

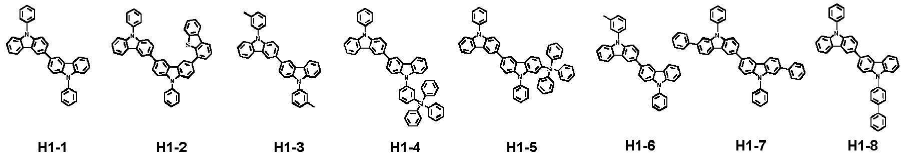

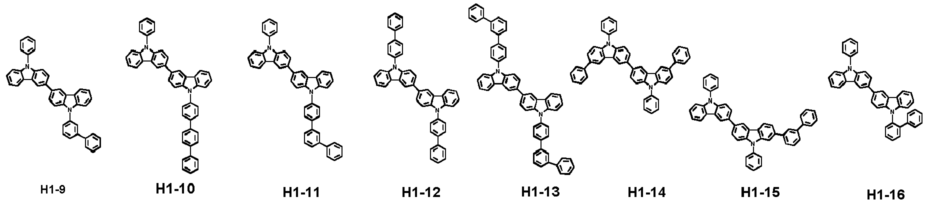

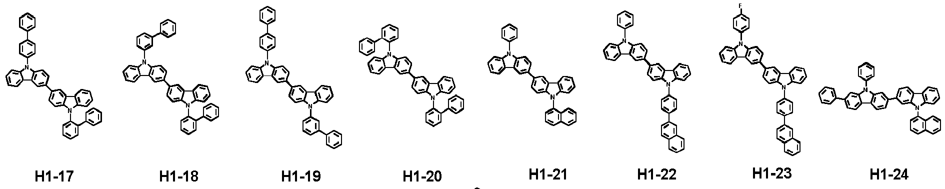

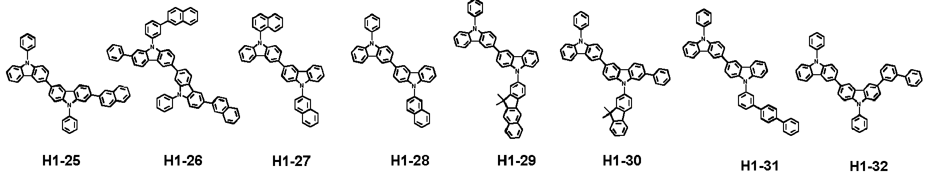

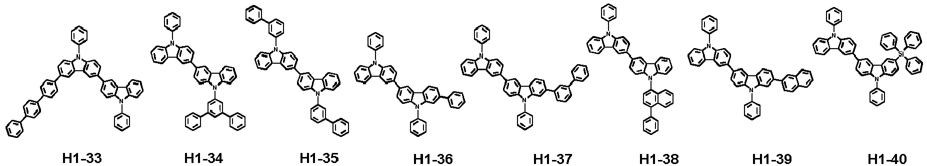

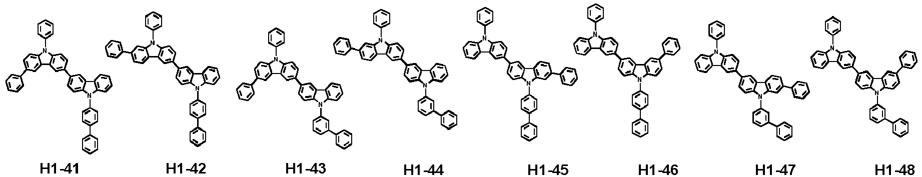

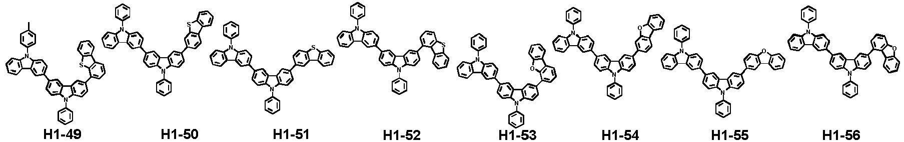

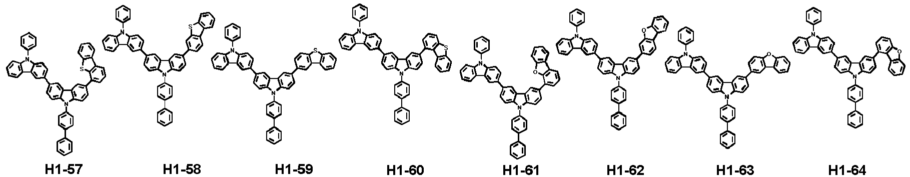

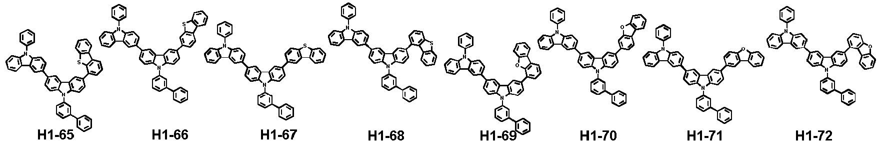

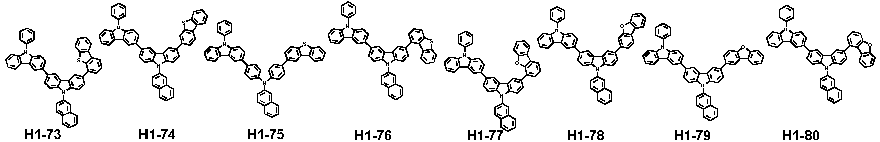

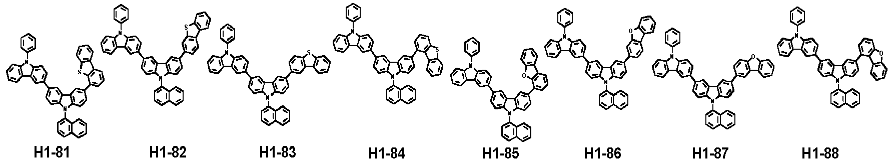

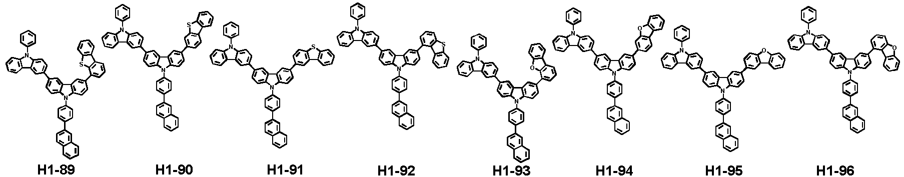

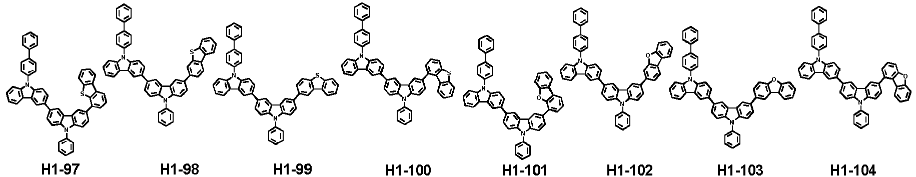

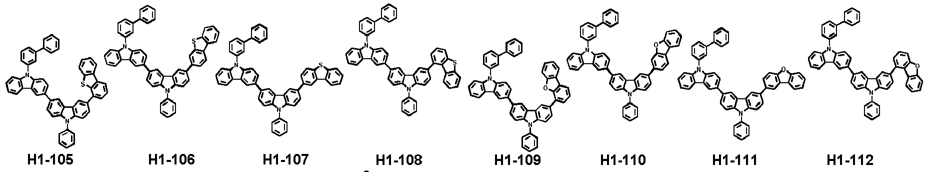

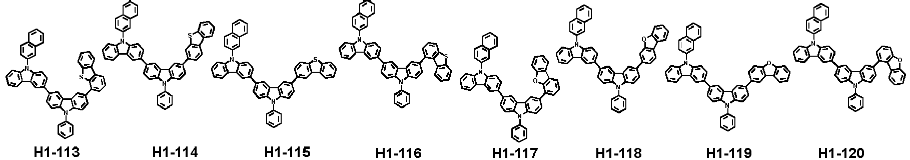

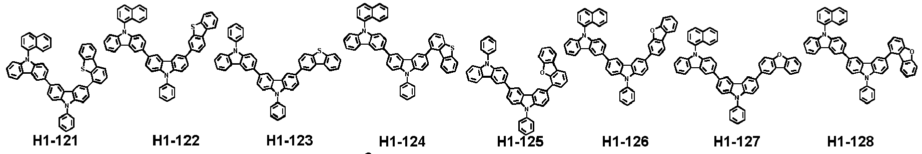

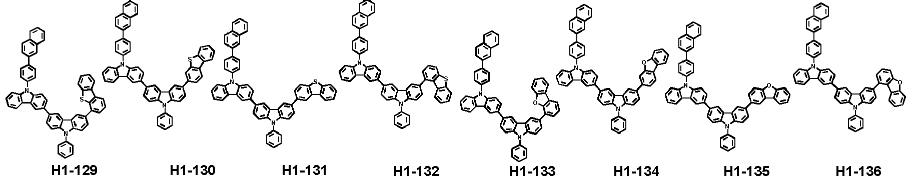

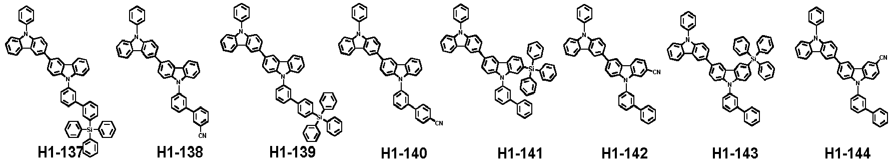

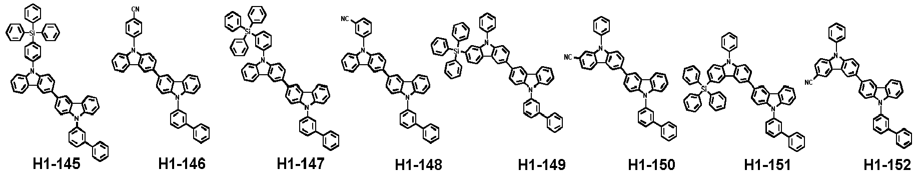

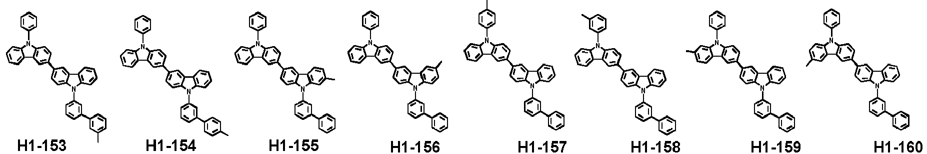

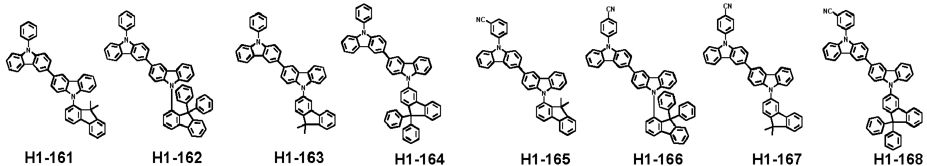

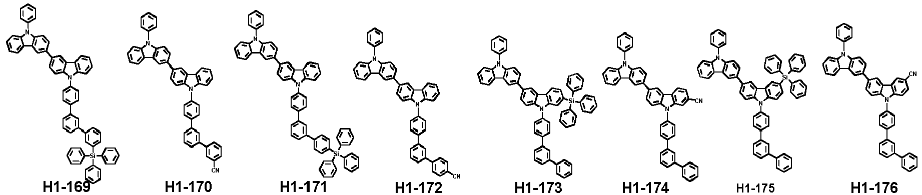

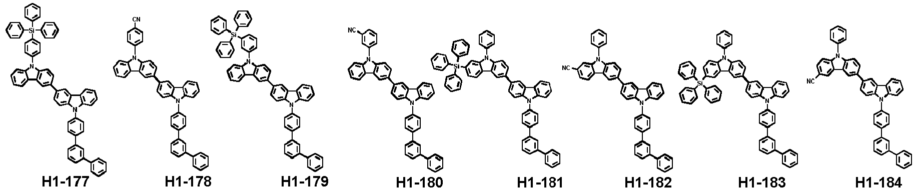

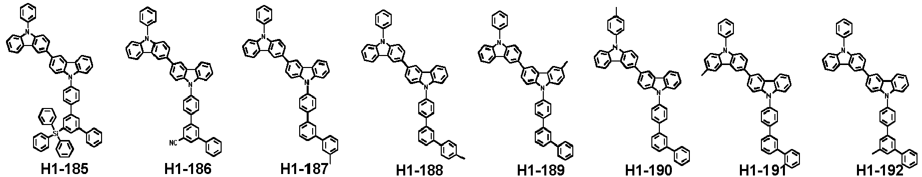

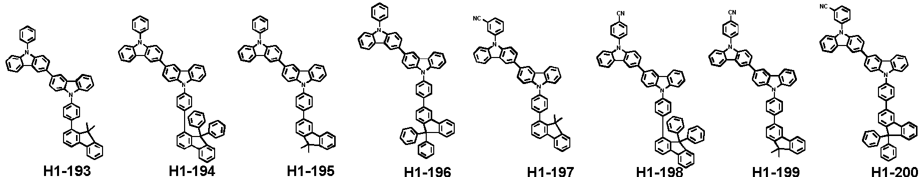

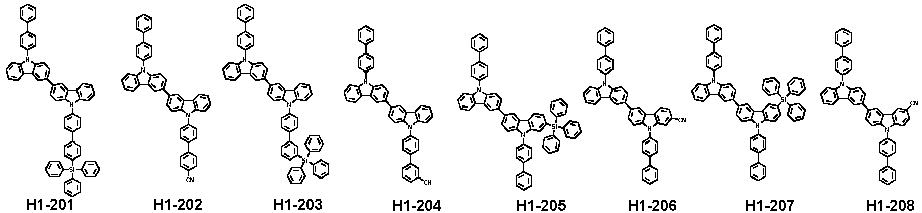

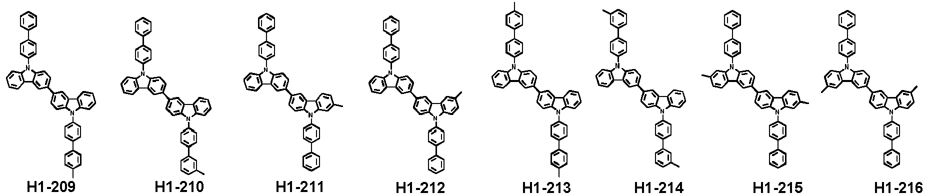

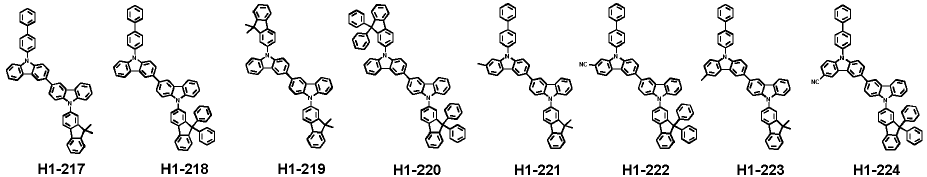

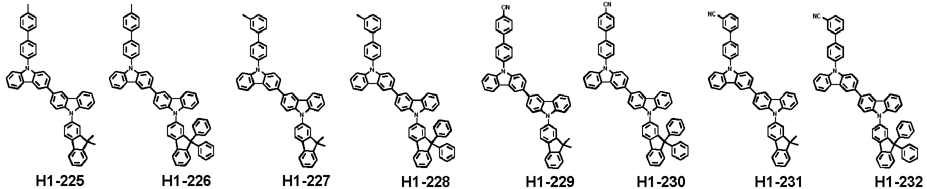

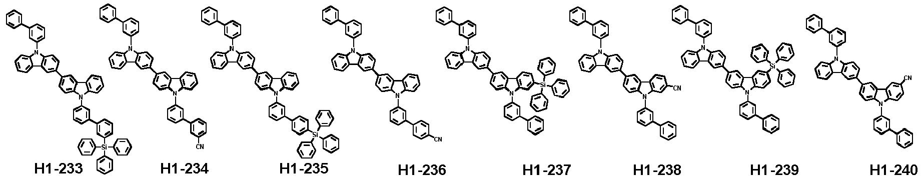

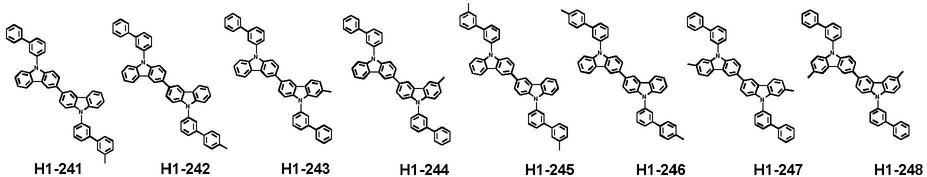

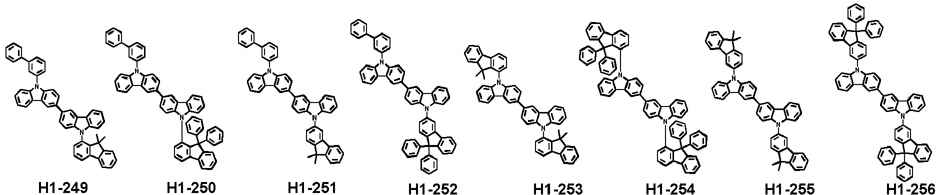

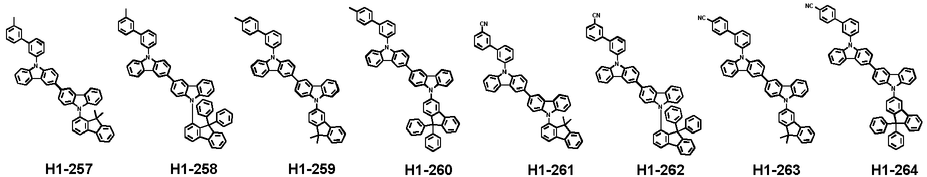

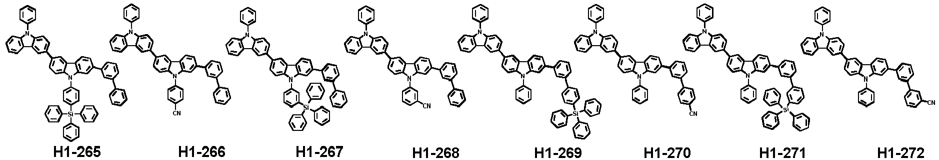

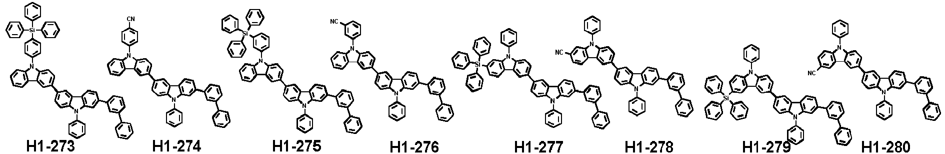

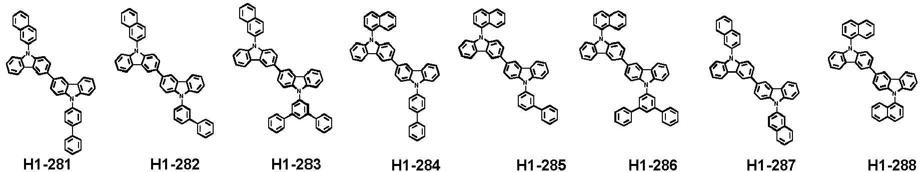

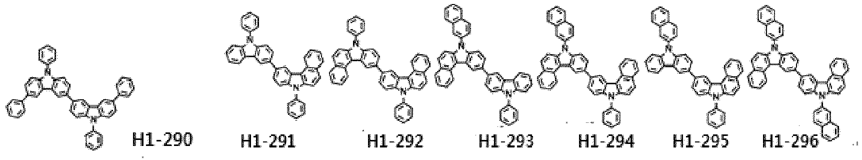

10. The organic electroluminescent device according to claim 1, wherein the compound represented by formula 2 is selected from the group consisting of the following compounds:
